# EUROPEAN PATENT APPLICATION

(11) **EP 1 690 942 A1**
(43) Date of publication of application: **16.08.2006**
(21) Application number: 05002963.6
(22) Date of filing: 11.02.2005
(51) Int. Cl.: C12N 15/82, C12N 15/71, C12N 15/74, C12N 15/85

(54) **Tetracycline-controlled insertion element for systematic mutagenesis**

(71) Applicant: Friedrich-Alexander-Universität Erlangen-Nürnberg, 91054 Erlangen (DE)
(72) Inventor: Hillen, Wolfgang, 91080 Uttenreuth (DE); Bertram, Ralph, 91207 Lauf (DE); Köstner, Martin, 90408 Nürnberg (DE)
(74) Representative: Vossius & Partner

(57) **Abstract**

The present invention relates to an insertion element, wherein said insertion element is a recombinant nucleic acid molecule which comprises, in 5' → 3' arrangement: (i) a first recombinase binding site; (ii) a resistance gene, operatively linked to a promoter and, optionally, to transcription control sequences; (iii) a tetracycline-controlled promoter; and (iv) a second recombinase binding site, wherein said tetracycline-controlled promoter is oriented to allow transcription in direction of the second recombinase binding site. The present invention also relates to a method for the generation of a conditional knock-out mutant of a cell, comprising the steps of: (a) introducing a nucleic acid molecule comprising the insertion element of any one of claims 1 to 16 into a cell, wherein the insertion element is transfected into the cell concomitant with purified recombinase; (b) integrating the insertion element into the cellular genome; and (c) selecting cells for resistance against a substrate for which the resistance gene of the insertion element confers resistance; wherein prior to or concomitant with step (a) an expression construct for a tetracycline-sensitive (poly)peptide is introduced into the cell, said (poly)peptide being capable of binding to the tetracycline-controlled promoter of the insertion element and of modulating its expression. Finally, the present invention relates to a cell containing the insertion element of the present invention, to a method for identifying genetic determinants of pathogen survival and to a method for the generation of a transgenic animal and plant.

SEQUENCE LISTING

<110> Universität Erlangen-Nürnberg; Lehrstuhl für Mikrobiologie

<120> Tetracycline-controlled insertion element for systematic mutagenesis

<130> K2194EP

<160> 393

<170> PatentIn version 3.1

<210> 1
<211> 19
<212> DNA
<213> Escherichia coli

<400> 1
actctatcat tgatagagt 19

<210> 2
<211> 19
<212> DNA
<213> Escherichia coli

<400> 2
tccctatcag tgatagaga 19

<210> 3
<211> 19
<212> DNA
<213> Escherichia coli

<400> 3

actctgtcat tgacagagt                                                    19

<210>  4

<211>  19

<212>  DNA

<213>  Escherichia coli

<400>  4
actgtatcat tgatacagt                                                    19

<210>  5

<211>  19

<212>  DNA

<213>  Escherichia coli

<400>  5
tccctgtcag tgacagaga                                                    19

<210>  6

<211>  19

<212>  DNA

<213>  Escherichia coli

<400>  6
tccgtatcag tgatacaga                                                    19

<210>  7

<211>  69

<212>  DNA

<213>  Escherichia coli

<400>  7
tcctaatttt tgttgacact ctatcattga tagagttatt ttaccactcc ctatcagtga      60

tagagaaaa                                                               69

<210>  8

<211>  69

<212>  DNA

<213>  Escherichia coli

<400>  8
tcctaatttt tgttgacact ctatcattga tagagttatt ttacctctcc ctatcagtga      60

tagagaaaa                                                              69

<210>  9

<211>  69

<212>  DNA

<213>  Escherichia coli

<400>  9
tcctaatttt tgttgacact ctatcattga tagagttatt ttacgtctcc ctatcagtga      60

tagagaaaa                                                              69

<210>  10

<211>  150

<212>  DNA

<213>  Bacillus subtilis

<400>  10
tctagacatc attaattcct cctttttgtt gacactctat cattgataga gttatttgtc      60

aaactagttt tttatttgga tcccctcgag ttcatgaaaa actaaaaaaa atattgacac     120

tctatcattg atagagtata attaaaataa                                      150

<210>  11

<211>  172

<212>  DNA

<213>  Bacillus subtilis

<400>  11
tctagacatc attaattcct cctttttgtt gacactctat cattgataga gttatttgtc      60

aaactagttt tttatttgga tcccctcgag ttcatgaaaa actaaaaaaa atattgacac     120

tctatcattg atagagtata attaaaataa gctactctat cattgataga gt          172

<210>  12

<211>  68

<212>  DNA

<213>  Bacillus subtilis

<400>  12
aattccaaaa actaaaaaaa atattgacac tctatcattg atagagtata attaacggga     60

tcccgcca                                                              68

<210>  13

<211>  89

<212>  DNA

<213>  Bacillus subtilis

<400>  13
aattccaaaa actaaaaaaa atattgacac tctatcattg atagagtata attaacggga     60

tcctctatca ttgatagagg atcccgcca                                       89

<210>  14

<211>  582

<212>  DNA

<213>  HeLa cell line

<400>  14
ggtaccgagc tcttacgcgt gctagcccgg gctcgagttt accactccct atcagtgata     60

gagaaaagtg aaagtcgagt ttaccactcc ctatcagtga tagagaaaag tgaaagtcga    120

gtttaccact ccctatcagt gatagagaaa agtgaaagtc gagtttacca ctccctatca    180

gtgatagaga aaagtgaaag tcgagtttac cactccctat cagtgataga gaaaagtgaa    240

agtcgagttt accactccct atcagtgata gagaaaagtg aaagtcgagt ttaccactcc    300

ctatcagtga tagagaaaag tgaaagtcga gctcggtacc cggagatctc aggctgtgcg    360

gttgggcggg gccctgtgcc ccactgcgga gtgcgggtcg ggaagcggag agagaagcag    420

ctgtgtaatc cgctggatgc ggaccagggc gctccccatt cccgtcggga gcccgccgat    480

tggctgggtg tgggcgcacg tgaccgacat gtggctgtat tggtgcagcc cgccagggtg    540

tcactggaga cagaatggag gtgctgccgg actcggaaat gg 582

<210> 15

<211> 620

<212> DNA

<213> HeLa cell line

<400> 15
ggtaccgagc tcttacgcgt gctagcccgg gctcgagttt accactccct atcagtgata 60

gagaaaagtg aaagtcgagt ttaccactcc ctatcagtga tagagaaaag tgaaagtcga 120

gtttaccact ccctatcagt gatagagaaa agtgaaagtc gagtttacca ctccctatca 180

gtgatagaga aaagtgaaag tcgagtttac cactccctat cagtgataga gaaaagtgaa 240

agtcgagttt accactccct atcagtgata gagaaaagtg aaagtcgagt ttaccactcc 300

ctatcagtga tagagaaaag tgaaagtcga gctcggtacc cggagatctt aggaagctgg 360

gaagtcttct aaggccggcc ccatgccacc aactacagac aggggtggag gaagttgagg 420

gccctgcagt tccttggctg ccacctgcag gtgcgtcccc agccaatcag cggcgccggg 480

ggcggtgcct gcgggctcac ctggcggccg cagcctctgc gctgctcact ggtgtgggag 540

ccgcggcgca ctactgagtt cccaagaact tctgctagac tcctgcccgg cctaacccgg 600

ccctgcccga ccgcacccgg 620

<210> 16

<211> 462

<212> DNA

<213> HeLa cell line

<400> 16
ggtaccgagc tcttacgcgt gctagcccgg gctcgagttt accactccct atcagtgata 60

gagaaaagtg aaagtcgagt ttaccactcc ctatcagtga tagagaaaag tgaaagtcga 120

gtttaccact ccctatcagt gatagagaaa agtgaaagtc gagtttacca ctccctatca 180

gtgatagaga aaagtgaaag tcgagtttac cactccctat cagtgataga gaaaagtgaa 240

agtcgagttt accactccct atcagtgata gagaaaagtg aaagtcgagt ttaccactcc 300

ctatcagtga tagagaaaag tgaaagtcga gctcggtacc cggagatctg ccgcagcctc 360

tgcgctgctc actggtgtgg gagccgcggc gcactactga gttcccaaga acttctgcta 420

gactcctgcc cggcctaacc cggccctgcc cgaccgcacc cg 462

<210> 17

<211> 468

<212> DNA

<213> HeLa cell line

<400> 17
tccctatcag tgatagagaa aagtgaaagt cgagtttacc actccctatc agtgatagag 60

aaaagtgaaa gtcgagttta ccactcccta tcagtgatag agaaaagtga aagtcgagtt 120

taccactccc tatcagtgat agagaaaagt gaaagtcgag tttaccactc cctatcagtg 180

atagagaaaa gtgaaagtcg agtttaccac tccctatcag tgatagagaa aagtgaaagt 240

cgagtttacc actccctatc agtgatagag aaaagtgaaa gtcgagctcg gtacccggag 300

atctgcgatc tgcatctcaa ttagtcagca accatagtcc cgcccctaac tccgcccatc 360

ccgcccctaa ctccgcccag ttccgcccat tctccgcccc atcgctgact aatttttttt 420

atttatgcag aggccgaggc cgcctcggcc tctgagctat tccagaag 468

<210> 18

<211> 723

<212> DNA

<213> HeLa cell line

<400> 18
cgatggagcg gagaatgggc ggaactgggc ggagttaggg gcgggatggg cggagttagg 60

ggcgggacta tggttgctga ctaattgaga tgcatgcttt gcatacttct gcctgctggg 120

gagcctgggg actttccaca cctggttgct gactaattga gatgcatgct ttgcatactt 180

ctgcctgctg gggagcctgg ggactttcca caccctaact gacacacatt ccacagcgga 240

tctcgagttt accactccct atcagtgata gagaaaagtg aaagtcgagt ttaccactcc 300

ctatcagtga tagagaaaag tgaaagtcga gtttaccact ccctatcagt gatagagaaa 360

agtgaaagtc gagtttacca ctccctatca gtgatagaga aaagtgaaag tcgagtttac 420

cactccctat cagtgataga gaaaagtgaa agtcgagttt accactccct atcagtgata 480

gagaaaagtg aaagtcgagt ttaccactcc ctatcagtga tagagaaaag tgaaagtcga 540

gctcggtacc cggagatctg cgatctgcat ctcaattagt cagcaaccat agtcccgccc 600

ctaactccgc ccatcccgcc cctaactccg cccagttccg cccattctcc gccccatcgc 660

tgactaattt tttttattta tgcagaggcc gaggccgcct cggcctctga gctattccag 720

aag 723

<210> 19

<211> 692

<212> DNA

<213> HeLa cell line

<400> 19
cgatggagcg gagaatgggc ggaactgggc ggagttaggg gcgggatggg cggagttagg 60

ggcgggacta tggttgctga ctaattgaga tgcatgcttt gcatacttct gcctgctggg 120

gagcctgggg actttccaca cctggttgct gactaattga gatgcatgct ttgcatactt 180

ctgcctgctg gggagcctgg ggactttcca caccctaact gacacacatt ccacagcgga 240

tctcgagttt accactccct atcagtgata gagaaaagtg aaagtcgagt ttaccactcc 300

ctatcagtga tagagaaaag tgaaagtcga gtttaccact ccctatcagt gatagagaaa 360

agtgaaagtc gagtttacca ctccctatca gtgatagaga aaagtgaaag tcgagtttac 420

cactccctat cagtgataga gaaaagtgaa agtcgagttt accactccct atcagtgata 480

gagaaaagtg aaagtcgagt ttaccactcc ctatcagtga tagagaaaag tgaaagtcga 540

gctcggtacc cggagatctg cgatcgtagg cgtgtacggt gggaggtcta tataagcaga 600

gctcgtttag tgaaccgtca gatcgcctgg agacgccatc cacgctgttt tgacctccat 660

agaagacacc gggaccgatc cagcctccgc gg 692

<210> 20

<211> 1089

<212> DNA

<213> HeLa cell line

<400> 20
ctcgaggagc ttggcccatt gcatacgttg tatccatatc ataatatgta catttatatt 60

ggctcatgtc caacattacc gccatgttga cattgattat tgactagtta ttaatagtaa 120

tcaattacgg ggtcattagt tcatagccca tatatggagt tccgcgttac ataacttacg 180

gtaaatggcc cgcctggctg accgcccaac gacccccgcc cattgacgtc aataatgacg 240

tatgttccca tagtaacgcc aatagggact ttccattgac gtcaatgggt ggagtattta 300

cgctaaactg cccacttggc agtacatcaa gtgtatcata tgccaagtac gccccctatt 360

gacgtcaatg acggtaaatg gcccgcctgg cattatgccc agtacatgac cttatgggac 420

tttcctactt ggcagtacat ctacgtatta gtcatcgcta ttaccatggt gatgcggttt 480

tggcagtaca tcaatgggcg tggatagcgg tttgactcac ggggatttcc aagtctccac 540

cccattgacg tcaatgggag tttgttttgg caccaaaatc aacgggactt tccaaaatgt 600

cgtaacaact ccgccccatt gacgcaaatg ggcgggatct cgagtttacc actccctatc 660

agtgatagag aaaagtgaaa gtcgagttta ccactcccta tcagtgatag agaaaagtga 720

aagtcgagtt taccactccc tatcagtgat agagaaaagt gaaagtcgag tttaccactc 780

cctatcagtg atagagaaaa gtgaaagtcg agtttaccac tccctatcag tgatagagaa 840

aagtgaaagt cgagtttacc actccctatc agtgatagag aaaagtgaaa gtcgagttta 900

ccactcccta tcagtgatag agaaaagtga aagtcgagct cggtacccgg agatctgcga 960

tcgtaggcgt gtacggtggg aggtctatat aagcagagct cgtttagtga accgtcagat 1020

cgcctggaga cgccatccac gctgttttga cctccataga agacaccggg accgatccag 1080

cctccgcgg 1089

<210> 21

<211> 1120

<212> DNA

<213> HeLa cell line

<400> 21
ctcgaggagc ttggcccatt gcatacgttg tatccatatc ataatatgta catttatatt 60

ggctcatgtc caacattacc gccatgttga cattgattat tgactagtta ttaatagtaa 120

tcaattacgg ggtcattagt tcatagccca tatatggagt tccgcgttac ataacttacg 180

gtaaatggcc cgcctggctg accgcccaac gacccccgcc cattgacgtc aataatgacg 240

tatgttccca tagtaacgcc aatagggact ttccattgac gtcaatgggt ggagtattta 300

cgctaaactg cccacttggc agtacatcaa gtgtatcata tgccaagtac gccccctatt 360

gacgtcaatg acggtaaatg gcccgcctgg cattatgccc agtacatgac cttatgggac 420

tttcctactt ggcagtacat ctacgtatta gtcatcgcta ttaccatggt gatgcggttt 480

tggcagtaca tcaatgggcg tggatagcgg tttgactcac ggggatttcc aagtctccac 540

cccattgacg tcaatgggag tttgttttgg caccaaaatc aacgggactt tccaaaatgt 600

cgtaacaact ccgccccatt gacgcaaatg ggcgggatct cgagtttacc actccctatc 660

agtgatagag aaaagtgaaa gtcgagttta ccactcccta tcagtgatag agaaaagtga 720

aagtcgagtt taccactccc tatcagtgat agagaaaagt gaaagtcgag tttaccactc    780

cctatcagtg atagagaaaa gtgaaagtcg agtttaccac tccctatcag tgatagagaa    840

aagtgaaagt cgagtttacc actccctatc agtgatagag aaaagtgaaa gtcgagttta    900

ccactcccta tcagtgatag agaaaagtga aagtcgagct cggtacccgg agatctgcga    960

tctgcatctc aattagtcag caaccatagt cccgccccta actccgccca tcccgcccct   1020

aactccgccc agttccgccc attctccgcc ccatcgctga ctaatttttt ttatttatgc   1080

agaggccgag gccgcctcgg cctctgagct attccagaag                         1120

<210>  22

<211>  216

<212>  PRT

<213>  Escherichia coli

<400>  22

Met Thr Lys Leu Gln Pro Asn Thr Val Ile Arg Ala Ala Leu Asp Leu
1               5                   10                  15

Leu Asn Glu Val Gly Val Asp Gly Leu Thr Thr Arg Lys Leu Ala Glu
            20                  25                  30

Arg Leu Gly Val Gln Gln Pro Ala Leu Tyr Trp His Phe Phe Thr Arg
        35                  40                  45

Asn Lys Arg Ala Leu Leu Asp Ala Leu Ala Glu Ala Met Leu Ala Glu
    50                  55                  60

Asn His Ser Thr Ser Val Pro Arg Ala Asp Asp Asp Trp Arg Ser Phe
65                  70                  75                  80

Leu Thr Gly Asn Ala Arg Ser Phe Arg Gln Ala Leu Leu Ala Tyr Arg
                85                  90                  95

Asp Gly Ala Arg Ile His Ala Gly Thr Arg Pro Gly Ala Pro Gln Met
            100                 105                 110

Glu Thr Ala Asp Ala Gln Leu Arg Phe Leu Cys Glu Ala Gly Phe Ser
        115                 120                 125

Ala Gly Asp Ala Val Asn Ala Leu Met Thr Ile Ser Tyr Val Gly Ala
    130                 135                 140

Val Leu Glu Glu Gln Ala Gly Asp Ser Glu Ser Gly Glu Arg Gly Gly
145                 150                 155                 160

Thr Val Glu Gln Ala Pro Leu Ser Pro Leu Leu Arg Ala Ala Ile Asp
                165                 170                 175

Ala Phe Asp Glu Ala Gly Pro Asp Ala Ala Phe Glu Gln Gly Leu Ala
            180                 185                 190

Val Ile Val Asp Gly Leu Ala Lys Arg Arg Leu Val Val Arg Asn Val
        195                 200                 205

Glu Gly Pro Arg Lys Gly Asp Asp
    210                 215

<210>  23

<211>  208

<212>  PRT

<213>  Escherichia coli

<400>  23

Met Met Ser Arg Leu Asp Lys Ser Lys Val Ile Asn Ser Ala Leu Glu
1               5                   10                  15

Leu Leu Asn Glu Val Gly Ile Glu Gly Leu Lys Thr Arg Lys Leu Ala
            20                  25                  30

Gln Lys Leu Gly Val Glu Gln Pro Thr Leu Tyr Trp His Val Lys Asn
        35                  40                  45

Lys Arg Ala Leu Leu Asp Ala Leu Ala Ile Glu Met Leu Asp Arg His
    50                  55                  60

His Thr His Phe Cys Pro Leu Glu Gly Glu Ser Trp Gln Asp Phe Leu
65                  70                  75                  80

Arg Asn Asn Ala Lys Ser Phe Arg Cys Ala Leu Leu Ser His Arg Asp
                85                  90                  95

Gly Ala Lys Val His Leu Gly Thr Arg Pro Thr Glu Lys Gln Tyr Glu
            100                 105                 110

Thr Leu Glu Asn Gln Leu Ala Phe Leu Cys Gln Gln Gly Phe Ser Leu
        115                 120                 125

Glu Asn Ala Leu Tyr Ala Leu Ser Ala Val Gly His Phe Thr Leu Gly
    130                 135                 140

Cys Val Leu Glu Asp Gln Glu His Gln Val Ala Lys Glu Glu Arg Glu
145                 150                 155                 160

Thr Pro Thr Thr Asp Ser Met Pro Ala Leu Leu Arg Gln Ala Ile Glu
                165                 170                 175

Leu Phe Glu His Gln Gly Ala Glu Pro Ala Phe Leu Phe Gly Leu Glu
            180                 185                 190

Leu Ile Ile Cys Gly Leu Glu Lys Gln Leu Lys Cys Glu Ser Gly Ser
        195                 200                 205

<210>  24

<211>  219

<212>  PRT

<213>  Escherichia coli

<400>  24

Met Asn Lys Leu Gln Arg Glu Ala Val Ile Arg Thr Ala Leu Glu Leu
1               5                   10                  15

Leu Asn Asp Val Gly Met Glu Gly Leu Thr Thr Arg Arg Leu Ala Glu
            20                  25                  30

Arg Leu Gly Val Gln Gln Pro Ala Leu Tyr Trp His Phe Lys Asn Lys
        35                  40                  45

Arg Ala Leu Leu Asp Ala Leu Ala Glu Ala Met Leu Thr Ile Asn His
    50                  55                  60

Thr His Ser Thr Pro Arg Asp Asp Asp Asp Trp Arg Ser Phe Leu Lys
65                  70                  75                  80

Gly Asn Ala Cys Ser Phe Arg Arg Ala Leu Leu Ala Tyr Arg Asp Gly
                85                  90                  95

Ala Arg Ile His Ala Gly Thr Arg Pro Ala Ala Pro Gln Met Glu Lys
            100                 105                 110

Ala Asp Ala Gln Leu Arg Phe Leu Cys Asp Ala Gly Phe Ser Ala Gly

115                 120                 125

Asp Ala Thr Tyr Ala Leu Met Ala Ile Ser Tyr Phe Thr Val Gly Ala
    130                 135                 140

Val Leu Glu Gln Gln Ala Ser Glu Ala Asp Ala Glu Glu Arg Gly Glu
145                 150                 155                 160

Asp Gln Leu Thr Thr Ser Ala Ser Thr Met Pro Ala Arg Leu Gln Ser
                165                 170                 175

Ala Met Lys Ile Val Tyr Glu Gly Gly Pro Asp Ala Ala Phe Glu Arg
            180                 185                 190

Gly Leu Ala Leu Ile Ile Gly Gly Leu Glu Lys Met Arg Leu Thr Thr
        195                 200                 205

Asn Asp Ile Glu Val Leu Lys Asn Val Asp Glu
    210                 215

<210>  25

<211>  208

<212>  PRT

<213>  Escherichia coli

<400>  25

Met Ala Arg Leu Asn Arg Glu Ser Val Ile Asp Ala Ala Leu Glu Leu
1               5                   10                  15

Leu Asn Glu Thr Gly Ile Asp Gly Leu Thr Thr Arg Lys Leu Ala Gln
            20                  25                  30

Lys Leu Gly Ile Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  26

<211>  211

<212>  PRT

<213>  Escherichia coli

<400>  26

Met Ala Arg Leu Ser Leu Asp Asp Val Ile Ser Met Ala Leu Thr Leu
1               5                   10                  15

Leu Asp Ser Glu Gly Leu Glu Gly Leu Thr Thr Arg Lys Leu Ala Gln
            20                  25                  30

Ser Leu Lys Ile Glu Gln Pro Thr Leu Tyr Trp His Val Arg Asn Lys
        35                  40                  45

Gln Thr Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys
    210

<210>  27

<211>  208

<212>  PRT

<213>  Escherichia coli

<400>  27

Met Thr Lys Leu Asp Lys Gly Thr Val Ile Ala Ala Ala Leu Glu Leu
1               5                   10                  15

Leu Asn Glu Val Gly Met Asp Ser Leu Thr Thr Arg Lys Leu Ala Glu
            20                  25                  30

Arg Leu Lys Val Gln Gln Pro Ala Leu Tyr Trp His Phe Gln Asn Lys
        35                  40                  45

Arg Ala Leu Leu Asp Ala Leu Ala Glu Ala Met Leu Ala Glu Arg His
    50                  55                  60

Thr Arg Ser Leu Pro Glu Glu Asn Glu Asp Trp Arg Val Phe Leu Lys
65                  70                  75                  80

Glu Asn Ala Leu Ser Phe Arg Thr Ala Leu Leu Ser Tyr Arg Asp Gly
                85                  90                  95

Ala Arg Ile His Ala Gly Thr Arg Pro Thr Glu Pro Asn Phe Gly Thr
            100                 105                 110

Ala Glu Thr Gln Ile Arg Phe Leu Cys Ala Glu Gly Phe Cys Pro Lys
        115                 120                 125

Arg Ala Val Trp Ala Leu Arg Ala Val Ser His Tyr Val Val Gly Ser
    130                 135                 140

Val Leu Glu Gln Gln Ala Ser Asp Ala Asp Glu Arg Val Pro Asp Arg
145                 150                 155                 160

Pro Asp Val Ser Glu Gln Ala Pro Ser Ser Phe Leu His Asp Leu Phe
                165                 170                 175

His Glu Leu Glu Thr Asp Gly Met Asp Ala Ala Phe Asn Phe Gly Leu
            180                 185                 190

Asp Ser Leu Ile Ala Gly Phe Glu Arg Leu Arg Ser Ser Thr Thr Asp
        195                 200                 205

<210>  28

<211>  207

<212>  PRT

<213>  Escherichia coli

<400>  28

Met Ser Arg Leu Asp Lys Glu Gln Val Ile Asp Asn Ala Leu Ile Leu
1               5                   10                  15

Leu Asn Glu Val Gly Ile Glu Gly Leu Thr Thr Arg Lys Leu Ala Gln
            20                  25                  30

Lys Ile Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Pro Asn Glu Thr Trp Gln Asp Phe Leu Arg

65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Glu Ser Gln Phe Glu Thr
            100                 105                 110

Ser Glu Gln Gln Leu Gln Phe Leu Cys Asp Ala Gly Phe Ser Leu Ser
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Glu Thr Asp Thr Val Ala Tyr Pro Pro Leu Leu Thr Gln Ala Val
                165                 170                 175

Ala Ile Met Asp Ser Asp Asn Gly Asp Ala Ala Phe Leu Phe Val Leu
            180                 185                 190

Asp Val Met Ile Ser Gly Leu Glu Thr Val Leu Lys Ser Ala Lys
        195                 200                 205

<210>  29

<211>  207

<212>  PRT

<213>  Escherichia coli

<400>  29

Met Ser Arg Leu Asp Lys Glu Gln Val Ile Asp Asn Ala Leu Ile Leu
1               5                   10                  15

Leu Asn Glu Val Gly Met Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Ala Asn Glu Ser Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Ala Asn Gln Phe Glu Thr
            100                 105                 110

Ser Glu Gln Gln Leu Gln Phe Leu Cys Asp Ala Gly Phe Thr Leu Thr
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Pro Lys Thr Glu Ile Asn Tyr Pro Pro Leu Leu Thr Gln Ala Ile
                165                 170                 175

Asp Ile Met Asp Ser Asp Asn Gly Glu Ala Ala Phe Leu Phe Val Leu
            180                 185                 190

Asp Val Met Ile Ser Gly Leu Glu Thr Val Leu Asn Asn His His
        195                 200                 205

<210>  30

<211>  199

<212>  PRT

<213>  Escherichia coli

<400>  30

Met Thr His His Arg Lys Arg Leu Asp Arg Thr Ser Val Leu Leu Gly
1               5                   10                  15

Ala Arg Gln Val Leu Asp Glu Thr Gly Leu Asp Gly Phe Thr Thr Arg
            20                  25                  30

Ala Leu Ala Ala His Leu Gln Val Gln Gln Pro Gly Leu Tyr Trp His
        35                  40                  45

Phe Arg Thr Lys Ala Glu Leu Leu Ala Ala Leu Ala Ser Asp Val Leu
    50                  55                  60

Asp Arg Glu His His Ala Ser Phe Pro Glu Ser Asp Glu Lys Trp Asp
65                  70                  75                  80

Ala Phe Leu Leu Arg Asn Ala Arg Ser Phe Arg His Ala Leu His Ala
                85                  90                  95

Val Arg Asp Gly Ala Arg Leu His Ala Glu His His Arg Arg Pro Ser
            100                 105                 110

Gly Asp Asp Thr Asp Glu Gly Ala Glu Ala Ser Gly Gln Gln Val Gly
        115                 120                 125

Leu Leu Val Ser Gln Gly Phe Asp Glu Gln Thr Ala Ile Ser Met Leu
    130                 135                 140

Ile Ala Val Ser Arg Tyr Thr Val Gly Val Val Leu Glu Glu Gln Thr
145                 150                 155                 160

Ala Glu Val Gly Asn Ala Ser Val Pro Glu Arg Asp Arg Glu Phe Asp
                165                 170                 175

Phe Gly Leu Thr Ala Leu Ile Asp Gly Phe Ser His Ser Arg Thr Ala
            180                 185                 190

Ser Leu Thr Ser Ser Glu Ser
        195

<210>  31

<211>  210

<212>  PRT

<213>  Escherichia coli

<400>  31

Met Met Ala Arg Leu Asp Lys Lys Arg Val Ile Glu Ser Ala Leu Ala
1               5                   10                  15

Leu Leu Asp Glu Val Gly Met Glu Gly Leu Thr Thr Arg Lys Leu Ala
            20                  25                  30

Gln Lys Leu Asn Ile Glu Gln Pro Ser Leu Tyr Trp His Val Lys Asn
        35                  40                  45

Lys Arg Ala Leu Leu Asp Ala Leu Ser Val Glu Ile Leu Leu Arg His
    50                  55                  60

His Asp His Phe Gln Pro Gln Lys Gly Glu Tyr Trp Ala Asp Phe Leu
65                  70                  75                  80

Arg Glu Asn Ala Lys Ser Phe Arg Arg Ala Leu Leu Ser His Arg Asp
                85                  90                  95

Ala Ala Lys Ile His Leu Gly Thr Arg Pro Ser Pro Glu Gln Phe Glu
            100                 105                 110

Thr Val Glu Ala Gln Leu Ala Phe Leu Cys Glu Gln Gly Phe Ser Leu
        115                 120                 125

Glu Glu Ala Leu Tyr Thr Leu Gly Val Val Ser His Phe Thr Leu Gly
    130                 135                 140

Ser Val Leu Glu Glu Arg Glu Tyr Leu Glu Ala Met Arg Asp Asp Asp
145                 150                 155                 160

Pro Ala Ile His Ala Ala Met Pro Pro Leu Leu Thr Lys Ala Leu Glu
                165                 170                 175

Ile Met Glu Gln Asp Thr Gly Glu Lys Pro Phe Leu Phe Gly Leu Glu
            180                 185                 190

Val Ile Ile Leu Gly Leu Glu Ala Lys Gln Lys Gln Lys Lys Gly Asn
        195                 200                 205

Gln Glu
    210

<210>  32

<211>  189

<212>  PRT

<213>  Escherichia coli

<400>  32

Met Ala Gln Lys Gln Ala Arg Leu Asp Arg Ala Ala Val Leu Arg Gly
1               5                   10                  15

Ala Arg His Val Leu Asn Asn Thr Gly Ile Asp Gly Phe Thr Thr Arg
            20                  25                  30

Ala Leu Ala Ala His Leu Arg Val Gln Gln Pro Ala Leu Tyr Trp His

35                  40                  45

Phe Arg Thr Lys Ala His Leu Leu Gly Ser Leu Ala Ala Asp Val Leu
    50                  55                  60

Asp Arg Glu His His Ala Ser Leu Pro Glu Ser Gly Glu Arg Trp Asp
65                  70                  75                  80

Asp Phe Leu Leu Arg Asn Ala Arg Ser Phe Arg Thr Ala Leu Leu Ala
                85                  90                  95

Val Arg Asp Gly Ala Arg Leu His Ala Glu Phe His Arg Gln Lys Ser
            100                 105                 110

Asp Gln Met Pro Ala Gly Ser Asp Ala Pro Glu Ser Gln Ile Glu Phe
        115                 120                 125

Leu Val Ser Glu Gly Phe Ala Glu Gly Ser Ala Val Arg Ala Leu Met
    130                 135                 140

Ala Ile Ser Arg Tyr Thr Val Gly Phe Val Leu Glu Glu Gln Thr Ala
145                 150                 155                 160

Leu Asp Asn Gly Cys Glu Pro Val Asp Gln Asp Leu Asp Phe Glu Phe
                165                 170                 175

Gly Leu Val Ala Met Val Glu Gly Leu Ala Ser Lys Arg
            180                 185

<210>  33

<211>  216

<212>  PRT

<213>  Escherichia coli

<400>  33

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Ala Met Leu Ala Glu Asn His
    50                  55                  60

Thr His Ser Val Pro Arg Ala Asp Asp Asp Trp Arg Ser Phe Leu Ile
65                  70                  75                  80

Gly Asn Ala Arg Ser Phe Arg Gln Ala Leu Leu Ala Tyr Arg Asp Gly
                85                  90                  95

Ala Arg Ile His Ala Gly Thr Arg Pro Gly Ala Pro Gln Met Glu Thr
            100                 105                 110

Ala Asp Ala Gln Leu Arg Phe Leu Cys Glu Ala Gly Phe Ser Ala Gly
        115                 120                 125

Asp Ala Val Asn Ala Leu Met Thr Ile Ser Tyr Phe Thr Val Gly Ala
    130                 135                 140

Val Leu Glu Glu Gln Ala Gly Asp Ser Asp Ala Gly Glu Arg Gly Gly
145                 150                 155                 160

Thr Val Glu Gln Ala Pro Leu Ser Pro Leu Leu Arg Ala Ala Ile Asp
                165                 170                 175

Ala Phe Asp Glu Ala Gly Pro Asp Ala Ala Phe Glu Gln Gly Leu Ala
            180                 185                 190

Val Ile Val Asp Gly Leu Ala Lys Arg Arg Leu Val Val Arg Asn Val
        195                 200                 205

Glu Gly Pro Arg Lys Gly Asp Asp
    210                 215

<210>  34

<211>  207

<212>  PRT

<213>  Escherichia coli

<400>  34

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Ser
        195                 200                 205

<210>  35

<211>  219

<212>  PRT

<213>  Escherichia coli

<400>  35

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Ala Met Leu Thr Ile Asn His
    50                  55                  60

Thr His Ser Thr Pro Arg Asp Asp Asp Asp Trp Arg Ser Phe Leu Lys
65                  70                  75                  80

Gly Asn Ala Cys Ser Phe Arg Arg Ala Leu Leu Ala Tyr Arg Asp Gly
                85                  90                  95

Ala Arg Ile His Ala Gly Thr Arg Pro Ala Ala Pro Gln Met Glu Lys
            100                 105                 110

Ala Asp Ala Gln Leu Arg Phe Leu Cys Asp Ala Gly Phe Ser Ala Gly
        115                 120                 125

Asp Ala Thr Tyr Ala Leu Met Ala Ile Ser Tyr Phe Thr Val Gly Ala
    130                 135                 140

Val Leu Glu Gln Gln Ala Ser Glu Ala Asp Ala Glu Glu Arg Gly Glu
145                 150                 155                 160

Asp Gln Leu Thr Thr Ser Ala Ser Thr Met Pro Ala Arg Leu Gln Ser
                165                 170                 175

Ala Met Lys Ile Val Tyr Glu Gly Gly Pro Asp Ala Ala Phe Glu Arg
            180                 185                 190

Gly Leu Ala Leu Ile Ile Gly Gly Leu Glu Lys Met Arg Leu Thr Thr
        195                 200                 205

Asn Asp Ile Glu Val Leu Lys Asn Val Asp Glu
    210                 215

<210>  36

<211>  208

<212>  PRT

<213>  Escherichia coli

<400>  36

Met Ser Arg Leu Asp Lys Ser Lys Val Ile Asn Ser Ala Leu Glu Leu

1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  37

<211>  203

<212>  PRT

<213>  Escherichia coli

<400>  37

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp Val Lys Asn Lys Arg
        35                  40                  45

Ala Leu Met Asn Met Leu Ser Glu Ala Ile Leu Ala Lys His His Thr
    50                  55                  60

Ser Ala Pro Leu Pro Thr Glu Ser Trp Gln Gln Phe Leu Gln Glu Asn
65                  70                  75                  80

Ala Leu Ser Phe Arg Ala Leu Leu Val His Arg Asp Gly Ala Arg Leu
                85                  90                  95

His Ile Gly Thr Ser Pro Thr Pro Pro Gln Glu Gln Ala Glu Ala Gln
            100                 105                 110

Leu Arg Cys Leu Cys Asp Ala Gly Phe Ser Val Glu Glu Ala Leu Ile
        115                 120                 125

Leu Gln Ser Ile Ser His Phe Thr Leu Gly Ala Val Leu Glu Glu Gln
    130                 135                 140

Ala Thr Asn Gln Glu Asn Asn His Val Ile Asp Ala Ala Pro Pro Leu
145                 150                 155                 160

Leu Gln Glu Ala Phe Asn Ile Gln Ala Thr Ser Ala Glu Met Ala Phe
                165                 170                 175

His Phe Gly Leu Lys Ser Leu Ile Phe Gly Phe Ser Ala Gln Asp Glu
            180                 185                 190

Lys Lys His Thr Pro Ile Glu Asp Gly Asn Lys
        195                 200

<210>  38

<211>  208

<212>  PRT

<213>  Escherichia coli

<400>  38

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Ala Met Leu Ala Glu Arg His
    50                  55                  60

Thr Arg Ser Leu Pro Glu Glu Asn Glu Asp Trp Arg Val Phe Leu Lys
65                  70                  75                  80

Glu Asn Ala Leu Ser Phe Arg Thr Ala Leu Leu Ser Tyr Arg Asp Gly
                85                  90                  95

Ala Arg Ile His Ala Gly Thr Arg Pro Thr Glu Pro Asn Phe Gly Thr
            100                 105                 110

Ala Glu Thr Gln Ile Arg Phe Leu Cys Ala Glu Gly Phe Cys Pro Lys
        115                 120                 125

Arg Ala Val Trp Ala Leu Arg Ala Val Ser His Tyr Val Val Gly Ser
    130                 135                 140

Val Leu Glu Gln Gln Ala Ser Asp Ala Asp Glu Arg Val Pro Asp Arg
145                 150                 155                 160

Pro Asp Val Ser Glu Gln Ala Pro Ser Ser Phe Leu His Asp Leu Phe
                165                 170                 175

His Glu Leu Glu Thr Asp Gly Met Asp Ala Ala Phe Asn Phe Gly Leu
            180                 185                 190

Asp Ser Leu Ile Ala Gly Phe Glu Arg Leu Arg Ser Ser Thr Thr Asp
        195                 200                 205

<210>  39

<211>  207

<212>  PRT

<213>  Escherichia coli

<400>  39

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Pro Asn Glu Thr Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Glu Ser Gln Phe Glu Thr
            100                 105                 110

Ser Glu Gln Gln Leu Gln Phe Leu Cys Asp Ala Gly Phe Ser Leu Ser
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Glu Thr Asp Thr Val Ala Tyr Pro Pro Leu Leu Thr Gln Ala Val
                165                 170                 175

Ala Ile Met Asp Ser Asp Asn Gly Asp Ala Ala Phe Leu Phe Val Leu
            180                 185                 190

Asp Val Met Ile Ser Gly Leu Glu Thr Val Leu Lys Ser Ala Lys
        195                 200                 205

<210>  40

<211>  207

<212>  PRT

<213>  Escherichia coli

<400>  40

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Ala Asn Glu Ser Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Ala Asn Gln Phe Glu Thr
            100                 105                 110

Ser Glu Gln Gln Leu Gln Phe Leu Cys Asp Ala Gly Phe Thr Leu Thr
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Pro Lys Thr Glu Ile Asn Tyr Pro Pro Leu Leu Thr Gln Ala Ile
                165                 170                 175

Asp Ile Met Asp Ser Asp Asn Gly Glu Ala Ala Phe Leu Phe Val Leu
            180                 185                 190

Asp Val Met Ile Ser Gly Leu Glu Thr Val Leu Asn Asn His His
        195                 200                 205

<210>  41

<211>  207

<212>  PRT

<213>  Escherichia coli

<400>  41

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Ala Asn Glu Ser Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Ala Asn Gln Phe Glu Thr
            100                 105                 110

Ser Glu Gln Gln Leu Gln Phe Leu Cys Asp Ala Gly Phe Thr Leu Thr
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Pro Lys Thr Glu Ile Asn Tyr Pro Pro Leu Leu Thr Gln Ala Ile
                165                 170                 175

Asp Ile Met Asp Ser Asp Asn Gly Glu Ala Ala Phe Leu Phe Val Leu
            180                 185                 190

Asp Val Met Ile Ser Gly Leu Glu Thr Val Leu Asn Asn His His
        195                 200                 205

<210>  42

<211>  208

<212>  PRT

<213>  Escherichia coli

<400>  42

Met Ser Arg Leu Asp Lys Ser Lys Val Ile Asn Ser Ala Leu Glu Leu
1               5                   10                  15

Val Asn Glu Phe Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  43

<211>  208

<212>  PRT

<213>  Escherichia coli

<400>  43

Met Ser Arg Leu Asp Lys Ser Lys Val Ile Asn Ser Ala Leu Ala Leu
1               5                   10                  15

Val Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  44

<211>  208

<212>  PRT

<213>  Escherichia coli

<400>  44

Met Ser Arg Leu Asp Lys Ser Lys Val Ile Asn Ser Ala Leu Glu Leu
1               5                   10                  15

Leu Tyr Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  45

<211>  208

<212>  PRT

<213>  Escherichia coli

<400>  45

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ser Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  46

<211>  208

<212>  PRT

<213>  Escherichia coli

<400>  46

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Val Leu Ala Arg Gln Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  47

<211>  208

<212>  PRT

<213>  Escherichia coli

<400>  47

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Leu Met Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  48

<211>  208

<212>  PRT

<213>  Escherichia coli

<400>  48

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg His Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  49

<211>  208

<212>  PRT

<213>  Escherichia coli

<400>  49

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Val
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 50

<211> 208

<212> PRT

<213> Escherichia coli

<400> 50

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Asn Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 51

<211> 208

<212> PRT

<213> Escherichia coli

<400> 51

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Ser Asn Ala Met Ser Phe Arg Arg Ala Leu Leu Arg Tyr Arg Ala Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val

195                 200                 205

<210>  52

<211>  208

<212>  PRT

<213>  Escherichia coli

<400>  52

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Cys Cys Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  53

<211>  208

<212>  PRT

<213>  Escherichia coli

<400>  53

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Cys Arg Asp Gly
                85                  90                  95

Ala Lys Met His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  54

<211>  208

<212>  PRT

<213>  Escherichia coli

<400>  54

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Ser Asp Gly
                85                  90                  95

Ala Ile Val Gln Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  55

<211>  208

<212>  PRT

<213>  Escherichia coli

<400>  55

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr His Asp Gly
                85                  90                  95

Ala Ile Leu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser

180                 185                 190

Leu Ile Arg Gly Phe Glu Val Gln Leu Thr Ala Leu Leu Gln Ile Val
        195                 200                 205

<210>  56

<211>  208

<212>  PRT

<213>  Escherichia coli

<400>  56

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  57

<211>  208

<212>  PRT

<213>  Escherichia coli

<400>  57

Met Ser Arg Leu Asp Lys Ser Lys Val Ile Asn Ser Ala Leu Glu Leu
1               5                   10                  15

Ser Asn Glu Val Gly Ile Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  58

<211>  208

<212>  PRT

<213>  Escherichia coli

<400>  58

Met Ser Arg Leu Asp Lys Ser Lys Val Ile Asn Ser Ala Leu Glu Leu
1               5                   10                  15

Leu Lys Asp Leu Gly Asn Ala Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  59

<211>  208

<212>  PRT

<213>  Escherichia coli

<400>  59

Met Ser Arg Leu Asp Lys Ser Lys Val Ile Asn Ser Ala Leu Glu Leu
1               5                   10                  15

Leu Ile Gly Gly Gly Ile Val Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile

165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  60

<211>  208

<212>  PRT

<213>  Escherichia coli

<400>  60

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  61

<211>  208

<212>  PRT

<213>  Escherichia coli

<400>  61

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 62

<211> 208

<212> PRT

<213> Escherichia coli

<400> 62

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  63

<211>  208

<212>  PRT

<213>  Escherichia coli

<400>  63

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala

145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  64

<211>  208

<212>  PRT

<213>  Escherichia coli

<400>  64

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Val His Leu Gly Ser Arg Pro Asp Glu Lys Gln Tyr Asp Thr
            100                 105                 110

Val Val Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  65

<211>  208

<212>  PRT

<213>  Escherichia coli

<400>  65

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asn Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val His Leu Thr Ala Leu Leu Gln Ile Val
        195                 200                 205

<210>  66

<211>  208

<212>  PRT

<213>  Escherichia coli

<400>  66

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  67

<211>  208

<212>  PRT

<213>  Escherichia coli

<400>  67

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala

130                 135                 140

Val Leu Glu Gln Gln Glu His Thr Ala Ala Leu Thr Asp Arg Pro Val
145                 150                 155                 160

Ala Pro Asp Glu Asn Leu Pro Pro Leu Leu Arg Glu Ala Leu Gln Ile
                165                 170                 175

Met Val Ser Asp Asp Gly Glu Gln Ala Phe Leu His Gly Leu Glu Ser
            180                 185                 190

Leu Ile Arg Trp Phe Glu Val Gln Leu Thr Ala Leu Leu Gln Ile Val
        195                 200                 205

<210>  68

<211>  208

<212>  PRT

<213>  Escherichia coli

<400>  68

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Asn Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Glu Gly
                85                  90                  95

Ala Lys Val Ala Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  69

<211>  208

<212>  PRT

<213>  Escherichia coli

<400>  69

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu Gln Gly Leu Glu Ser
            180                 185                 190

Leu Ile Arg Gly Phe Glu Val Gln Leu Thr Ala Leu Leu Gln Ile Val
        195                 200                 205

<210>  70

<211>  208

<212>  PRT

<213>  Escherichia coli

<400>  70

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Ser Gln Ile Val
        195                 200                 205

<210>  71

<211>  208

<212>  PRT

<213>  Escherichia coli

<400>  71

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Phe Asp Thr
            100                 105                 110

Val Glu Thr Gln Leu Arg Phe Met Thr Glu Asn Gly Phe Ser Leu Arg

115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  72

<211>  208

<212>  PRT

<213>  Escherichia coli

<400>  72

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Val Arg Gly Phe Glu Val Gln Leu Thr Ala Leu Leu Gln Ile Val
        195                 200                 205

<210>  73

<211>  208

<212>  PRT

<213>  Escherichia coli

<400>  73

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Cys Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  74

<211>  208

<212>  PRT

<213>  Escherichia coli

<400>  74

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Val Ala Gly Glu Ser Trp Gln Ser Phe Leu Arg
65                  70                  75                  80

Asn Asn Ala Met Ser Phe Arg Arg Ala Leu Gln Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln 'Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  75

<211>  208

<212>  PRT

<213>  Escherichia coli

<400>  75

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Val His Leu Gly Thr Arg Pro Asp Glu Lys Gln Tyr Asp Thr

            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 76

<211> 208

<212> PRT

<213> Escherichia coli

<400> 76

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 77

<211> 208

<212> PRT

<213> Escherichia coli

<400> 77

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Val Gly Glu Ser Trp Gln Ser Phe Leu Arg
65                  70                  75                  80

Asn Asn Ala Met Ser Phe Arg Arg Ala Leu Leu Arg Tyr Arg Gly Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Arg Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  78

<211>  208

<212>  PRT

<213>  Escherichia coli

<400>  78

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Asn Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Arg Val His His Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Gly
            180                 185                 190

Leu Ile Arg Gly Phe Glu Val Gln Leu Thr Ala Leu Leu Gln Ile Val
        195                 200                 205

<210>  79

<211>  208

<212>  PRT

<213>  Escherichia coli

<400>  79

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ser Leu Leu Phe Ala Leu Gly Ile Glu Ile Leu Ala Arg Gln His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly

85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  80

<211>  208

<212>  PRT

<213>  Escherichia coli

<400>  80

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Asn Phe Gly Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  81

<211>  208

<212>  PRT

<213>  Escherichia coli

<400>  81

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Val Glu Ile Leu Ala Ser His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  82

<211>  208

<212>  PRT

<213>  Escherichia coli

<400>  82

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Asn Thr Phe Ala Arg Tyr His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  83

<211>  208

<212>  PRT

<213>  Escherichia coli

<400>  83

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Gln Ala Leu Ala Val Lys Ile Leu Val Pro Asp His
    50                  55                  60

Asp Tyr Ser Leu Pro Ala Ala Gly Glu Ser Trp Gln Ser Phe Leu Arg

65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  84

<211>  208

<212>  PRT

<213>  Escherichia coli

<400>  84

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asn Gly Leu Ala Phe Glu Ser Phe Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  85

<211>  208

<212>  PRT

<213>  Escherichia coli

<400>  85

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asp Ala Leu Pro Leu Val Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  86

<211>  208

<212>  PRT

<213>  Escherichia coli

<400>  86

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

His Ala Leu Leu Asp Ala Leu Pro Leu Glu Ile Met Ala Arg Gln His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  87

<211>  208

<212>  PRT

<213>  Escherichia coli

<400>  87

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Gly Ala Leu Leu Asp Ala Leu Ala Val Asn Ile Leu Ala Leu Gln His

50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  88

<211>  208

<212>  PRT

<213>  Escherichia coli

<400>  88

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Lys Ile Val Thr Gly His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  89

<211>  208

<212>  PRT

<213>  Escherichia coli

<400>  89

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Phe Ala Leu Ala Leu Glu Ser Leu Ser Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  90

<211>  208

<212>  PRT

<213>  Escherichia coli

<400>  90

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Asp Val Gln Asp Ala Leu Ala Val Glu Ile Leu Ser Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  91

<211>  208

<212>  PRT

<213>  Escherichia coli

<400>  91

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys

35                  40                  45

Gln Ala Leu Leu Asp Ala Leu Val Ala Gln Ile Leu Ala Arg Gln His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  92

<211>  208

<212>  PRT

<213>  Escherichia coli

<400>  92

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Leu Val Thr Phe Ala Ser His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  93

<211>  208

<212>  PRT

<213>  Escherichia coli

<400>  93

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Val Ala Leu Ala Val Lys Ile Val Ala Gly His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  94

<211>  208

<212>  PRT

<213>  Escherichia coli

<400>  94

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Glu Ser Leu Ala Val Glu Met Leu Pro Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  95

<211>  208

<212>  PRT

<213>  Escherichia coli

<400>  95

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln

20                  25                  30

Lys Leu Gly Val Glu Gln Pro Thr Leu Tyr Trp His Val Lys Asn Lys
        35                  40                  45

Arg Ser Leu Leu Tyr Ala Leu Gly Ile Glu Ile Leu Ala Arg Gln His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  96

<211>  208

<212>  PRT

<213>  Escherichia coli

<400>  96

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Glu Ser Leu Ala Val Glu Ile Leu Pro Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  97

<211>  208

<212>  PRT

<213>  Escherichia coli

<400>  97

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ser Leu Ala Ser His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  98

<211>  208

<212>  PRT

<213>  Escherichia coli

<400>  98

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Val Glu Ile Leu Gly Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  99

<211>  208

<212>  PRT

<213>  Escherichia coli

<400>  99

Met Ser Arg Leu Asp Lys Ser Lys Val Ile Asn Ser Ala Leu Glu Leu

1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Val Asp Ala Leu Val Leu Glu Thr Leu Ser Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  100

<211>  208

<212>  PRT

<213>  Escherichia coli

<400>  100

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Met Cys Val Glu Ile Phe Ala Arg Asn His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  101

<211>  208

<212>  PRT

<213>  Escherichia coli

<400>  101

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Ala Asp Leu Ala Arg Tyr His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  102

<211>  208

<212>  PRT

<213>  Escherichia coli

<400>  102

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asn Ser Leu Pro Pro Glu Ile Leu Ala Leu His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  103

<211>  208

<212>  PRT

<213>  Escherichia coli

<400>  103

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Phe Leu Ser Arg His Gln
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Thr Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  104

<211>  208

<212>  PRT

<213>  Escherichia coli

<400>  104

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Phe Thr Met Ala Val Glu Ser Leu Ala Arg Gln His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  105

<211>  208

<212>  PRT

<213>  Escherichia coli

<400>  105

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Phe Ala Met Ala Val Glu Ser Leu Ala Arg Gln His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  106

<211>  208

<212>  PRT

<213>  Escherichia coli

<400>  106

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Pro Val Glu Ile Leu Ala Leu His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  107

<211>  208

<212>  PRT

<213>  Escherichia coli

<400>  107

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Val Val Glu His Leu Ala Leu His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  108

<211>  208

<212>  PRT

<213>  Escherichia coli

<400>  108

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Gly Val Gly Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  109

<211>  208

<212>  PRT

<213>  Escherichia coli



<400>  109

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Arg Leu Glu Arg Asn His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  110

<211>  208

<212>  PRT

<213>  Escherichia coli

<400>  110

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Ala Ala Leu Ala Val Glu Ile Leu Pro Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  111

<211>  208

<212>  PRT

<213>  Escherichia coli

<400>  111

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Thr Ala Met Pro Val Glu Ile Leu Ser Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  112

<211>  208

<212>  PRT

<213>  Escherichia coli

<400>  112

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asp Ala Met Pro Leu Val Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu Tyr Gly Leu Glu Ser
            180                 185                 190

Leu Ile Arg Gly Phe Glu Val Gln Leu Thr Ala Leu Leu Gln Ile Val
        195                 200                 205

<210>  113

<211>  208

<212>  PRT

<213>  Escherichia coli

<400>  113

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Leu Val Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  114

<211>  208

<212>  PRT

<213>  Escherichia coli

<400>  114

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Glu Ser Leu Ala Val Glu Ile Leu Arg Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val

195                 200                 205

<210>  115

<211>  208

<212>  PRT

<213>  Escherichia coli

<400>  115

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Thr Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  116

<211>  208

<212>  PRT

<213>  Escherichia coli

<400>  116

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Tyr Asp Thr
            100                 105                 110

Ala Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  117

<211>  208

<212>  PRT

<213>  Escherichia coli

<400>  117

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Val His Ile Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  118

<211>  208

<212>  PRT

<213>  Escherichia coli

<400>  118

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Gly Asp Trp
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser

180                 185                 190

Leu Ile Arg Gly Phe Glu Val Gln Leu Thr Ala Leu Leu Gln Ile Val
        195                 200                 205

<210>  119

<211>  208

<212>  PRT

<213>  Escherichia coli

<400>  119

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gln
                85                  90                  95

Ala Lys Val His Leu Ser Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  120

<211>  208

<212>  PRT

<213>  Escherichia coli

<400>  120

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Glu Gly
                85                  90                  95

Glu Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  121

<211>  208

<212>  PRT

<213>  Escherichia coli

<400>  121

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Ser Arg Glu Gly
                85                  90                  95

Ala Lys Val His Leu Ala Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  122

<211>  208

<212>  PRT

<213>  Escherichia coli

<400>  122

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Phe Arg Asp Trp
                85                  90                  95

Glu Lys Val Asn Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile

165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  123

<211>  208

<212>  PRT

<213>  Escherichia coli

<400>  123

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Thr Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  124

<211>  208

<212>  PRT

<213>  Escherichia coli

<400>  124

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ser Lys Leu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  125

<211>  208

<212>  PRT

<213>  Escherichia coli

<400>  125

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Thr Arg Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  126

<211>  208

<212>  PRT

<213>  Escherichia coli

<400>  126

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Gly Pro Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala

145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  127

<211>  208

<212>  PRT

<213>  Escherichia coli

<400>  127

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Val Arg
65                  70                  75                  80

Asn Asn Ala Met Ser Phe Arg Arg Ala Leu Leu Arg Tyr Arg Asp Gly
                85                  90                  95

Thr Arg Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  128

<211>  208

<212>  PRT

<213>  Escherichia coli

<400>  128

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Leu Ala Gly
                85                  90                  95

Ala Lys Val Gln Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  129

<211>  208

<212>  PRT

<213>  Escherichia coli

<400>  129

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Tyr Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  130

<211>  208

<212>  PRT

<213>  Escherichia coli

<400>  130

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg His Arg Asp Trp
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala

130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  131

<211>  208

<212>  PRT

<213>  Escherichia coli

<400>  131

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Asn Arg Asp Trp
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  132

<211>  208

<212>  PRT

<213>  Escherichia coli

<400>  132

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Arg His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  133

<211>  208

<212>  PRT

<213>  Escherichia coli

<400>  133

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Thr Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  134

<211>  208

<212>  PRT

<213>  Escherichia coli

<400>  134

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Pro Arg Asp His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg

115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  135

<211>  208

<212>  PRT

<213>  Escherichia coli

<400>  135

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Phe Arg Asn Gly
                85                  90                  95

Ala Lys Phe His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  136

<211>  208

<212>  PRT

<213>  Escherichia coli

<400>  136

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  137

<211>  208

<212>  PRT

<213>  Escherichia coli

<400>  137

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Gly Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  138

<211>  208

<212>  PRT

<213>  Escherichia coli

<400>  138

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ser Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Arg His Leu Gly Thr Arg Pro Asp Glu Lys Gln Tyr Asp Thr

            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 139

<211> 208

<212> PRT

<213> Escherichia coli

<400> 139

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Ala Val Glu Ile Leu Pro Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  140

<211>  208

<212>  PRT

<213>  Escherichia coli

<400>  140

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Val Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Val Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 141

<211> 208

<212> PRT

<213> Escherichia coli

<400> 141

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ser Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  142

<211>  208

<212>  PRT

<213>  Escherichia coli

<400>  142

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ser Val Glu Ala Trp Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly

85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  143

<211>  208

<212>  PRT

<213>  Escherichia coli

<400>  143

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Leu His Ile Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  144

<211>  208

<212>  PRT

<213>  Escherichia coli

<400>  144

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Thr Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Arg His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  145

<211>  208

<212>  PRT

<213>  Escherichia coli

<400>  145

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ser Leu Ala Gly His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Gly
                85                  90                  95

Ala Lys Leu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  146

<211>  208

<212>  PRT

<213>  Escherichia coli

<400>  146

Met Ser Arg Leu Asp Lys Ser Lys Val Ile Asn Ser Ala Leu Glu Leu
1               5                   10                  15

Ser Asn Glu Val Gly Ile Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg

65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  147

<211>  208

<212>  PRT

<213>  Escherichia coli

<400>  147

Met Ser Arg Leu Asp Lys Ser Lys Val Ile Asn Ser Ala Leu Glu Leu
1               5                   10                  15

His Asn Glu Val Gly Phe Gly Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  148

<211>  208

<212>  PRT

<213>  Escherichia coli

<400>  148

Met Ser Arg Leu Asp Lys Ser Lys Val Ile Asn Ser Ala Leu Glu Leu
1               5                   10                  15

Leu Lys Asp Leu Gly Asn Ala Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  149

<211>  208

<212>  PRT

<213>  Escherichia coli

<400>  149

Met Ser Arg Leu Asp Lys Ser Lys Val Ile Asn Ser Ala Leu Val Leu
1               5                   10                  15

Ala Asn Asp Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  150

<211>  207

<212>  PRT

<213>  Escherichia coli

<400>  150

Met Ser Arg Leu Asp Lys Ser Lys Val Ile Asn Ser Ala Leu Glu Leu
1               5                   10                  15

Phe Asn Val Gly Ile Glu Gly Leu Thr Thr Arg Lys Leu Ala Gln Lys
            20                  25                  30

Leu Gly Val Glu Gln Pro Thr Leu Tyr Trp His Val Lys Asn Lys Arg
        35                  40                  45

Ala Leu Leu Asp Ala Leu Ala Val Glu Ile Leu Ala Arg His His Asp

50                  55                  60

Tyr Ser Leu Pro Ala Ala Gly Glu Ser Trp Gln Ser Phe Leu Arg Asn
65                  70                  75                  80

Asn Ala Met Ser Phe Arg Arg Ala Leu Leu Arg Tyr Arg Asp Gly Ala
                85                  90                  95

Lys Val His Leu Gly Thr Arg Pro Asp Glu Lys Gln Tyr Asp Thr Val
            100                 105                 110

Glu Thr Gln Leu Arg Phe Met Thr Glu Asn Gly Phe Ser Leu Arg Asp
        115                 120                 125

Gly Leu Tyr Ala Ile Ser Ala Val Ser His Phe Thr Leu Gly Ala Val
    130                 135                 140

Leu Glu Gln Gln Glu His Thr Ala Ala Leu Thr Asp Arg Pro Ala Ala
145                 150                 155                 160

Pro Asp Glu Asn Leu Pro Pro Leu Leu Arg Glu Ala Leu Gln Ile Met
                165                 170                 175

Asp Ser Asp Asp Gly Glu Gln Ala Phe Leu His Gly Leu Glu Ser Leu
            180                 185                 190

Ile Arg Gly Phe Glu Val Gln Leu Thr Ala Leu Leu Gln Ile Val
        195                 200                 205

<210>  151

<211>  208

<212>  PRT

<213>  Escherichia coli

<400>  151

Met Ser Arg Leu Asp Lys Ser Lys Val Ile Asn Ser Ala Ser Glu Leu
1               5                   10                  15

Leu Tyr Glu Val Gly Phe Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  152

<211>  208

<212>  PRT

<213>  Escherichia coli

<400>  152

Met Ser Arg Leu Asp Lys Ser Lys Val Ile Asn Ser Ala Leu Ala Leu
1               5                   10                  15

Gly Asn Glu Val Gly Ile Glu Gly Val Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  153

<211>  208

<212>  PRT

<213>  Escherichia coli

<400>  153

Met Ser Arg Leu Asp Lys Ser Lys Val Ile Asn Ser Ala Ser Glu Leu
1               5                   10                  15

Leu Lys Glu Val Gly Ile Val Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  154

<211>  208

<212>  PRT

<213>  Escherichia coli

<400>  154

Met Ser Arg Leu Asp Lys Ser Lys Val Ile Asn Ser Ala Thr Glu Leu
1               5                   10                  15

Leu Tyr Glu Val Gly Phe Glu Gly Leu Thr Thr Arg Lys Leu Ala Gln
            20                  25                  30

Lys Leu Gly Val Glu Gln Pro Thr Leu Tyr Trp His Val Lys Asn Lys

35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  155

<211>  208

<212>  PRT

<213>  Escherichia coli

<400>  155

Met Ser Arg Leu Asp Lys Ser Lys Val Ile Asn Ser Ala Leu Glu Leu
1               5                   10                  15

Gly Asn Glu Val Gly Met Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  156

<211>  208

<212>  PRT

<213>  Escherichia coli

<400>  156

Met Ser Arg Leu Asp Lys Ser Lys Val Ile Asn Ser Ala Leu Glu Leu
1               5                   10                  15

Leu Asn Glu Gly Ala Ile Gly Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  157

<211>  208

<212>  PRT

<213>  Escherichia coli

<400>  157

Met Ser Arg Leu Asp Lys Ser Lys Val Ile Asn Ser Ala Leu Glu Leu
1               5                   10                  15

Leu Asn Glu Gly Arg Asn Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  158

<211>  208

<212>  PRT

<213>  Escherichia coli

<400>  158

Met Ser Arg Leu Asp Lys Ser Lys Val Ile Asn Ser Ala Leu Glu Leu
1               5                   10                  15

Leu Ile Gly Gly Gly Ile Val Gly Leu Thr Thr Arg Lys Leu Ala Gln

20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  159

<211>  208

<212>  PRT

<213>  Escherichia coli

<400>  159

Met Ser Arg Leu Asp Lys Ser Lys Val Ile Asn Ser Ala Leu Glu Leu
1               5                   10                  15

Leu Asp Glu Arg Gly Asn Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  160

<211>  208

<212>  PRT

<213>  Escherichia coli

<400>  160

Met Ser Arg Leu Asp Lys Ser Lys Val Ile Asn Ser Ala Leu Glu Leu
1               5                   10                  15

Leu Tyr Glu Asp Gly Thr Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Ser Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  161

<211>  208

<212>  PRT

<213>  Escherichia coli

<400>  161

Met Ser Arg Leu Asp Lys Ser Lys Val Ile Asn Ser Ala Leu Glu Leu
1               5                   10                  15

Val Tyr Glu Val Gly Thr Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  162

<211>  208

<212>  PRT

<213>  Escherichia coli

<400>  162

Met Ser Arg Leu Asp Lys Ser Lys Val Ile Asn Ser Ala Leu Glu Leu

1               5                   10                  15

Ser Asn Glu Val Gly Ile Asp Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  163

<211>  208

<212>  PRT

<213>  Escherichia coli

<400>  163

Met Ser Arg Leu Asp Lys Ser Lys Val Ile Asn Ser Ala Leu Val Leu
1               5                   10                  15

Phe Tyr Glu Val Gly Met Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  164

<211>  205

<212>  PRT

<213>  Escherichia coli

<400>  164

Met Ser Arg Leu Asp Lys Ser Lys Val Ile Asn Ser Ala Leu Glu Leu
1               5                   10                  15

Leu Asn Glu Val Ser Leu Thr Thr Arg Lys Leu Ala Gln Lys Leu Gly
            20                  25                  30

Val Glu Gln Pro Thr Leu Tyr Trp His Val Lys Asn Lys Arg Ala Leu
        35                  40                  45

Leu Asp Ala Leu Ala Val Glu Ile Leu Ala Arg His His Asp Tyr Ser
    50                  55                  60

Leu Pro Ala Ala Gly Glu Ser Trp Gln Ser Phe Leu Arg Asn Asn Ala
65                  70                  75                  80

Met Ser Phe Arg Arg Ala Leu Leu Arg Tyr Arg Asp Gly Ala Lys Val
                85                  90                  95

His Leu Gly Thr Arg Pro Asp Glu Lys Gln Tyr Asp Thr Val Glu Thr
            100                 105                 110

Gln Leu Arg Phe Met Thr Glu Asn Gly Phe Ser Leu Arg Asp Gly Leu
        115                 120                 125

Tyr Ala Ile Ser Ala Val Ser His Phe Thr Leu Gly Ala Val Leu Glu
    130                 135                 140

Gln Gln Glu His Thr Ala Ala Leu Thr Asp Arg Pro Ala Ala Pro Asp
145                 150                 155                 160

Glu Asn Leu Pro Pro Leu Leu Arg Glu Ala Leu Gln Ile Met Asp Ser
                165                 170                 175

Asp Asp Gly Glu Gln Ala Phe Leu His Gly Leu Glu Ser Leu Ile Arg
            180                 185                 190

Gly Phe Glu Val Gln Leu Thr Ala Leu Leu Gln Ile Val
        195                 200                 205

<210>  165

<211>  208

<212>  PRT

<213>  Escherichia coli

<400>  165

Met Ser Arg Leu Asp Lys Ser Lys Val Ile Asn Ser Ala Glu Glu Leu
1               5                   10                  15

Leu Asn Glu Gly Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  166

<211>  208

<212>  PRT

<213>  Escherichia coli

<400>  166

Met Ser Arg Leu Asp Lys Ser Lys Val Ile Asn Ser Ala Leu Asp Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  167

<211>  208

<212>  PRT

<213>  Escherichia coli

<400>  167

Met Ser Arg Leu Asp Lys Ser Lys Val Ile Asn Ser Ala Val Glu Leu
1               5                   10                  15

Val Lys Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  168

<211>  208

<212>  PRT

<213>  Escherichia coli

<400>  168

Met Ser Arg Leu Asp Lys Ser Lys Val Ile Asn Ser Ala Leu Glu Leu
1               5                   10                  15

Leu Tyr Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Ala Val Glu Thr Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  169

<211>  208

<212>  PRT

<213>  Escherichia coli

<400>  169

Met Ser Arg Leu Asp Lys Ser Lys Val Ile Asn Ser Ala Leu Glu Leu
1               5                   10                  15

Leu Tyr Glu Val Gly Ile Val Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Ala Ser Leu Ser Val Glu Ile Leu Thr Arg Tyr His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  170

<211>  208

<212>  PRT

<213>  Escherichia coli

<400>  170

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Asn Arg Asp Gly
                85                  90                  95

Ala Lys Val His Phe Asp Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  171

<211>  208

<212>  PRT

<213>  Escherichia coli

<400>  171

Met Ser Arg Leu Asp Lys Ser Lys Val Ile Asn Ser Ala Ile Glu Leu
1               5                   10                  15

Leu Asn Glu Val Gly Leu Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr His Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  172

<211>  208

<212>  PRT

<213>  Escherichia coli

<400>  172

Met Ser Arg Leu Asp Lys Ser Lys Val Ile Asn Ser Ala Arg Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg His Gly
                85                  90                  95

Ala Lys Val His Pro Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  173

<211>  208

<212>  PRT

<213>  Escherichia coli

<400>  173

Met Ser Arg Leu Asp Lys Ser Lys Val Ile Asn Ser Ala Leu Glu Leu
1               5                   10                  15

Phe Arg Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asn Ala Leu Ala Val Glu Met Leu Thr Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  174

<211>  208

<212>  PRT

<213>  Escherichia coli

<400>  174

Met Ser Arg Leu Asp Lys Ser Lys Val Ile Asn Ser Ala Leu Glu Leu
1               5                   10                  15

Leu Tyr Glu Val Glu Ile Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  175

<211>  208

<212>  PRT

<213>  Escherichia coli

<400>  175

Met Ser Arg Leu Asp Lys Ser Lys Val Ile Asn Ser Ala Leu Glu Leu
1               5                   10                  15

Leu Asn Glu Val Gly Ile His Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Ala Val Gln Thr Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  176

<211>  208

<212>  PRT

<213>  Escherichia coli

<400>  176

Met Ser Arg Leu Asp Lys Ser Lys Val Ile Asn Ser Ala Leu Glu Leu
1               5                   10                  15

Leu Asn Asp Glu Gly Ile Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Leu Glu Asn Phe Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  177

<211>  208

<212>  PRT

<213>  Escherichia coli

<400>  177

Met Ser Arg Leu Asp Lys Ser Lys Val Ile Asn Ser Ala Ile Glu Leu
1               5                   10                  15

Leu Asn Glu Val Arg Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Val Leu Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val

195                 200                 205

<210>  178

<211>  208

<212>  PRT

<213>  Escherichia coli

<400>  178

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Arg
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  179

<211>  208

<212>  PRT

<213>  Escherichia coli

<400>  179

Met Ser Arg Leu Asp Lys Ser Lys Val Ile Asn Ser Ala Leu Glu Leu
1               5                   10                  15

Leu Lys Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Val Val
                85                  90                  95

Val Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  180

<211>  208

<212>  PRT

<213>  Escherichia coli

<400>  180

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Val Tyr Ile Leu Ala Arg Tyr His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  181

<211>  208

<212>  PRT

<213>  Escherichia coli

<400>  181

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Cys Arg Glu Gly
                85                  90                  95

Thr Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser

180                 185                 190

Leu Ile Arg Gly Phe Glu Val Gln Leu Thr Ala Leu Leu Gln Ile Val
        195                 200                 205

<210>  182

<211>  208

<212>  PRT

<213>  Escherichia coli

<400>  182

Met Ser Arg Leu Asp Lys Ser Lys Val Ile Asn Ser Ala Phe Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Val
                85                  90                  95

Ala Glu Leu His Pro Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  183

<211>  208

<212>  PRT

<213>  Escherichia coli

<400>  183

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Cys Arg Asp Arg
                85                  90                  95

Ala Asn Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
                180                 185                 190

Leu Ile Arg Gly Phe Glu Val Gln Leu Thr Ala Leu Leu Gln Ile Val
            195                 200                 205

<210>  184

<211>  208

<212>  PRT

<213>  Escherichia coli

<400>  184

Met Ser Arg Leu Asp Lys Ser Lys Val Ile Asn Ser Ala Val Val Leu
1               5                   10                  15

Leu Asn Glu Val Gly Ile Asp Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Leu
                85                  90                  95

Ala Lys Val His Leu Ala Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 185

<211> 208

<212> PRT

<213> Escherichia coli

<400> 185

Met Ser Arg Leu Asp Lys Ser Lys Val Ile Asn Ser Ala Leu Glu Leu
1               5                   10                  15

Leu Lys Val Val Gly Thr Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ser Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile

                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 186

<211> 208

<212> PRT

<213> Escherichia coli

<400> 186

Met Ser Arg Leu Asp Lys Ser Lys Val Ile Asn Ser Ala Leu Glu Leu
1               5                   10                  15

Leu Asn Glu Gly Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Val Glu Leu Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Thr Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  187

<211>  208

<212>  PRT

<213>  Escherichia coli

<400>  187

Met Ser Arg Leu Asp Lys Ser Lys Val Ile Asn Ser Ala Leu Glu Leu
1               5                   10                  15

Met Asn Asp Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Glu Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  188

<211>  208

<212>  PRT

<213>  Escherichia coli

<400>  188

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Leu Asn Cys Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 189

<211> 208

<212> PRT

<213> Escherichia coli

<400> 189

Met Ser Arg Leu Asp Lys Ser Lys Val Ile Asn Ser Ala Phe Ala Leu
1               5                   10                  15

Val Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Val
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala

145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  190

<211>  208

<212>  PRT

<213>  Escherichia coli

<400>  190

Met Ser Arg Leu Asp Lys Ser Lys Val Ile Asn Ser Ala Leu Glu Leu
1               5                   10                  15

Leu Asn Glu Gly Gly Asn Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Gln Thr Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  191

<211>  208

<212>  PRT

<213>  Escherichia coli

<400>  191

Met Ser Arg Leu Asp Lys Ser Lys Val Ile Asn Ser Ala Leu Glu Leu
1               5                   10                  15

Leu Asn Val Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Cys Arg Tyr Gly
                85                  90                  95

Thr Asn Leu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  192

<211>  208

<212>  PRT

<213>  Escherichia coli

<400>  192

Met Ser Arg Leu Asp Lys Ser Lys Val Ile Asn Ser Ala Leu Glu Leu
1               5                   10                  15

Leu Asn Glu Phe Arg Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Ser Thr Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  193

<211>  208

<212>  PRT

<213>  Escherichia coli

<400>  193

Met Ser Arg Leu Asp Lys Ser Lys Val Ile Asn Ser Ala Leu Gln Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Met Ala Val Glu Arg Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala

130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  194

<211>  206

<212>  PRT

<213>  Escherichia coli

<400>  194

Met Ser Arg Leu Asp Lys Ser Lys Val Ile Asn Ser Ala Leu Glu Leu
1               5                   10                  15

Leu Tyr Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Val Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Leu Arg Cys Ser Leu Arg His Cys Cys Lys
        195                 200                 205

<210>  195

<211>  208

<212>  PRT

<213>  Escherichia coli

<400>  195

Met Ser Arg Leu Asp Lys Ser Lys Val Ile Asn Ser Ala Val Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Asp Gly Gly Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  196

<211>  208

<212>  PRT

<213>  Escherichia coli

<400>  196

Met Ser Arg Leu Asp Lys Ser Lys Val Ile Asn Ser Ala Leu Glu Leu
1               5                   10                  15

Leu Asn Glu Val Gly Ile Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg His Gly
                85                  90                  95

Ala Lys Val His His Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  197

<211>  208

<212>  PRT

<213>  Escherichia coli

<400>  197

Met Ser Arg Leu Asp Lys Ser Lys Val Ile Asn Ser Ala Leu Glu Leu
1               5                   10                  15

His Asn Glu Val Gly Phe Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Pro Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg

115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  198

<211>  208

<212>  PRT

<213>  Escherichia coli

<400>  198

Met Ser Arg Leu Asp Lys Ser Lys Val Ile Asn Ser Ala Leu Val Leu
1               5                   10                  15

Leu His Asp Asp Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Pro His Gly
                85                  90                  95

Ala Lys Val Asn Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  199

<211>  208

<212>  PRT

<213>  Escherichia coli

<400>  199

Met Ser Arg Leu Asp Lys Ser Lys Val Ile Asn Ser Ala Leu Val Leu
1               5                   10                  15

Leu Asn Glu Ala Gly Phe Gln Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Val Glu Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  200

<211>  208

<212>  PRT

<213>  Escherichia coli

<400>  200

Met Ser Arg Leu Asp Lys Ser Lys Val Ile Asn Ser Ala Leu Glu Gln
1               5                   10                  15

Leu His Val Gly Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Thr
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  201

<211>  208

<212>  PRT

<213>  Escherichia coli

<400>  201

Met Ser Arg Leu Asp Lys Ser Lys Val Ile Asn Ser Ala Leu Glu Leu
1               5                   10                  15

Leu Asn Glu Val Gly Ile Glu Ala Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Pro Glu Gly
                85                  90                  95

Ala Lys Val His Leu Asp Thr Arg Pro Asp Glu Lys Gln Tyr Asp Thr

            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 202

<211> 208

<212> PRT

<213> Escherichia coli

<400> 202

Met Ser Arg Leu Asp Lys Ser Lys Val Ile Asn Ser Ala Leu Gly Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  203

<211>  208

<212>  PRT

<213>  Escherichia coli

<400>  203

Met Ser Arg Leu Asp Lys Ser Lys Val Ile Asn Ser Ala Leu Asp Leu
1               5                   10                  15

Ile Thr Glu Val Gly Ile Gly Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Val Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 204

<211> 208

<212> PRT

<213> Escherichia coli

<400> 204

Met Ser Arg Leu Asp Lys Ser Lys Val Ile Asn Ser Ala Val Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Thr Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  205

<211>  208

<212>  PRT

<213>  Escherichia coli

<400>  205

Met Ser Arg Leu Asp Lys Ser Lys Val Ile Asn Ser Ala Phe Glu Leu
1               5                   10                  15

Leu Asn Glu Val Ala Ile Met Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Met

85                  90                  95

Pro Lys Val His Pro Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  206

<211>  208

<212>  PRT

<213>  Escherichia coli

<400>  206

Met Ser Arg Leu Asp Lys Ser Lys Val Ile Asn Ser Ala Leu Glu Arg
1               5                   10                  15

Leu Asn Asp Val Gly Ile Asp Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Gly Gly
                85                  90                  95

Ala Lys Ala His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  207

<211>  208

<212>  PRT

<213>  Escherichia coli

<400>  207

Met Ser Arg Leu Asp Lys Ser Lys Val Ile Asn Ser Ala Leu Glu Leu
1               5                   10                  15

Phe Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Phe Ala Val His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  208

<211>  208

<212>  PRT

<213>  Escherichia coli

<400>  208

Met Ser Arg Leu Asp Lys Ser Lys Val Ile Asn Ser Ala Leu Glu Leu
1               5                   10                  15

Leu Asn Glu Gly Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asn Ala Leu Gly Leu Glu Phe Phe Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  209

<211>  208

<212>  PRT

<213>  Escherichia coli

<400>  209

Met Ser Arg Leu Asp Lys Ser Lys Val Ile Asn Ser Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Tyr Ala Leu Pro Val Glu Ile Leu Glu Arg His His
    50                  55                  60

Asp Tyr Ser Leu Pro Ala Ala Gly Glu Ser Trp Gln Ser Phe Leu Arg

65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  210

<211>  208

<212>  PRT

<213>  Escherichia coli

<400>  210

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Pro Asp Val
                85                  90                  95

Thr Asn Val Gln Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  211

<211>  208

<212>  PRT

<213>  Escherichia coli

<400>  211

Met Ser Arg Leu Asp Lys Ser Lys Val Ile Asn Ser Ala Leu Glu Gln
1               5                   10                  15

Leu Tyr Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Gly Lys Val Ser Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  212

<211>  208

<212>  PRT

<213>  Escherichia coli

<400>  212

Met Ser Arg Leu Asp Lys Ser Lys Val Ile Asn Ser Ala Leu Asp Leu
1               5                   10                  15

Leu Asn Val Val Gly Ile Lys Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr His Asn Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  213

<211>  208

<212>  PRT

<213>  Escherichia coli

<400>  213

Met Ser Arg Leu Asp Lys Ser Lys Val Ile Asn Ser Ala Phe Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His

50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ala Gly
                85                  90                  95

Ala Lys Gly His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  214

<211>  208

<212>  PRT

<213>  Escherichia coli

<400>  214

Met Ser Arg Leu Asp Lys Ser Lys Val Ile Asn Ser Ala Leu Gly Leu
1               5                   10                  15

Leu Lys Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr His Asp Gly
                85                  90                  95

Ala Lys Val His Leu Val Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  215

<211>  208

<212>  PRT

<213>  Escherichia coli

<400>  215

Met Ser Arg Leu Asp Lys Ser Lys Val Ile Asn Ser Ala Val Glu Leu
1               5                   10                  15

Phe Asn Glu Ala Gly Met Glu Gly Phe Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg His Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  216

<211>  208

<212>  PRT

<213>  Escherichia coli

<400>  216

Met Ser Arg Leu Asp Lys Ser Lys Val Ile Asn Ser Ala Leu Glu Leu
1               5                   10                  15

Leu His Glu Val Gly Ile Glu Gly Phe Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Pro Asn Val His His Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  217

<211>  208

<212>  PRT

<213>  Escherichia coli

<400>  217

Met Ser Arg Leu Asp Lys Ser Lys Val Ile Asn Ser Ala Leu Ala Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys

35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Met
                85                  90                  95

Pro Lys Val His Pro Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  218

<211>  208

<212>  PRT

<213>  Escherichia coli

<400>  218

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Pro Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  219

<211>  208

<212>  PRT

<213>  Escherichia coli

<400>  219

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Cys Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  220

<211>  208

<212>  PRT

<213>  Escherichia coli

<400>  220

Met Ser Arg Leu Asp Lys Ser Lys Val Ile Asn Ser Ala Leu Glu Leu
1               5                   10                  15

Leu Asn Glu Gly Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  221

<211>  208

<212>  PRT

<213>  Escherichia coli

<400>  221

Met Ser Arg Leu Asp Lys Ser Lys Val Ile Asn Ser Ala Leu Glu Leu
1               5                   10                  15

Leu Asn Val Ala Gly Ile Glu Gly Leu Thr Thr Arg Lys Leu Ala Gln

20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Ile Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  222

<211>  208

<212>  PRT

<213>  Escherichia coli

<400>  222

Met Ser Arg Leu Asp Lys Ser Lys Val Ile Asn Ser Ala Leu Glu Leu
1               5                   10                  15

Leu Asp Glu Val Gly Ile Ala Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asn Gly
                85                  90                  95

Ala Lys Val Pro Ser Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  223

<211>  208

<212>  PRT

<213>  Escherichia coli

<400>  223

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Pro Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 224

<211> 208

<212> PRT

<213> Escherichia coli

<400> 224

Met Ser Arg Leu Asp Lys Ser Lys Val Ile Asn Ser Ala Leu Glu Leu
1               5                   10                  15

Leu Asn Glu Val Gly Val Asp Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asn Gly
                85                  90                  95

Ala Lys Val Pro Ser Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  225

<211>  208

<212>  PRT

<213>  Escherichia coli

<400>  225

Met Ser Arg Leu Asp Lys Ser Lys Val Ile Asn Ser Ala Leu Glu Leu

1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Tyr Glu
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  226

<211>  208

<212>  PRT

<213>  Escherichia coli

<400>  226

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp His
                85                  90                  95

Ala Lys Arg His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  227

<211>  208

<212>  PRT

<213>  Escherichia coli

<400>  227

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Lys
                85                  90                  95

Ala Lys Ala His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  228

<211>  208

<212>  PRT

<213>  Escherichia coli

<400>  228

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Thr His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  229

<211>  208

<212>  PRT

<213>  Escherichia coli

<400>  229

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Pro
                85                  90                  95

Ala Lys Ser His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  230

<211>  208

<212>  PRT

<213>  Escherichia coli

<400>  230

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Ile
                85                  90                  95

Ala Lys Lys His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  231

<211>  208

<212>  PRT

<213>  Escherichia coli

<400> 231

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Asn
                85                  90                  95

Ala Lys Gln His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 232

<211> 208

<212> PRT

<213>  Escherichia coli

<400>  232

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Leu
                85                  90                  95

Ala Lys Lys His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  233

<211>  208

<212>  PRT

<213>  Escherichia coli

<400>  233

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Asn
                85                  90                  95

Ala Lys His His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  234

<211>  208

<212>  PRT

<213>  Escherichia coli

<400>  234

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp His
                85                  90                  95

Ala Lys Asn His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  235

<211>  208

<212>  PRT

<213>  Escherichia coli

<400>  235

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Asn
                85                  90                  95

Ala Lys Pro His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  236

<211>  208

<212>  PRT

<213>  Escherichia coli

<400>  236

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Tyr His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  237

<211>  208

<212>  PRT

<213>  Escherichia coli

<400>  237

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp His
                85                  90                  95

Ala Lys Gln His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  238

<211>  208

<212>  PRT

<213>  Escherichia coli

<400>  238

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Thr
                85                  90                  95

Ala Lys Asp His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  239

<211>  208

<212>  PRT

<213>  Escherichia coli

<400>  239

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Asn
                85                  90                  95

Ala Lys Asn His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  240

<211>  208

<212>  PRT

<213>  Escherichia coli

<400>  240

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Pro
                85                  90                  95

Ala Lys Pro His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val

195                 200                 205

<210>  241

<211>  208

<212>  PRT

<213>  Escherichia coli

<400>  241

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Pro
                85                  90                  95

Ala Lys Tyr His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  242

<211>  208

<212>  PRT

<213>  Escherichia coli

<400>  242

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Thr
                85                  90                  95

Ala Lys Lys His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  243

<211>  208

<212>  PRT

<213>  Escherichia coli

<400>  243

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Thr
                85                  90                  95

Ala Lys Pro His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  244

<211>  208

<212>  PRT

<213>  Escherichia coli

<400>  244

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Arg
                85                  90                  95

Ala Lys Ser His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser

180                 185                 190

Leu Ile Arg Gly Phe Glu Val Gln Leu Thr Ala Leu Leu Gln Ile Val
        195                 200                 205

<210>  245

<211>  208

<212>  PRT

<213>  Escherichia coli

<400>  245

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Ser
                85                  90                  95

Ala Lys Lys His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  246

<211>  208

<212>  PRT

<213>  Escherichia coli

<400>  246

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Pro
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  247

<211>  208

<212>  PRT

<213>  Escherichia coli

<400>  247

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Asp Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  248

<211>  208

<212>  PRT

<213>  Escherichia coli

<400>  248

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Arg Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile

165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  249

<211>  208

<212>  PRT

<213>  Escherichia coli

<400>  249

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Glu
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  250

<211>  208

<212>  PRT

<213>  Escherichia coli

<400>  250

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Lys Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  251

<211>  208

<212>  PRT

<213>  Escherichia coli

<400>  251

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Arg Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  252

<211>  208

<212>  PRT

<213>  Escherichia coli

<400>  252

Met Ser Arg Leu Asp Lys Ser Lys Val Ile Asn Ser Ala Leu Glu Leu
1               5                   10                  15

Gly Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala

145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  253

<211>  208

<212>  PRT

<213>  Escherichia coli

<400>  253

Met Ser Arg Leu Asp Lys Ser Lys Val Ile Asn Ser Ala Leu Glu Leu
1               5                   10                  15

Ser Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  254

<211>  208

<212>  PRT

<213>  Escherichia coli

<400>  254

Met Ser Arg Leu Asp Lys Ser Lys Val Ile Asn Ser Ala Leu Glu Leu
1               5                   10                  15

Ala Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  255

<211>  208

<212>  PRT

<213>  Escherichia coli

<400>  255

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Asp His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  256

<211>  208

<212>  PRT

<213>  Escherichia coli

<400>  256

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Lys His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala

130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  257

<211>  437

<212>  PRT

<213>  HeLa

<400>  257

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu
        435

<210>  258

<211>  212

<212>  PRT

<213>  HeLa

<400>  258

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Gly Val Thr Lys
    210

<210>  259

<211>  445

<212>  PRT

<213>  HeLa

<400>  259

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu

115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Gly Val Thr Lys
        435                 440                 445

<210>  260

<211>  437

<212>  PRT

<213>  HeLa

<400>  260

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu
        435

<210>  261

<211>  443

<212>  PRT

<213>  HeLa

<400>  261

Met Met Ser Arg Leu Asp Lys Ser Lys Val Ile Asn Ser Ala Leu Glu
1               5                   10                  15

Leu Leu Asn Glu Val Gly Ile Glu Gly Leu Thr Thr Arg Lys Leu Ala
            20                  25                  30

Gln Lys Leu Gly Val Glu Gln Pro Thr Leu Tyr Trp His Val Lys Asn
        35                  40                  45

Lys Arg Ala Leu Leu Asp Ala Leu Ala Val Glu Ile Leu Ala Arg His
    50                  55                  60

His Asp Tyr Ser Leu Pro Ala Ala Gly Glu Ser Trp Gln Ser Phe Leu
65                  70                  75                  80

Arg Asn Asn Ala Met Ser Phe Arg Arg Ala Leu Leu Arg Tyr Arg Asp
                85                  90                  95

Gly Ala Lys Val His Leu Gly Thr Arg Pro Asp Glu Lys Gln Tyr Asp
            100                 105                 110

Thr Val Glu Thr Gln Leu Arg Phe Met Thr Glu Asn Gly Phe Ser Leu
        115                 120                 125

Arg Asp Gly Leu Tyr Ala Ile Ser Ala Val Ser His Phe Thr Leu Gly

130                 135                 140

Ala Val Leu Glu Gln Gln Glu His Thr Ala Ala Leu Thr Asp Arg Pro
145                 150                 155                 160

Ala Ala Pro Asp Glu Asn Leu Pro Pro Leu Leu Arg Glu Ala Leu Gln
                165                 170                 175

Ile Met Asp Ser Asp Asp Gly Glu Gln Ala Phe Leu His Gly Leu Glu
            180                 185                 190

Ser Leu Ile Arg Gly Phe Glu Val Gln Leu Thr Ala Leu Leu Gln Ile
        195                 200                 205

Asp Ser Gly Gly Gly Gly Ser Gly Gly Gly Gly Ser Gly Gly Gly Gly
    210                 215                 220

Ser Gly Gly Gly Gly Ser Gly Gly Gly Gly Ser Arg Ser Arg Leu Asp
225                 230                 235                 240

Lys Ser Lys Val Ile Asn Ser Ala Leu Glu Leu Leu Asn Glu Val Gly
                245                 250                 255

Ile Glu Gly Leu Thr Thr Arg Lys Leu Ala Gln Lys Leu Gly Val Glu
            260                 265                 270

Gln Pro Thr Leu Tyr Trp His Val Lys Asn Lys Arg Ala Leu Leu Asp
        275                 280                 285

Ala Leu Ala Val Glu Ile Leu Ala Arg His His Asp Tyr Ser Leu Pro
    290                 295                 300

Ala Ala Gly Glu Ser Trp Gln Ser Phe Leu Arg Asn Asn Ala Met Ser
305                 310                 315                 320

Phe Arg Arg Ala Leu Leu Arg Tyr Arg Asp Gly Ala Lys Val His Leu
                325                 330                 335

Gly Thr Arg Pro Asp Glu Lys Gln Tyr Asp Thr Val Glu Thr Gln Leu
            340                 345                 350

Arg Phe Met Thr Glu Asn Gly Phe Ser Leu Arg Asp Gly Leu Tyr Ala
        355                 360                 365

Ile Ser Ala Val Ser His Phe Thr Leu Gly Ala Val Leu Glu Gln Gln
    370                 375                 380

Glu His Thr Ala Ala Leu Thr Asp Arg Pro Ala Ala Pro Asp Glu Asn
385                 390                 395                 400

Leu Pro Pro Leu Leu Arg Glu Ala Leu Gln Ile Met Asp Ser Asp Asp
                405                 410                 415

Gly Glu Gln Ala Phe Leu His Gly Leu Glu Ser Leu Ile Arg Gly Phe
            420                 425                 430

Glu Val Gln Leu Thr Ala Leu Leu Gln Ile Val
        435                 440

<210>  262

<211>  571

<212>  PRT

<213>  HeLa

<400>  262

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ser Pro Lys Lys Lys Arg
        435                 440                 445

Lys Val Asp Gly Gly Gly Ala Leu Ser Pro Gln His Ser Ala Val Thr
    450                 455                 460

Gln Gly Ser Ile Ile Lys Asn Lys Glu Gly Met Asp Ala Lys Ser Leu
465                 470                 475                 480

Thr Ala Trp Ser Arg Thr Leu Val Thr Phe Lys Asp Val Phe Val Asp
                485                 490                 495

Phe Thr Arg Glu Glu Trp Lys Leu Leu Asp Thr Ala Gln Gln Ile Val
            500                 505                 510

Tyr Arg Asn Val Met Leu Glu Asn Tyr Lys Asn Leu Val Ser Leu Gly
        515                 520                 525

Tyr Gln Leu Thr Lys Pro Asp Val Ile Leu Arg Leu Glu Lys Gly Glu
    530                 535                 540

Glu Pro Trp Leu Val Glu Arg Glu Ile His Gln Glu Thr His Pro Asp
545                 550                 555                 560

Ser Glu Thr Ala Phe Glu Ile Lys Ser Ser Val
                565                 570

<210>  263

<211>  339

<212>  PRT

<213>  HeLa

<400>  263

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln

20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Ser Pro Lys Lys Lys Arg Lys Val Asp Gly Gly Gly Ala Leu
    210                 215                 220

Ser Pro Gln His Ser Ala Val Thr Gln Gly Ser Ile Ile Lys Asn Lys
225                 230                 235                 240

Glu Gly Met Asp Ala Lys Ser Leu Thr Ala Trp Ser Arg Thr Leu Val
                245                 250                 255

Thr Phe Lys Asp Val Phe Val Asp Phe Thr Arg Glu Glu Trp Lys Leu
            260                 265                 270

Leu Asp Thr Ala Gln Gln Ile Val Tyr Arg Asn Val Met Leu Glu Asn
        275                 280                 285

Tyr Lys Asn Leu Val Ser Leu Gly Tyr Gln Leu Thr Lys Pro Asp Val
    290                 295                 300

Ile Leu Arg Leu Glu Lys Gly Glu Glu Pro Trp Leu Val Glu Arg Glu
305                 310                 315                 320

Ile His Gln Glu Thr His Pro Asp Ser Glu Thr Ala Phe Glu Ile Lys
                325                 330                 335

Ser Ser Val

<210> 264

<211> 339

<212> PRT

<213> HeLa

<400> 264

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ile Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Ser Pro Lys Lys Lys Arg Lys Val Asp Gly Gly Gly Ala Leu
    210                 215                 220

Ser Pro Gln His Ser Ala Val Thr Gln Gly Ser Ile Ile Lys Asn Lys
225                 230                 235                 240

Glu Gly Met Asp Ala Lys Ser Leu Thr Ala Trp Ser Arg Thr Leu Val
                245                 250                 255

Thr Phe Lys Asp Val Phe Val Asp Phe Thr Arg Glu Glu Trp Lys Leu
            260                 265                 270

Leu Asp Thr Ala Gln Gln Ile Val Tyr Arg Asn Val Met Leu Glu Asn
        275                 280                 285

Tyr Lys Asn Leu Val Ser Leu Gly Tyr Gln Leu Thr Lys Pro Asp Val
    290                 295                 300

Ile Leu Arg Leu Glu Lys Gly Glu Glu Pro Trp Leu Val Glu Arg Glu
305                 310                 315                 320

Ile His Gln Glu Thr His Pro Asp Ser Glu Thr Ala Phe Glu Ile Lys
                325                 330                 335

Ser Ser Val

<210>  265

<211>  338

<212>  PRT

<213>  HeLa

<400>  265

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Ser Pro Lys Lys Lys Arg Lys Val Asp Gly Gly Gly Ala Leu Ser
    210                 215                 220

Pro Gln His Ser Ala Val Thr Gln Gly Ser Ile Ile Lys Asn Lys Glu

225                 230                 235                 240

Gly Met Asp Ala Lys Ser Leu Thr Ala Trp Ser Arg Thr Leu Val Thr
                245                 250                 255

Phe Lys Asp Val Phe Val Asp Phe Thr Arg Glu Glu Trp Lys Leu Leu
            260                 265                 270

Asp Thr Ala Gln Gln Ile Val Tyr Arg Asn Val Met Leu Glu Asn Tyr
        275                 280                 285

Lys Asn Leu Val Ser Leu Gly Tyr Gln Leu Thr Lys Pro Asp Val Ile
    290                 295                 300

Leu Arg Leu Glu Lys Gly Glu Glu Pro Trp Leu Val Glu Arg Glu Ile
305                 310                 315                 320

His Gln Glu Thr His Pro Asp Ser Glu Thr Ala Phe Glu Ile Lys Ser
                325                 330                 335

Ser Val

<210>  266

<211>  328

<212>  PRT

<213>  HeLa

<400>  266

<210>  267

<211>  1169

<212>  PRT

<213>  HeLa

<400>  267

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Glu Gly Glu Ala Val Glu
        435                 440                 445

Ala Ile Val Glu Glu Ser Glu Thr Phe Ile Lys Gly Lys Glu Arg Lys

450                 455                 460

Thr Tyr Gln Arg Arg Arg Glu Gly Gly Gln Glu Asp Glu Ala Cys His
465                 470                 475                 480

Ile Ala Pro Asn Gln Ala Asp Gly Gly Glu Val Val Gln Asp Val Asn
                485                 490                 495

Ser Gly Val Gln Met Val Met Met Glu Gln Leu Asp Pro Thr Leu Leu
            500                 505                 510

Gln Met Lys Thr Glu Val Met Glu Gly Ala Val Pro Gln Glu Thr Glu
        515                 520                 525

Ala Thr Val Asp Asp Thr Gln Ile Ile Thr Leu Gln Val Val Asn Met
    530                 535                 540

Glu Glu Gln Pro Ile Asn Leu Gly Glu Leu Gln Leu Val Gln Val Pro
545                 550                 555                 560

Val Pro Val Thr Val Pro Val Ala Thr Thr Ser Val Glu Glu Leu Gln
                565                 570                 575

Gly Ala Tyr Glu Asn Glu Val Ser Lys Gly Gly Leu Gln Glu Gly Glu
            580                 585                 590

Pro Met Ile Cys His Thr Leu Pro Leu Pro Glu Gly Phe Gln Val Val
        595                 600                 605

Lys Val Gly Ala Asn Gly Glu Val Glu Thr Leu Glu Gln Gly Glu Leu
    610                 615                 620

Gln Pro Gln Glu Asp Pro Asn Trp Gln Lys Asp Pro Asp Tyr Gln Pro
625                 630                 635                 640

Pro Ala Lys Lys Thr Lys Lys Asn Lys Lys Ser Lys Leu Arg Tyr Thr
                645                 650                 655

Glu Glu Gly Lys Asp Val Asp Val Ser Val Tyr Asp Phe Glu Glu Glu
            660                 665                 670

Gln Gln Glu Gly Leu Leu Ser Glu Val Asn Ala Glu Lys Val Val Gly
        675                 680                 685

Asn Met Lys Pro Pro Lys Pro Thr Lys Ile Lys Lys Lys Gly Val Lys
    690                 695                 700

Lys Thr Phe Gln Cys Glu Leu Cys Ser Tyr Thr Cys Pro Arg Arg Ser
705                 710                 715                 720

Asn Leu Asp Arg His Met Lys Ser His Thr Asp Glu Arg Pro His Lys
                725                 730                 735

Cys His Leu Cys Gly Arg Ala Phe Arg Thr Val Thr Leu Leu Arg Asn
            740                 745                 750

His Leu Asn Thr His Thr Gly Thr Arg Pro His Lys Cys Pro Asp Cys
        755                 760                 765

Asp Met Ala Phe Val Thr Ser Gly Glu Leu Val Arg His Arg Arg Tyr
    770                 775                 780

Lys His Thr His Glu Lys Pro Phe Lys Cys Ser Met Cys Asp Tyr Ala
785                 790                 795                 800

Ser Val Glu Val Ser Lys Leu Lys Arg His Ile Arg Ser His Thr Gly
                805                 810                 815

Glu Arg Pro Phe Gln Cys Ser Leu Cys Ser Tyr Ala Ser Arg Asp Thr
            820                 825                 830

Tyr Lys Leu Lys Arg His Met Arg Thr His Ser Gly Glu Lys Pro Tyr
        835                 840                 845

Glu Cys Tyr Ile Cys His Ala Arg Phe Thr Gln Ser Gly Thr Met Lys
    850                 855                 860

Met His Ile Leu Gln Lys His Thr Glu Asn Val Ala Lys Phe His Cys
865                 870                 875                 880

Pro His Cys Asp Thr Val Ile Ala Arg Lys Ser Asp Leu Gly Val His
                885                 890                 895

Leu Arg Lys Gln His Ser Tyr Ile Glu Gln Gly Lys Lys Cys Arg Tyr
            900                 905                 910

Cys Asp Ala Val Phe His Glu Arg Tyr Ala Leu Ile Gln His Gln Lys
        915                 920                 925

Ser His Lys Asn Glu Lys Arg Phe Lys Cys Asp Gln Cys Asp Tyr Ala
    930                 935                 940

Cys Arg Gln Glu Arg His Met Val Met His Lys Arg Thr His Thr Gly
945                 950                 955                 960

Glu Lys Pro Tyr Ala Cys Ser His Cys Asp Lys Thr Phe Arg Gln Lys
                965                 970                 975

Gln Leu Leu Asp Met His Phe Lys Arg Tyr His Asp Pro Asn Phe Val
            980                 985                 990

Pro Ala Ala Phe Val Cys Ser Lys Cys Gly Lys Thr Phe Thr Arg Arg
        995                 1000                1005

Asn Thr Met Ala Arg His Ala Asp Asn Cys Ser Gly Leu Asp Gly
    1010                1015                1020

Gly Glu Gly Glu Asn Gly Gly Glu Thr Lys Lys Gly Lys Arg Gly
    1025                1030                1035

Arg Lys Arg Lys Met Arg Ser Lys Lys Glu Asp Ser Ser Asp Ser
    1040                1045                1050

Glu Glu Asn Ala Glu Pro Asp Leu Asp Asp Asn Glu Asp Glu Glu
    1055                1060                1065

Glu Thr Ala Val Glu Ile Glu Ala Glu Pro Glu Val Glu Pro Glu
    1070                1075                1080

Ala Pro Ala Pro Pro Pro Ser Lys Lys Arg Arg Gly Arg Pro Pro
    1085                1090                1095

Gly Lys Ala Ala Thr Gln Thr Lys Gln Ser Gln Pro Ala Ala Ile
    1100                1105                1110

Ile Gln Val Glu Asp Gln Asn Thr Gly Glu Ile Glu Asn Ile Ile
    1115                1120                1125

Val Glu Val Lys Lys Glu Pro Asp Ala Glu Thr Val Glu Glu Glu
    1130                1135                1140

Glu Glu Ala Gln Pro Ala Val Val Glu Ala Pro Asn Gly Asp Leu
    1145                1150                1155

Thr Pro Glu Met Ile Leu Ser Met Met Asp Arg
    1160                1165

<210>  268

<211>  1169

<212>  PRT

<213>  HeLa

<400>  268

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser

225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Glu Gly Glu Ala Val Glu
        435                 440                 445

Ala Ile Val Glu Glu Ser Glu Thr Phe Ile Lys Gly Lys Glu Arg Lys
    450                 455                 460

Thr Tyr Gln Arg Arg Arg Glu Gly Gly Gln Glu Asp Glu Ala Cys His
465                 470                 475                 480

Ile Ala Pro Asn Gln Ala Asp Gly Gly Glu Val Val Gln Asp Val Asn
                485                 490                 495

Ser Gly Val Gln Met Val Met Met Glu Gln Leu Asp Pro Thr Leu Leu
            500                 505                 510

Gln Met Lys Thr Glu Val Met Glu Gly Ala Val Pro Gln Glu Thr Glu
        515                 520                 525

Ala Thr Val Asp Asp Thr Gln Ile Ile Thr Leu Gln Val Val Asn Met
    530                 535                 540

Glu Glu Gln Pro Ile Asn Leu Gly Glu Leu Gln Leu Val Gln Val Pro
545                 550                 555                 560

Val Pro Val Thr Val Pro Val Ala Thr Thr Ser Val Glu Glu Leu Gln
                565                 570                 575

Gly Ala Tyr Glu Asn Glu Val Ser Lys Gly Gly Leu Gln Glu Gly Glu
            580                 585                 590

Pro Met Ile Cys His Thr Leu Pro Leu Pro Glu Gly Phe Gln Val Val
        595                 600                 605

Lys Val Gly Ala Asn Gly Glu Val Glu Thr Leu Glu Gln Gly Glu Leu
    610                 615                 620

Gln Pro Gln Glu Asp Pro Asn Trp Gln Lys Asp Pro Asp Tyr Gln Pro
625                 630                 635                 640

Pro Ala Lys Lys Thr Lys Lys Asn Lys Lys Ser Lys Leu Arg Tyr Thr
                645                 650                 655

Glu Glu Gly Lys Asp Val Asp Val Ser Val Tyr Asp Phe Glu Glu Glu
            660                 665                 670

Gln Gln Glu Gly Leu Leu Ser Glu Val Asn Ala Glu Lys Val Val Gly
        675                 680                 685

Asn Met Lys Pro Pro Lys Pro Thr Lys Ile Lys Lys Lys Gly Val Lys
    690                 695                 700

Lys Thr Phe Gln Cys Glu Leu Cys Ser Tyr Thr Cys Pro Arg Arg Ser
705                 710                 715                 720

Asn Leu Asp Arg His Met Lys Ser His Thr Asp Glu Arg Pro His Lys
                725                 730                 735

Cys His Leu Cys Gly Arg Ala Phe Arg Thr Val Thr Leu Leu Arg Asn
            740                 745                 750

His Leu Asn Thr His Thr Gly Thr Arg Pro His Lys Cys Pro Asp Cys
        755                 760                 765

Asp Met Ala Phe Val Thr Ser Gly Glu Leu Val Arg His Arg Arg Tyr
    770                 775                 780

Lys His Thr His Glu Lys Pro Phe Lys Cys Ser Met Cys Asp Tyr Ala
785                 790                 795                 800

Ser Val Glu Val Ser Lys Leu Lys Arg His Ile Arg Ser His Thr Gly
                805                 810                 815

Glu Arg Pro Phe Gln Cys Ser Leu Cys Ser Tyr Ala Ser Arg Asp Thr
            820                 825                 830

Tyr Lys Leu Lys Arg His Met Arg Thr His Ser Gly Glu Lys Pro Tyr
        835                 840                 845

Glu Cys Tyr Ile Cys His Ala Arg Phe Thr Gln Ser Gly Thr Met Lys
    850                 855                 860

Met His Ile Leu Gln Lys His Thr Glu Asn Val Ala Lys Phe His Cys
865                 870                 875                 880

Pro His Cys Asp Thr Val Ile Ala Arg Lys Ser Asp Leu Gly Val His
                885                 890                 895

Leu Arg Lys Gln His Ser Tyr Ile Glu Gln Gly Lys Lys Cys Arg Tyr
            900                 905                 910

Cys Asp Ala Val Phe His Glu Arg Tyr Ala Leu Ile Gln His Gln Lys
        915                 920                 925

Ser His Lys Asn Glu Lys Arg Phe Lys Cys Asp Gln Cys Asp Tyr Ala
    930                 935                 940

Cys Arg Gln Glu Arg His Met Val Met His Lys Arg Thr His Thr Gly
945                 950                 955                 960

Glu Lys Pro Tyr Ala Cys Ser His Cys Asp Lys Thr Phe Arg Gln Lys
                965                 970                 975

Gln Leu Leu Asp Met His Phe Lys Arg Tyr His Asp Pro Asn Phe Val
            980                 985                 990

Pro Ala Ala Phe Val Cys Ser Lys Cys Gly Lys Thr Phe Thr Arg Arg
        995                 1000                1005

Asn Thr Met Ala Arg His Ala Asp Asn Cys Ser Gly Leu Asp Gly
    1010                1015                1020

Gly Glu Gly Glu Asn Gly Gly Glu Thr Lys Lys Gly Lys Arg Gly
    1025                1030                1035

Arg Lys Arg Lys Met Arg Ser Lys Lys Glu Asp Ser Ser Asp Ser
    1040                1045                1050

Glu Glu Asn Ala Glu Pro Asp Leu Asp Asp Asn Glu Asp Glu Glu
    1055                1060                1065

Glu Thr Ala Val Glu Ile Glu Ala Glu Pro Glu Val Glu Pro Glu
    1070                1075                1080

Ala Pro Ala Pro Pro Pro Ser Lys Lys Arg Arg Gly Arg Pro Pro
    1085                1090                1095

Gly Lys Ala Ala Thr Gln Thr Lys Gln Ser Gln Pro Ala Ala Ile
    1100                1105                1110

Ile Gln Val Glu Asp Gln Asn Thr Gly Glu Ile Glu Asn Ile Ile
    1115                1120                1125

Val Glu Val Lys Lys Glu Pro Asp Ala Glu Thr Val Glu Glu Glu
    1130                1135                1140

Glu Glu Ala Gln Pro Ala Val Val Glu Ala Pro Asn Gly Asp Leu
    1145                1150                1155

Thr Pro Glu Met Ile Leu Ser Met Met Asp Arg
    1160                1165

<210> 269

<211> 936

<212> PRT

<213> HeLa

<400> 269

Met Ser Arg Leu Asp Lys Ser Lys Val Ile Asn Ser Ala Leu Glu Leu

1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Glu Gly Glu Ala Val Glu Ala Ile Val Glu Glu Ser Glu Thr Phe
    210                 215                 220

Ile Lys Gly Lys Glu Arg Lys Thr Tyr Gln Arg Arg Arg Glu Gly Gly
225                 230                 235                 240

Gln Glu Asp Glu Ala Cys His Ile Ala Pro Asn Gln Ala Asp Gly Gly
                245                 250                 255

Glu Val Val Gln Asp Val Asn Ser Gly Val Gln Met Val Met Met Glu
            260                 265                 270

Gln Leu Asp Pro Thr Leu Leu Gln Met Lys Thr Glu Val Met Glu Gly
        275                 280                 285

Ala Val Pro Gln Glu Thr Glu Ala Thr Val Asp Asp Thr Gln Ile Ile
    290                 295                 300

Thr Leu Gln Val Val Asn Met Glu Glu Gln Pro Ile Asn Leu Gly Glu
305                 310                 315                 320

Leu Gln Leu Val Gln Val Pro Val Pro Val Thr Val Pro Val Ala Thr
                325                 330                 335

Thr Ser Val Glu Glu Leu Gln Gly Ala Tyr Glu Asn Glu Val Ser Lys
            340                 345                 350

Gly Gly Leu Gln Glu Gly Glu Pro Met Ile Cys His Thr Leu Pro Leu
        355                 360                 365

Pro Glu Gly Phe Gln Val Val Lys Val Gly Ala Asn Gly Glu Val Glu
    370                 375                 380

Thr Leu Glu Gln Gly Glu Leu Gln Pro Gln Glu Asp Pro Asn Trp Gln
385                 390                 395                 400

Lys Asp Pro Asp Tyr Gln Pro Pro Ala Lys Lys Thr Lys Lys Asn Lys
                405                 410                 415

Lys Ser Lys Leu Arg Tyr Thr Glu Glu Gly Lys Asp Val Asp Val Ser
            420                 425                 430

Val Tyr Asp Phe Glu Glu Glu Gln Gln Glu Gly Leu Leu Ser Glu Val
        435                 440                 445

Asn Ala Glu Lys Val Val Gly Asn Met Lys Pro Pro Lys Pro Thr Lys
    450                 455                 460

Ile Lys Lys Lys Gly Val Lys Lys Thr Phe Gln Cys Glu Leu Cys Ser
465                 470                 475                 480

Tyr Thr Cys Pro Arg Arg Ser Asn Leu Asp Arg His Met Lys Ser His
                485                 490                 495

Thr Asp Glu Arg Pro His Lys Cys His Leu Cys Gly Arg Ala Phe Arg
            500                 505                 510

Thr Val Thr Leu Leu Arg Asn His Leu Asn Thr His Thr Gly Thr Arg
        515                 520                 525

Pro His Lys Cys Pro Asp Cys Asp Met Ala Phe Val Thr Ser Gly Glu
    530                 535                 540

Leu Val Arg His Arg Arg Tyr Lys His Thr His Glu Lys Pro Phe Lys
545                 550                 555                 560

Cys Ser Met Cys Asp Tyr Ala Ser Val Glu Val Ser Lys Leu Lys Arg
                565                 570                 575

His Ile Arg Ser His Thr Gly Glu Arg Pro Phe Gln Cys Ser Leu Cys
            580                 585                 590

Ser Tyr Ala Ser Arg Asp Thr Tyr Lys Leu Lys Arg His Met Arg Thr
        595                 600                 605

His Ser Gly Glu Lys Pro Tyr Glu Cys Tyr Ile Cys His Ala Arg Phe
    610                 615                 620

Thr Gln Ser Gly Thr Met Lys Met His Ile Leu Gln Lys His Thr Glu
625                 630                 635                 640

Asn Val Ala Lys Phe His Cys Pro His Cys Asp Thr Val Ile Ala Arg
                645                 650                 655

Lys Ser Asp Leu Gly Val His Leu Arg Lys Gln His Ser Tyr Ile Glu
            660                 665                 670

Gln Gly Lys Lys Cys Arg Tyr Cys Asp Ala Val Phe His Glu Arg Tyr
        675                 680                 685

Ala Leu Ile Gln His Gln Lys Ser His Lys Asn Glu Lys Arg Phe Lys
    690                 695                 700

Cys Asp Gln Cys Asp Tyr Ala Cys Arg Gln Glu Arg His Met Val Met
705                 710                 715                 720

His Lys Arg Thr His Thr Gly Glu Lys Pro Tyr Ala Cys Ser His Cys
                725                 730                 735

Asp Lys Thr Phe Arg Gln Lys Gln Leu Leu Asp Met His Phe Lys Arg
            740                 745                 750

Tyr His Asp Pro Asn Phe Val Pro Ala Ala Phe Val Cys Ser Lys Cys
        755                 760                 765

Gly Lys Thr Phe Thr Arg Arg Asn Thr Met Ala Arg His Ala Asp Asn
    770                 775                 780

Cys Ser Gly Leu Asp Gly Gly Glu Gly Glu Asn Gly Gly Glu Thr Lys
785                 790                 795                 800

Lys Gly Lys Arg Gly Arg Lys Arg Lys Met Arg Ser Lys Lys Glu Asp
                805                 810                 815

Ser Ser Asp Ser Glu Glu Asn Ala Glu Pro Asp Leu Asp Asp Asn Glu
            820                 825                 830

Asp Glu Glu Glu Thr Ala Val Glu Ile Glu Ala Glu Pro Glu Val Glu
        835                 840                 845

Pro Glu Ala Pro Ala Pro Pro Pro Ser Lys Lys Arg Arg Gly Arg Pro
    850                 855                 860

Pro Gly Lys Ala Ala Thr Gln Thr Lys Gln Ser Gln Pro Ala Ala Ile
865                 870                 875                 880

Ile Gln Val Glu Asp Gln Asn Thr Gly Glu Ile Glu Asn Ile Ile Val
                885                 890                 895

Glu Val Lys Lys Glu Pro Asp Ala Glu Thr Val Glu Glu Glu Glu Glu
            900                 905                 910

Ala Gln Pro Ala Val Val Glu Ala Pro Asn Gly Asp Leu Thr Pro Glu
        915                 920                 925

Met Ile Leu Ser Met Met Asp Arg
    930                 935

<210>  270

<211>  940

<212>  PRT

<213>  HeLa

<400>  270
   Met Ser Arg Leu Asp Lys Ser Lys Val Ile Asn Ser Ala Leu Glu Leu
   1               5                   10                  15
   Leu Asn Glu Val Gly Ile Glu Gly Leu Thr Thr Arg Lys Leu Ala Gln
   20                  25                  30
   Lys Leu Gly Val Glu Gln Pro Thr Leu Tyr Trp His Val Lys Asn Lys
           35                  40                  45
   Arg Ala Leu Leu Asp Ala Leu Ala Ile Glu Met Leu Asp Arg His His
       50                  55                  60
   Thr His Phe Cys Pro Leu Glu Gly Glu Ser Trp Gln Asp Phe Leu Arg
   65                  70                  75                  80
   Asn Asn Ala Lys Ser Phe Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Asp Gln Glu His Gln Val Ala Lys Glu Glu Arg Glu Thr
   145                 150                 155                 160
   Pro Thr Thr Asp Ser Met Pro Pro Leu Leu Arg Gln Ala Ile Glu Leu
                   165                 170                 175
   Phe Asp His Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Asp Ser
           195                 200                 205
   Gly Gly Gly Gly Ser Glu Gly Glu Ala Val Glu Ala Ile Val Glu Glu
       210                 215                 220
   Ser Glu Thr Phe Ile Lys Gly Lys Glu Arg Lys Thr Tyr Gln Arg Arg
   225                 230                 235                 240
   Arg Glu Gly Gly Gln Glu Asp Glu Ala Cys His Ile Ala Pro Asn Gln
                   245                 250                 255
   Ala Asp Gly Gly Glu Val Val Gln Asp Val Asn Ser Gly Val Gln Met
               260                 265                 270
   Val Met Met Glu His Leu Asp Pro Thr Leu Leu Gln Met Lys Thr Glu
           275                 280                 285
   Val Met Glu Gly Ala Val Pro Gln Glu Thr Glu Ala Thr Val Asp Asp
       290                 295                 300
   Thr Gln Ile Ile Thr Leu Gln Val Val Asn Met Glu Glu Gln Pro Ile
   305                 310                 315                 320
   Asn Leu Gly Glu Leu Gln Leu Val Gln Val Pro Val Pro Val Thr Val
                   325                 330                 335
   Pro Val Ala Thr Thr Ser Val Glu Glu Leu Gln Gly Ala Tyr Glu Asn
               340                 345                 350
   Glu Val Ser Lys Gly Gly Leu Gln Glu Gly Glu Pro Met Ile Cys His
           355                 360                 365
   Thr Leu Pro Leu Pro Glu Gly Phe Gln Val Val Lys Val Gly Ala Asn
       370                 375                 380
   Gly Glu Val Glu Thr Leu Glu Gln Gly Glu Leu Gln Pro Gln Glu Asp
   385                 390                 395                 400
   Pro Asn Trp Gln Lys Asp Pro Asp Tyr Gln Pro Pro Ala Lys Lys Thr
                   405                 410                 415
   Lys Lys Asn Lys Lys Ser Lys Leu Arg Tyr Thr Glu Glu Gly Lys Asp
               420                 425                 430
   Val Asp Val Ser Val Tyr Asp Phe Glu Glu Glu Gln Gln Glu Gly Leu
           435                 440                 445
   Leu Ser Glu Val Asn Ala Glu Lys Val Val Gly Asn Met Lys Pro Pro
       450                 455                 460
   Lys Pro Thr Lys Ile Lys Lys Lys Gly Val Lys Lys Thr Phe Gln Cys
   465                 470                 475                 480
   Glu Leu Cys Ser Tyr Thr Cys Pro Arg Arg Ser Asn Leu Asp Arg His
                   485                 490                 495
   Met Lys Ser His Thr Asp Glu Arg Pro His Lys Cys His Leu Cys Gly
               500                 505                 510
   Arg Ala Phe Arg Thr Val Thr Leu Leu Arg Asn His Leu Asn Thr His
           515                 520                 525
   Thr Gly Thr Arg Pro His Lys Cys Pro Asp Cys Asp Met Ala Phe Val
       530                 535                 540
   Thr Ser Gly Glu Leu Val Arg His Arg Arg Tyr Lys His Thr His Glu
   545                 550                 555                 560
   Lys Pro Phe Lys Cys Ser Met Cys Asp Tyr Ala Ser Val Glu Val Ser
                   565                 570                 575
   Lys Leu Lys Arg His Ile Arg Ser His Thr Gly Glu Arg Pro Phe Gln
               580                 585                 590
   Cys Ser Leu Cys Ser Tyr Ala Ser Arg Asp Thr Tyr Lys Leu Lys Arg
           595                 600                 605
   His Met Arg Thr His Ser Gly Glu Lys Pro Tyr Glu Cys Tyr Ile Cys
       610                 615                 620
   His Ala Arg Phe Thr Gln Ser Gly Thr Met Lys Met His Ile Leu Gln
   625                 630                 635                 640
   Lys His Thr Glu Asn Val Ala Lys Phe His Cys Pro His Cys Asp Thr
                   645                 650                 655
   Val Ile Ala Arg Lys Ser Asp Leu Gly Val His Leu Arg Lys Gln His
               660                 665                 670
   Ser Tyr Ile Glu Gln Gly Lys Lys Cys Arg Tyr Cys Asp Ala Val Phe
           675                 680                 685
   His Glu Arg Tyr Ala Leu Ile Gln His Gln Lys Ser His Lys Asn Glu
       690                 695                 700
   Lys Arg Phe Lys Cys Asp Gln Cys Asp Tyr Ala Cys Arg Gln Glu Arg
   705                 710                 715                 720
   His Met Val Met His Lys Arg Thr His Thr Gly Glu Lys Pro Tyr Ala
                   725                 730                 735
   Cys Ser His Cys Asp Lys Thr Phe Arg Gln Lys Gln Leu Leu Asp Met
               740                 745                 750
   His Phe Lys Arg Tyr His Asp Pro Asn Phe Val Pro Ala Ala Phe Val
           755                 760                 765
   Cys Ser Lys Cys Gly Lys Thr Phe Thr Arg Arg Asn Thr Met Ala Arg
       770                 775                 780
   His Ala Asp Asn Cys Ser Gly Leu Asp Gly Gly Glu Gly Glu Asn Gly
   785                 790                 795                 800
   Gly Glu Thr Lys Lys Gly Lys Arg Gly Arg Lys Arg Lys Met Arg Ser
                   805                 810                 815
   Lys Lys Glu Asp Ser Ser Asp Ser Glu Glu Asn Ala Glu Pro Asp Leu
               820                 825                 830
   Asp Asp Asn Glu Asp Glu Glu Glu Thr Ala Val Glu Ile Glu Ala Glu
           835                 840                 845
   Pro Glu Val Ser Ala Glu Ala Pro Ala Pro Pro Pro Ser Lys Lys Arg
       850                 855                 860
   Arg Gly Arg Pro Pro Gly Lys Ala Ala Thr Gln Thr Lys Gln Ser Gln
   865                 870                 875                 880
   Pro Ala Ala Ile Ile Gln Val Glu Asp Gln Asn Thr Gly Glu Ile Glu
                   885                 890                 895
   Asn Ile Ile Val Glu Val Lys Lys Glu Pro Asp Ala Glu Thr Val Glu
               900                 905                 910
   Glu Glu Glu Glu Ala Gln Pro Ala Val Val Glu Ala Pro Asn Gly Asp
           915                 920                 925
   Leu Thr Pro Glu Met Ile Leu Ser Met Met Asp Arg
       930                 935                 940

<210>  271

<211>  962

<212>  PRT

<213>  HeLa

<400>  271

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys

35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Glu Gly Glu Ala Val
225                 230                 235                 240

Glu Ala Ile Val Glu Glu Ser Glu Thr Phe Ile Lys Gly Lys Glu Arg
                245                 250                 255

Lys Thr Tyr Gln Arg Arg Arg Glu Gly Gly Gln Glu Asp Glu Ala Cys
            260                 265                 270

His Ile Ala Pro Asn Gln Ala Asp Gly Gly Glu Val Val Gln Asp Val
        275                 280                 285

Asn Ser Gly Val Gln Met Val Met Met Glu His Leu Asp Pro Thr Leu
    290                 295                 300

Leu Gln Met Lys Thr Glu Val Met Glu Gly Ala Val Pro Gln Glu Thr
305                 310                 315                 320

Glu Ala Thr Val Asp Asp Thr Gln Ile Ile Thr Leu Gln Val Val Asn
                325                 330                 335

Met Glu Glu Gln Pro Ile Asn Leu Gly Glu Leu Gln Leu Val Gln Val
            340                 345                 350

Pro Val Pro Val Thr Val Pro Val Ala Thr Thr Ser Val Glu Glu Leu
        355                 360                 365

Gln Gly Ala Tyr Glu Asn Glu Val Ser Lys Gly Gly Leu Gln Glu Gly
    370                 375                 380

Glu Pro Met Ile Cys His Thr Leu Pro Leu Pro Glu Gly Phe Gln Val
385                 390                 395                 400

Val Lys Val Gly Ala Asn Gly Glu Val Glu Thr Leu Glu Gln Gly Glu
                405                 410                 415

Leu Gln Pro Gln Glu Asp Pro Asn Trp Gln Lys Asp Pro Asp Tyr Gln
            420                 425                 430

Pro Pro Ala Lys Lys Thr Lys Lys Asn Lys Lys Ser Lys Leu Arg Tyr
        435                 440                 445

Thr Glu Glu Gly Lys Asp Val Asp Val Ser Val Tyr Asp Phe Glu Glu
    450                 455                 460

Glu Gln Gln Glu Gly Leu Leu Ser Glu Val Asn Ala Glu Lys Val Val
465                 470                 475                 480

Gly Asn Met Lys Pro Pro Lys Pro Thr Lys Ile Lys Lys Lys Gly Val
                485                 490                 495

Lys Lys Thr Phe Gln Cys Glu Leu Cys Ser Tyr Thr Cys Pro Arg Arg
            500                 505                 510

Ser Asn Leu Asp Arg His Met Lys Ser His Thr Asp Glu Arg Pro His
        515                 520                 525

Lys Cys His Leu Cys Gly Arg Ala Phe Arg Thr Val Thr Leu Leu Arg
    530                 535                 540

Asn His Leu Asn Thr His Thr Gly Thr Arg Pro His Lys Cys Pro Asp
545                 550                 555                 560

Cys Asp Met Ala Phe Val Thr Ser Gly Glu Leu Val Arg His Arg Arg
                565                 570                 575

Tyr Lys His Thr His Glu Lys Pro Phe Lys Cys Ser Met Cys Asp Tyr
            580                 585                 590

Ala Ser Val Glu Val Ser Lys Leu Lys Arg His Ile Arg Ser His Thr
        595                 600                 605

Gly Glu Arg Pro Phe Gln Cys Ser Leu Cys Ser Tyr Ala Ser Arg Asp
    610                 615                 620

Thr Tyr Lys Leu Lys Arg His Met Arg Thr His Ser Gly Glu Lys Pro
625                 630                 635                 640

Tyr Glu Cys Tyr Ile Cys His Ala Arg Phe Thr Gln Ser Gly Thr Met
                645                 650                 655

Lys Met His Ile Leu Gln Lys His Thr Glu Asn Val Ala Lys Phe His
            660                 665                 670

Cys Pro His Cys Asp Thr Val Ile Ala Arg Lys Ser Asp Leu Gly Val
        675                 680                 685

His Leu Arg Lys Gln His Ser Tyr Ile Glu Gln Gly Lys Lys Cys Arg
    690                 695                 700

Tyr Cys Asp Ala Val Phe His Glu Arg Tyr Ala Leu Ile Gln His Gln
705                 710                 715                 720

Lys Ser His Lys Asn Glu Lys Arg Phe Lys Cys Asp Gln Cys Asp Tyr
                725                 730                 735

Ala Cys Arg Gln Glu Arg His Met Val Met His Lys Arg Thr His Thr
            740                 745                 750

Gly Glu Lys Pro Tyr Ala Cys Ser His Cys Asp Lys Thr Phe Arg Gln
        755                 760                 765

Lys Gln Leu Leu Asp Met His Phe Lys Arg Tyr His Asp Pro Asn Phe
    770                 775                 780

Val Pro Ala Ala Phe Val Cys Ser Lys Cys Gly Lys Thr Phe Thr Arg
785                 790                 795                 800

Arg Asn Thr Met Ala Arg His Ala Asp Asn Cys Ser Gly Leu Asp Gly
                805                 810                 815

Gly Glu Gly Glu Asn Gly Gly Glu Thr Lys Lys Gly Lys Arg Gly Arg
            820                 825                 830

Lys Arg Lys Met Arg Ser Lys Lys Glu Asp Ser Ser Asp Ser Glu Glu
        835                 840                 845

Asn Ala Glu Pro Asp Leu Asp Asp Asn Glu Asp Glu Glu Glu Thr Ala
    850                 855                 860

Val Glu Ile Glu Ala Glu Pro Glu Val Ser Ala Glu Ala Pro Ala Pro
865                 870                 875                 880

Pro Pro Ser Lys Lys Arg Arg Gly Arg Pro Pro Gly Lys Ala Ala Thr
                885                 890                 895

Gln Thr Lys Gln Ser Gln Pro Ala Ala Ile Ile Gln Val Glu Asp Gln
            900                 905                 910

Asn Thr Gly Glu Ile Glu Asn Ile Ile Val Glu Val Lys Lys Glu Pro
        915                 920                 925

Asp Ala Glu Thr Val Glu Glu Glu Glu Ala Gln Pro Ala Val Val
    930                 935                 940

Glu Ala Pro Asn Gly Asp Leu Thr Pro Glu Met Ile Leu Ser Met Met
945                 950                 955                 960

Asp Arg

<210>  272

<211>  1714

<212>  PRT

<213>  HeLa

<400>  272

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln

            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Glu Gly Glu Ala Val Glu
        435                 440                 445

Ala Ile Val Glu Glu Ser Glu Thr Phe Ile Lys Gly Lys Glu Arg Lys
    450                 455                 460

Thr Tyr Gln Arg Arg Arg Glu Gly Gly Gln Glu Asp Glu Ala Cys His
465                 470                 475                 480

Ile Ala Pro Asn Gln Ala Asp Gly Gly Glu Val Val Gln Asp Val Asn
                485                 490                 495

Ser Gly Val Gln Met Val Met Met Glu Gln Leu Asp Pro Thr Leu Leu
            500                 505                 510

Gln Met Lys Thr Glu Val Met Glu Gly Ala Val Pro Gln Glu Thr Glu
        515                 520                 525

Ala Thr Val Asp Asp Thr Gln Ile Ile Thr Leu Gln Val Val Asn Met
    530                 535                 540

Glu Glu Gln Pro Ile Asn Leu Gly Glu Leu Gln Leu Val Gln Val Pro
545                 550                 555                 560

Val Pro Val Thr Val Pro Val Ala Thr Thr Ser Val Glu Glu Leu Gln
                565                 570                 575

Gly Ala Tyr Glu Asn Glu Val Ser Lys Gly Gly Leu Gln Glu Gly Glu
            580                 585                 590

Pro Met Ile Cys His Thr Leu Pro Leu Pro Glu Gly Phe Gln Val Val
        595                 600                 605

Lys Val Gly Ala Asn Gly Glu Val Glu Thr Leu Glu Gln Gly Glu Leu
    610                 615                 620

Gln Pro Gln Glu Asp Pro Asn Trp Gln Lys Asp Pro Asp Tyr Gln Pro
625                 630                 635                 640

Pro Ala Lys Lys Thr Lys Lys Asn Lys Lys Ser Lys Leu Arg Tyr Thr
                645                 650                 655

Glu Glu Gly Lys Asp Val Asp Val Ser Val Tyr Asp Phe Glu Glu Glu
            660                 665                 670

Gln Gln Glu Gly Leu Leu Ser Glu Val Asn Ala Glu Lys Val Val Gly
        675                 680                 685

Asn Met Lys Pro Pro Lys Pro Thr Lys Ile Lys Lys Lys Gly Val Lys
    690                 695                 700

Lys Thr Ser Gly Leu Asp Gly Gly Glu Gly Glu Asn Gly Gly Glu Thr
705                 710                 715                 720

Lys Lys Gly Lys Arg Gly Arg Lys Arg Lys Met Arg Ser Lys Lys Glu
                725                 730                 735

Asp Ser Ser Asp Ser Glu Glu Asn Ala Glu Pro Asp Leu Asp Asp Asn
            740                 745                 750

Glu Asp Glu Glu Glu Thr Ala Val Glu Ile Glu Ala Glu Pro Glu Val
        755                 760                 765

Glu Pro Glu Ala Pro Ala Pro Pro Pro Ser Lys Lys Arg Arg Gly Arg
    770                 775                 780

Pro Pro Gly Lys Ala Ala Thr Gln Thr Lys Gln Ser Gln Pro Ala Ala
785                 790                 795                 800

Ile Ile Gln Val Glu Asp Gln Asn Thr Gly Glu Ile Glu Asn Ile Ile
                805                 810                 815

Val Glu Val Lys Lys Glu Pro Asp Ala Glu Thr Val Glu Glu Glu Glu
            820                 825                 830

Glu Ala Gln Pro Ala Val Val Glu Ala Pro Asn Gly Asp Leu Thr Pro
        835                 840                 845

Glu Met Ile Leu Ser Met Met Asp Arg Met Ser Arg Leu Asp Lys Ser
    850                 855                 860

Lys Val Ile Asn Ser Ala Leu Glu Leu Leu Asn Glu Val Gly Ile Glu
865                 870                 875                 880

Gly Leu Thr Thr Arg Lys Leu Ala Gln Lys Leu Gly Val Glu Gln Pro
                885                 890                 895

Thr Leu Tyr Trp His Val Lys Asn Lys Arg Ala Leu Leu Asp Ala Leu
            900                 905                 910

Ala Ile Glu Met Leu Asp Arg His His Thr His Phe Cys Pro Leu Glu
        915                 920                 925

Gly Glu Ser Trp Gln Asp Phe Leu Arg Asn Asn Ala Lys Ser Phe Arg
    930                 935                 940

Cys Ala Leu Leu Ser His Arg Asp Gly Ala Lys Val His Leu Gly Thr
945                 950                 955                 960

Arg Pro Thr Glu Lys Gln Tyr Glu Thr Leu Glu Asn Gln Leu Ala Phe
                965                 970                 975

Leu Cys Gln Gln Gly Phe Ser Leu Glu Asn Ala Leu Tyr Ala Leu Ser
            980                 985                 990

Ala Val Gly His Phe Thr Leu Gly  Cys Val Leu Glu Asp  Gln Glu His
        995                 1000                 1005

Gln Val  Ala Lys Glu Glu Arg  Glu Thr Pro Thr Thr  Asp Ser Met
    1010                 1015                 1020

Pro Pro  Leu Leu Arg Gln Ala  Ile Glu Leu Phe Asp  His Gln Gly

1025                 1030                 1035

Ala Glu Pro Ala Phe Leu Phe Gly Leu Glu Leu Ile Ile Cys Gly
    1040                 1045                 1050

Leu Glu Lys Gln Leu Lys Cys Glu Ser Gly Asp Ser Gly Gly Gly
    1055                 1060                 1065

Gly Ser Gly Gly Gly Gly Ser Gly Gly Gly Gly Ser Gly Gly Gly
    1070                 1075                 1080

Gly Ser Gly Gly Gly Gly Ser Arg Ser Arg Leu Asp Lys Ser Lys
    1085                 1090                 1095

Val Ile Asn Ser Ala Leu Glu Leu Leu Asn Glu Val Gly Ile Glu
    1100                 1105                 1110

Gly Leu Thr Thr Arg Lys Leu Ala Gln Lys Leu Gly Val Glu Gln
    1115                 1120                 1125

Pro Thr Leu Tyr Trp His Val Lys Asn Lys Arg Ala Leu Leu Asp
    1130                 1135                 1140

Ala Leu Ala Ile Glu Met Leu Asp Arg His His Thr His Phe Cys
    1145                 1150                 1155

Pro Leu Glu Gly Glu Ser Trp Gln Asp Phe Leu Arg Asn Asn Ala
    1160                 1165                 1170

Lys Ser Phe Arg Cys Ala Leu Leu Ser His Arg Asp Gly Ala Lys
    1175                 1180                 1185

Val His Leu Gly Thr Arg Pro Thr Glu Lys Gln Tyr Glu Thr Leu
    1190                 1195                 1200

Glu Asn Gln Leu Ala Phe Leu Cys Gln Gln Gly Phe Ser Leu Glu
    1205                 1210                 1215

Asn Ala Leu Tyr Ala Leu Ser Ala Val Gly His Phe Thr Leu Gly
    1220                 1225                 1230

Cys Val Leu Glu Asp Gln Glu His Gln Val Ala Lys Glu Glu Arg
    1235                 1240                 1245

Glu Thr Pro Thr Thr Asp Ser Met Pro Pro Leu Leu Arg Gln Ala
    1250                 1255                 1260

Ile Glu Leu Phe Asp His Gln Gly Ala Glu Pro Ala Phe Leu Phe
    1265                1270                1275

Gly Leu Glu Leu Ile Ile Cys Gly Leu Glu Lys Gln Leu Lys Cys
    1280                1285                1290

Glu Ser Gly Gly Pro Ala Glu Gly Glu Ala Val Glu Ala Ile Val
    1295                1300                1305

Glu Glu Ser Glu Thr Phe Ile Lys Gly Lys Glu Arg Lys Thr Tyr
    1310                1315                1320

Gln Arg Arg Arg Glu Gly Gly Gln Glu Asp Glu Ala Cys His Ile
    1325                1330                1335

Ala Pro Asn Gln Ala Asp Gly Gly Glu Val Val Gln Asp Val Asn
    1340                1345                1350

Ser Gly Val Gln Met Val Met Met Glu Gln Leu Asp Pro Thr Leu
    1355                1360                1365

Leu Gln Met Lys Thr Glu Val Met Glu Gly Ala Val Pro Gln Glu
    1370                1375                1380

Thr Glu Ala Thr Val Asp Asp Thr Gln Ile Ile Thr Leu Gln Val
    1385                1390                1395

Val Asn Met Glu Glu Gln Pro Ile Asn Leu Gly Glu Leu Gln Leu
    1400                1405                1410

Val Gln Val Pro Val Pro Val Thr Val Pro Val Ala Thr Thr Ser
    1415                1420                1425

Val Glu Glu Leu Gln Gly Ala Tyr Glu Asn Glu Val Ser Lys Gly
    1430                1435                1440

Gly Leu Gln Glu Gly Glu Pro Met Ile Cys His Thr Leu Pro Leu
    1445                1450                1455

Pro Glu Gly Phe Gln Val Val Lys Val Gly Ala Asn Gly Glu Val
    1460                1465                1470

Glu Thr Leu Glu Gln Gly Glu Leu Gln Pro Gln Glu Asp Pro Asn
    1475                1480                1485

Trp Gln Lys Asp Pro Asp Tyr Gln Pro Pro Ala Lys Lys Thr Lys
    1490                1495                1500

Lys Asn Lys Lys Ser Lys Leu Arg Tyr Thr Glu Glu Gly Lys Asp
    1505                1510                1515

Val Asp Val Ser Val Tyr Asp Phe Glu Glu Glu Gln Gln Glu Gly
    1520                1525                1530

Leu Leu Ser Glu Val Asn Ala Glu Lys Val Val Gly Asn Met Lys
    1535                1540                1545

Pro Pro Lys Pro Thr Lys Ile Lys Lys Lys Gly Val Lys Lys Thr
    1550                1555                1560

Ser Gly Leu Asp Gly Gly Glu Gly Glu Asn Gly Gly Glu Thr Lys
    1565                1570                1575

Lys Gly Lys Arg Gly Arg Lys Arg Lys Met Arg Ser Lys Lys Glu
    1580                1585                1590

Asp Ser Ser Asp Ser Glu Glu Asn Ala Glu Pro Asp Leu Asp Asp
    1595                1600                1605

Asn Glu Asp Glu Glu Glu Thr Ala Val Glu Ile Glu Ala Glu Pro
    1610                1615                1620

Glu Val Glu Pro Glu Ala Pro Ala Pro Pro Pro Ser Lys Lys Arg
    1625                1630                1635

Arg Gly Arg Pro Pro Gly Lys Ala Ala Thr Gln Thr Lys Gln Ser
    1640                1645                1650

Gln Pro Ala Ala Ile Ile Gln Val Glu Asp Gln Asn Thr Gly Glu
    1655                1660                1665

Ile Glu Asn Ile Ile Val Glu Val Lys Lys Glu Pro Asp Ala Glu
    1670                1675                1680

Thr Val Glu Glu Glu Glu Glu Ala Gln Pro Ala Val Val Glu Ala
    1685                1690                1695

Pro Asn Gly Asp Leu Thr Pro Glu Met Ile Leu Ser Met Met Asp
    1700                1705                1710

Arg

<210> 273

<211>  706

<212>  PRT

<213>  HeLa

<400>  273

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly

210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Glu Gly Glu Ala Val Glu
        435                 440                 445

Ala Ile Val Glu Glu Ser Glu Thr Phe Ile Lys Gly Lys Glu Arg Lys
    450                 455                 460

Thr Tyr Gln Arg Arg Arg Glu Gly Gly Gln Glu Asp Glu Ala Cys His
465                 470                 475                 480

Ile Ala Pro Asn Gln Ala Asp Gly Gly Glu Val Val Gln Asp Val Asn
                485                 490                 495

Ser Gly Val Gln Met Val Met Met Glu Gln Leu Asp Pro Thr Leu Leu
            500                 505                 510

Gln Met Lys Thr Glu Val Met Glu Gly Ala Val Pro Gln Glu Thr Glu
        515                 520                 525

Ala Thr Val Asp Asp Thr Gln Ile Ile Thr Leu Gln Val Val Asn Met
    530                 535                 540

Glu Glu Gln Pro Ile Asn Leu Gly Glu Leu Gln Leu Val Gln Val Pro
545                 550                 555                 560

Val Pro Val Thr Val Pro Val Ala Thr Thr Ser Val Glu Glu Leu Gln
                565                 570                 575

Gly Ala Tyr Glu Asn Glu Val Ser Lys Gly Gly Leu Gln Glu Gly Glu
            580                 585                 590

Pro Met Ile Cys His Thr Leu Pro Leu Pro Glu Gly Phe Gln Val Val
        595                 600                 605

Lys Val Gly Ala Asn Gly Glu Val Glu Thr Leu Glu Gln Gly Glu Leu
    610                 615                 620

Gln Pro Gln Glu Asp Pro Asn Trp Gln Lys Asp Pro Asp Tyr Gln Pro
625                 630                 635                 640

Pro Ala Lys Lys Thr Lys Lys Asn Lys Lys Ser Lys Leu Arg Tyr Thr
                645                 650                 655

Glu Glu Gly Lys Asp Val Asp Val Ser Val Tyr Asp Phe Glu Glu Glu
            660                 665                 670

Gln Gln Glu Gly Leu Leu Ser Glu Val Asn Ala Glu Lys Val Val Gly
        675                 680                 685

Asn Met Lys Pro Pro Lys Pro Thr Lys Ile Lys Lys Lys Gly Val Lys
    690                 695                 700

Lys Thr
705

<210> 274

<211> 631

<212> PRT

<213> HeLa

<400> 274

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Glu Gly Glu Ala Val Glu
        435                 440                 445

Ala Ile Val Glu Glu Ser Glu Thr Phe Ile Lys Gly Lys Glu Arg Lys

450                 455                 460

Thr Tyr Gln Arg Arg Arg Glu Gly Gly Gln Glu Asp Glu Ala Cys His
465                 470                 475                 480

Ile Ala Pro Asn Gln Ala Asp Gly Gly Glu Val Val Gln Asp Val Asn
                485                 490                 495

Ser Gly Val Gln Met Val Met Met Glu Gln Leu Asp Pro Thr Leu Leu
            500                 505                 510

Gln Met Lys Thr Glu Val Met Glu Gly Ala Val Pro Gln Glu Thr Glu
        515                 520                 525

Ala Thr Val Asp Asp Thr Gln Ile Ile Thr Leu Gln Val Val Asn Met
    530                 535                 540

Glu Glu Gln Pro Ile Asn Leu Gly Glu Leu Gln Leu Val Gln Val Pro
545                 550                 555                 560

Val Pro Val Thr Val Pro Val Ala Thr Thr Ser Val Glu Glu Leu Gln
                565                 570                 575

Gly Ala Tyr Glu Asn Glu Val Ser Lys Gly Gly Leu Gln Glu Gly Glu
            580                 585                 590

Pro Met Ile Cys His Thr Leu Pro Leu Pro Glu Gly Phe Gln Val Val
        595                 600                 605

Lys Val Gly Ala Asn Gly Glu Val Glu Thr Leu Glu Gln Gly Glu Leu
    610                 615                 620

Gln Pro Gln Glu Asp Pro Asn
625                 630

<210>  275

<211>  1006

<212>  PRT

<213>  HeLa

<400>  275

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ser Val Glu Glu Leu Gln
        435                 440                 445

Gly Ala Tyr Glu Asn Glu Val Ser Lys Gly Gly Leu Gln Glu Gly Glu
    450                 455                 460

Pro Met Ile Cys His Thr Leu Pro Leu Pro Glu Gly Phe Gln Val Val
465                 470                 475                 480

Lys Val Gly Ala Asn Gly Glu Val Glu Thr Leu Glu Gln Gly Glu Leu
                485                 490                 495

Gln Pro Gln Glu Asp Pro Asn Met Ser Arg Leu Asp Lys Ser Lys Val
            500                 505                 510

Ile Asn Ser Ala Leu Glu Leu Leu Asn Glu Val Gly Ile Glu Gly Leu
        515                 520                 525

Thr Thr Arg Lys Leu Ala Gln Lys Leu Gly Val Glu Gln Pro Thr Leu
    530                 535                 540

Tyr Trp His Val Lys Asn Lys Arg Ala Leu Leu Asp Ala Leu Ala Ile
545                 550                 555                 560

Glu Met Leu Asp Arg His His Thr His Phe Cys Pro Leu Glu Gly Glu
                565                 570                 575

Ser Trp Gln Asp Phe Leu Arg Asn Asn Ala Lys Ser Phe Arg Cys Ala
            580                 585                 590

Leu Leu Ser His Arg Asp Gly Ala Lys Val His Leu Gly Thr Arg Pro
        595                 600                 605

Thr Glu Lys Gln Tyr Glu Thr Leu Glu Asn Gln Leu Ala Phe Leu Cys
    610                 615                 620

Gln Gln Gly Phe Ser Leu Glu Asn Ala Leu Tyr Ala Leu Ser Ala Val
625                 630                 635                 640

Gly His Phe Thr Leu Gly Cys Val Leu Glu Asp Gln Glu His Gln Val
                645                 650                 655

Ala Lys Glu Glu Arg Glu Thr Pro Thr Thr Asp Ser Met Pro Pro Leu
            660                 665                 670

Leu Arg Gln Ala Ile Glu Leu Phe Asp His Gln Gly Ala Glu Pro Ala
        675                 680                 685

Phe Leu Phe Gly Leu Glu Leu Ile Ile Cys Gly Leu Glu Lys Gln Leu
    690                 695                 700

Lys Cys Glu Ser Gly Asp Ser Gly Gly Gly Gly Ser Gly Gly Gly Gly
705                 710                 715                 720

Ser Gly Gly Gly Gly Ser Gly Gly Gly Gly Ser Gly Gly Gly Gly Ser
                725                 730                 735

Arg Ser Arg Leu Asp Lys Ser Lys Val Ile Asn Ser Ala Leu Glu Leu
            740                 745                 750

Leu Asn Glu Val Gly Ile Glu Gly Leu Thr Thr Arg Lys Leu Ala Gln
        755                 760                 765

Lys Leu Gly Val Glu Gln Pro Thr Leu Tyr Trp His Val Lys Asn Lys

770                 775                 780

Arg Ala Leu Leu Asp Ala Leu Ala Ile Glu Met Leu Asp Arg His His
785                 790                 795                 800

Thr His Phe Cys Pro Leu Glu Gly Glu Ser Trp Gln Asp Phe Leu Arg
                805                 810                 815

Asn Asn Ala Lys Ser Phe Arg Cys Ala Leu Leu Ser His Arg Asp Gly
            820                 825                 830

Ala Lys Val His Leu Gly Thr Arg Pro Thr Glu Lys Gln Tyr Glu Thr
        835                 840                 845

Leu Glu Asn Gln Leu Ala Phe Leu Cys Gln Gln Gly Phe Ser Leu Glu
    850                 855                 860

Asn Ala Leu Tyr Ala Leu Ser Ala Val Gly His Phe Thr Leu Gly Cys
865                 870                 875                 880

Val Leu Glu Asp Gln Glu His Gln Val Ala Lys Glu Glu Arg Glu Thr
                885                 890                 895

Pro Thr Thr Asp Ser Met Pro Pro Leu Leu Arg Gln Ala Ile Glu Leu
            900                 905                 910

Phe Asp His Gln Gly Ala Glu Pro Ala Phe Leu Phe Gly Leu Glu Leu
        915                 920                 925

Ile Ile Cys Gly Leu Glu Lys Gln Leu Lys Cys Glu Ser Gly Gly Pro
    930                 935                 940

Ala Ser Val Glu Glu Leu Gln Gly Ala Tyr Glu Asn Glu Val Ser Lys
945                 950                 955                 960

Gly Gly Leu Gln Glu Gly Glu Pro Met Ile Cys His Thr Leu Pro Leu
                965                 970                 975

Pro Glu Gly Phe Gln Val Val Lys Val Gly Ala Asn Gly Glu Val Glu
            980                 985                 990

Thr Leu Glu Gln Gly Glu Leu Gln  Pro Gln Glu Asp Pro  Asn
        995                 1000                1005

<210>  276

<211>  578

<212>  PRT

<213>  HeLa

<400>  276

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ser Val Glu Glu Leu Gln
        435                 440                 445

Gly Ala Tyr Glu Asn Glu Val Ser Lys Gly Gly Leu Gln Glu Gly Glu
    450                 455                 460

Pro Met Ile Cys His Thr Leu Pro Leu Pro Glu Gly Phe Gln Val Val
465                 470                 475                 480

Lys Val Gly Ala Asn Gly Glu Val Glu Thr Leu Glu Gln Gly Glu Leu
                485                 490                 495

Gln Pro Gln Glu Asp Pro Asn Trp Gln Lys Asp Pro Asp Tyr Gln Pro
            500                 505                 510

Pro Ala Lys Lys Thr Lys Lys Asn Lys Lys Ser Lys Leu Arg Tyr Thr
        515                 520                 525

Glu Glu Gly Lys Asp Val Asp Val Ser Val Tyr Asp Phe Glu Glu Glu
    530                 535                 540

Gln Gln Glu Gly Leu Leu Ser Glu Val Asn Ala Glu Lys Val Val Gly
545                 550                 555                 560

Asn Met Lys Pro Pro Lys Pro Thr Lys Ile Lys Lys Lys Gly Val Lys
                565                 570                 575

Lys Thr

<210>  277

<211>  483

<212>  PRT

<213>  HeLa

<400>  277

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly

85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ser Val Glu Glu Leu Gln
        435                 440                 445

Gly Ala Tyr Glu Asn Glu Val Ser Lys Gly Gly Leu Gln Glu Gly Glu
    450                 455                 460

Pro Met Ile Cys His Thr Leu Pro Leu Pro Glu Gly Phe Gln Val Val
465                 470                 475                 480

Lys Val Gly

<210> 278

<211> 483

<212> PRT

<213> HeLa

<400> 278

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Gly Glu Pro Met Ile Cys
        435                 440                 445

His Thr Leu Pro Leu Pro Glu Gly Phe Gln Val Val Lys Val Gly Ala
    450                 455                 460

Asn Gly Glu Val Glu Thr Leu Glu Gln Gly Glu Leu Gln Pro Gln Glu
465                 470                 475                 480

Asp Pro Asn

<210>  279

<211>  463

<212>  PRT

<213>  HeLa

<400>  279

Met Ser Arg Leu Asp Lys Ser Lys Val Ile Asn Ser Ala Leu Glu Leu

1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Gly Glu Pro Met Ile Cys
        435                 440                 445

His Thr Leu Pro Leu Pro Glu Gly Phe Gln Val Val Lys Val Gly
    450                 455                 460

<210>  280

<211>  903

<212>  PRT

<213>  HeLa

<400>  280

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Cys Glu Leu Cys Ser Tyr
        435                 440                 445

Thr Cys Pro Arg Arg Ser Asn Leu Asp Arg His Met Lys Ser His Thr
    450                 455                 460

Asp Glu Arg Pro His Lys Cys His Leu Cys Gly Arg Ala Phe Arg Thr
465                 470                 475                 480

Val Thr Leu Leu Arg Asn His Leu Asn Thr His Thr Gly Thr Arg Pro
                485                 490                 495

His Lys Cys Pro Asp Cys Asp Met Ala Phe Val Thr Ser Gly Glu Leu

            500                 505                 510

Val Arg His Arg Arg Tyr Lys His Thr His Glu Lys Pro Phe Lys Cys
        515                 520                 525

Ser Met Cys Asp Tyr Ala Ser Val Glu Val Ser Lys Leu Lys Arg His
    530                 535                 540

Ile Arg Ser His Thr Gly Glu Arg Pro Phe Gln Cys Ser Leu Cys Ser
545                 550                 555                 560

Tyr Ala Ser Arg Asp Thr Tyr Lys Leu Lys Arg His Met Arg Thr His
                565                 570                 575

Ser Gly Glu Lys Pro Tyr Glu Cys Tyr Ile Cys His Ala Arg Phe Thr
            580                 585                 590

Gln Ser Gly Thr Met Lys Met His Ile Leu Gln Lys His Thr Glu Asn
        595                 600                 605

Val Ala Lys Phe His Cys Pro His Cys Asp Thr Val Ile Ala Arg Lys
    610                 615                 620

Ser Asp Leu Gly Val His Leu Arg Lys Gln His Ser Tyr Ile Glu Gln
625                 630                 635                 640

Gly Lys Lys Cys Arg Tyr Cys Asp Ala Val Phe His Glu Arg Tyr Ala
                645                 650                 655

Leu Ile Gln His Gln Lys Ser His Lys Asn Glu Lys Arg Phe Lys Cys
            660                 665                 670

Asp Gln Cys Asp Tyr Ala Cys Arg Gln Glu Arg His Met Val Met His
        675                 680                 685

Lys Arg Thr His Thr Gly Glu Lys Pro Tyr Ala Cys Ser His Cys Asp
    690                 695                 700

Lys Thr Phe Arg Gln Lys Gln Leu Leu Asp Met His Phe Lys Arg Tyr
705                 710                 715                 720

His Asp Pro Asn Phe Val Pro Ala Ala Phe Val Cys Ser Lys Cys Gly
                725                 730                 735

Lys Thr Phe Thr Arg Arg Asn Thr Met Ala Arg His Ala Asp Asn Cys
            740                 745                 750

Ser Gly Leu Asp Gly Gly Glu Gly Glu Asn Gly Gly Glu Thr Lys Lys
        755                 760                 765

Gly Lys Arg Gly Arg Lys Arg Lys Met Arg Ser Lys Lys Glu Asp Ser
    770                 775                 780

Ser Asp Ser Glu Glu Asn Ala Glu Pro Asp Leu Asp Asp Asn Glu Asp
785                 790                 795                 800

Glu Glu Glu Thr Ala Val Glu Ile Glu Ala Glu Pro Glu Val Ser Ala
                805                 810                 815

Glu Ala Pro Ala Pro Pro Pro Ser Lys Lys Arg Arg Gly Arg Pro Pro
            820                 825                 830

Gly Lys Ala Ala Thr Gln Thr Lys Gln Ser Gln Pro Ala Ala Ile Ile
        835                 840                 845

Gln Val Glu Asp Gln Asn Thr Gly Glu Ile Glu Asn Ile Ile Val Glu
    850                 855                 860

Val Lys Lys Glu Pro Asp Ala Glu Thr Val Glu Glu Glu Glu Glu Ala
865                 870                 875                 880

Gln Pro Ala Val Val Glu Ala Pro Asn Gly Asp Leu Thr Pro Glu Met
                885                 890                 895

Ile Leu Ser Met Met Asp Arg
            900

<210>  281

<211>  463

<212>  PRT

<213>  HeLa

<400>  281

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Leu Pro Glu Gly Phe Gln
        435                 440                 445

Val Val Lys Val Gly Ala Asn Gly Glu Val Glu Thr Leu Glu Gln
    450                 455                 460

<210>  282

<211>  227

<212>  PRT

<213>  HeLa

<400>  282

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys

35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Pro Gly Gln Pro Leu Asp Leu Ser Cys Lys Arg
    210                 215                 220

Pro Arg Pro
225

<210>  283

<211>  229

<212>  PRT

<213>  HeLa

<400>  283

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Pro Gly Gln Pro Leu Asp Leu Ser Cys
    210                 215                 220

Lys Arg Pro Arg Pro
225

<210>  284

<211>  220

<212>  PRT

<213>  HeLa

<400>  284

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Met Trp Arg Pro Trp Arg Asn
    210                 215                 220

<210>  285

<211>  223

<212>  PRT

<213>  HeLa

<400>  285

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp

195                 200                 205

Gly Asn Lys Gly Gly Pro Ala Ser Met Trp Arg Pro Trp Arg Asn
    210                 215                 220

<210>  286

<211>  227

<212>  PRT

<213>  HeLa

<400>  286

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Pro Gly Gln Pro Leu Asp Leu Ser Cys Arg Arg
    210                 215                 220

Pro Arg Pro
225

<210>  287

<211>  227

<212>  PRT

<213>  HeLa

<400>  287

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gln Thr Val Pro Leu Asp Leu Ser Cys Lys Arg
    210                 215                 220

Pro Arg Pro
225

<210>  288

<211>  227

<212>  PRT

<213>  HeLa

<400>  288

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gln Thr Val Pro Leu Asp Leu Ser Cys Arg Arg
    210                 215                 220

Pro Arg Pro
225

<210>  289

<211>  229

<212>  PRT

<213>  HeLa

<400>  289

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln

65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Gln Thr Val Pro Leu Asp Leu Ser Cys
    210                 215                 220

Arg Arg Pro Arg Pro
225

<210>  290

<211>  223

<212>  PRT

<213>  HeLa

<400>  290

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Ser Met Trp Arg Pro Trp Arg Asn
    210                 215                 220

<210>  291

<211>  229

<212>  PRT

<213>  HeLa

<400>  291

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Gln Thr Val Pro Leu Asp Leu Ser Cys
    210                 215                 220

Arg Arg Pro Arg Pro
225

<210>  292

<211>  367

<212>  PRT

<213>  HeLa

<400>  292

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Met Ala Thr Gln Val Met Gly Gln Ser
    210                 215                 220

Ser Gly Gly Gly Gly Leu Phe Thr Ser Ser Gly Asn Ile Gly Met Ala

225                 230                 235                 240

Leu Pro Asn Asp Met Tyr Asp Leu His Asp Leu Ser Lys Ala Glu Leu
                245                 250                 255

Ala Ala Pro Gln Leu Ile Met Leu Ala Asn Val Ala Leu Thr Gly Glu
            260                 265                 270

Val Asn Gly Ser Cys Cys Asp Tyr Leu Val Gly Glu Glu Arg Gln Met
        275                 280                 285

Ala Glu Leu Met Pro Val Gly Asp Asn Asn Phe Ser Asp Ser Glu Glu
    290                 295                 300

Gly Glu Gly Leu Glu Glu Ser Ala Asp Ile Lys Gly Glu Pro His Gly
305                 310                 315                 320

Leu Glu Asn Met Glu Leu Arg Ser Leu Glu Leu Ser Val Val Glu Pro
                325                 330                 335

Gln Pro Val Phe Glu Ala Ser Gly Ala Pro Asp Ile Tyr Ser Ser Asn
            340                 345                 350

Lys Asp Leu Pro Pro Glu Thr Pro Gly Ala Glu Asp Lys Gly Lys
        355                 360                 365

<210>  293

<211>  360

<212>  PRT

<213>  HeLa

<400>  293

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Gln Asn Thr Arg Glu Asn Leu Thr Gly
    210                 215                 220

Ile Asn Ser Thr Val Glu Glu Pro Val Ser Pro Met Leu Pro Pro Ser
225                 230                 235                 240

Ala Val Glu Glu Arg Glu Ala Val Ser Lys Thr Ala Leu Ala Ser Pro
                245                 250                 255

Pro Ala Thr Met Ala Ala Asn Glu Ser Gln Glu Ile Asp Glu Asp Glu
            260                 265                 270

Gly Ile His Ser His Glu Gly Ser Asp Leu Ser Asp Asn Met Ser Glu
        275                 280                 285

Gly Ser Asp Asp Ser Gly Leu His Gly Ala Arg Pro Val Pro Gln Glu
    290                 295                 300

Ser Ser Arg Lys Asn Ala Lys Glu Ala Leu Ala Val Lys Ala Ala Lys
305                 310                 315                 320

Gly Asp Phe Val Cys Ile Phe Cys Asp Arg Ser Phe Arg Lys Gly Lys
                325                 330                 335

Asp Tyr Ser Lys His Leu Asn Arg His Leu Val Asn Val Tyr Tyr Leu
            340                 345                 350

Glu Glu Ala Ala Gln Gly Gln Glu
        355                 360

<210>  294

<211>  504

<212>  PRT

<213>  HeLa

<400>  294

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Met Asp Leu Val Leu Asp Asp Ser Lys
    210                 215                 220

Arg Val Ala Lys Arg Lys Leu Ile Glu Gln Asn Arg Glu Arg Arg Arg
225                 230                 235                 240

Lys Glu Glu Met Ile Arg Ser Leu Gln Gln Arg Pro Glu Pro Thr Pro
                245                 250                 255

Glu Glu Trp Asp Leu Ile His Val Ala Thr Glu Ala His Arg Ser Thr
            260                 265                 270

Asn Ala Gln Gly Ser His Trp Lys Gln Arg Arg Lys Phe Leu Pro Asp
        275                 280                 285

Asp Ile Gly Gln Ser Pro Ile Val Ser Met Pro Asp Gly Asp Lys Val
    290                 295                 300

Asp Leu Glu Ala Phe Ser Glu Phe Thr Lys Ile Ile Thr Pro Ala Ile
305                 310                 315                 320

Thr Arg Val Val Asp Phe Ala Lys Lys Leu Pro Met Phe Ser Glu Leu
                325                 330                 335

Pro Cys Glu Asp Gln Ile Ile Leu Leu Lys Gly Cys Cys Met Glu Ile
            340                 345                 350

Met Ser Leu Arg Ala Ala Val Arg Tyr Asp Pro Glu Ser Asp Thr Leu
        355                 360                 365

Thr Leu Ser Gly Glu Met Ala Val Lys Arg Glu Gln Leu Lys Asn Gly
    370                 375                 380

Gly Leu Gly Val Val Ser Asp Ala Ile Phe Glu Leu Gly Lys Ser Leu
385                 390                 395                 400

Ser Ala Phe Asn Leu Asp Asp Thr Glu Val Ala Leu Leu Gln Ala Val

405                 410                 415

Leu Leu Met Ser Thr Asp Arg Ser Gly Leu Leu Cys Val Asp Lys Ile
            420                 425                 430

Glu Lys Ser Gln Glu Ala Tyr Leu Leu Ala Phe Glu His Tyr Val Asn
        435                 440                 445

His Arg Lys His Asn Ile Pro His Phe Trp Pro Lys Leu Leu Met Lys
    450                 455                 460

Val Thr Asp Leu Arg Met Ile Gly Ala Cys His Ala Ser Arg Phe Leu
465                 470                 475                 480

His Met Lys Val Glu Cys Pro Thr Glu Leu Phe Pro Pro Leu Phe Leu
                485                 490                 495

Glu Val Phe Glu Asp Gln Glu Val
            500

<210>  295

<211>  234

<212>  PRT

<213>  HeLa

<400>  295

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Ser Met Trp Arg Pro Trp Arg Asn Gly
    210                 215                 220

Pro Ala Ser Met Trp Arg Pro Trp Arg Asn
225                 230

<210>  296

<211>  246

<212>  PRT

<213>  HeLa

<400>  296

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Gln Thr Val Pro Leu Asp Leu Ser Cys
    210                 215                 220

Lys Arg Pro Arg Pro Gly Pro Ala Gln Thr Val Pro Leu Asp Leu Ser
225                 230                 235                 240

Cys Arg Arg Pro Arg Pro
                245

<210>  297

<211>  256

<212>  PRT

<213>  HeLa

<400>  297

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu
        115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Ser Met Trp Arg Pro Trp Arg Asn Gly
    210                 215                 220

Pro Ala Ser Met Trp Arg Pro Trp Arg Asn Gly Pro Ala Ser Met Trp
225                 230                 235                 240

Arg Pro Trp Arg Asn Gly Pro Ala Ser Met Trp Arg Pro Trp Arg Asn
                245                 250                 255

<210>  298

<211>  239

<212>  PRT

<213>  HeLa

<400>  298

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Ala Pro Asn Thr Gln Thr Ala Ser Pro Arg Ala Leu Ala Asp Ser
    210                 215                 220

Leu Met Gln Leu Ala Arg Gln Val Ser Arg Leu Glu Ser Gly Gln
225                 230                 235

<210>  299

<211>  243

<212>  PRT

<213>  HeLa

<400>  299

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Ala Pro Asn Thr Gln Thr Ala Ser Pro Arg Ala
    210                 215                 220

Leu Ala Asp Ser Leu Met Gln Leu Ala Arg Gln Val Ser Arg Leu Glu
225                 230                 235                 240

Ser Gly Gln

<210> 300

<211> 265

<212> PRT

<213> HeLa

<400> 300

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Ala Pro Asn Thr Gln
225                 230                 235                 240

Thr Ala Ser Pro Arg Ala Leu Ala Asp Ser Leu Met Gln Leu Ala Arg
                245                 250                 255

Gln Val Ser Arg Leu Glu Ser Gly Gln
            260                 265

<210>  301

<211>  472

<212>  PRT

<213>  HeLa

<400>  301

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His

50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ala Pro Asn Thr Gln Thr
        435                 440                 445

Ala Ser Pro Arg Ala Leu Ala Asp Ser Leu Met Gln Leu Ala Arg Gln
    450                 455                 460

Val Ser Arg Leu Glu Ser Gly Gln
465                 470

<210>  302

<211>  477

<212>  PRT

<213>  HeLa

<400>  302

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Leu Gln Arg Val Lys Gln
        435                 440                 445

Leu Glu Asp Lys Val Glu Glu Leu Leu Ser Lys Asn Tyr His Leu Glu
    450                 455                 460

Asn Glu Val Ala Arg Leu Lys Lys Leu Val Gly Glu Arg
465                 470                 475

<210>  303

<211>  560

<212>  PRT

<213>  HeLa

<400>  303

Met Ser Arg Leu Asp Lys Ser Lys Val Ile Asn Ser Ala Leu Glu Leu

1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Gly Glu Pro Leu Leu Ala
        435                 440                 445

Gln Gln His Ile Glu Gly His Tyr Gln Val Asp Pro Ser Leu Phe Lys
    450                 455                 460

Pro Asn Ala Asp Phe Leu Leu Arg Val Ser Gly Met Ser Met Lys Asp
465                 470                 475                 480

Ile Gly Ile Met Asp Gly Asp Leu Leu Ala Val His Lys Thr Gln Asp
                485                 490                 495

Val Arg Asn Gly Gln Val Val Val Ala Arg Ile Asp Asp Glu Val Thr
            500                 505                 510

Val Lys Arg Leu Lys Lys Gln Gly Asn Lys Val Glu Leu Leu Pro Glu
        515                 520                 525

Asn Ser Glu Phe Lys Pro Ile Val Val Asp Leu Arg Gln Gln Ser Phe
    530                 535                 540

Thr Ile Glu Gly Leu Ala Val Gly Val Ile Arg Asn Gly Asp Trp Leu
545                 550                 555                 560

<210>  304

<211>  477

<212>  PRT

<213>  HeLa

<400>  304

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu

405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Leu Gln Arg Val Lys Gln
        435                 440                 445

Leu Glu Asp Lys Val Glu Glu Leu Leu Ser Lys Asn Tyr His Leu Glu
    450                 455                 460

Asn Glu Val Ala Arg Leu Lys Lys Leu Val Gly Glu Arg
465                 470                 475

<210>  305

<211>  560

<212>  PRT

<213>  HeLa

<400>  305

Met Ser Arg Leu Asp Lys Ser Lys Val Ile Asn Gly Ala Leu Glu Leu
1               5                   10                  15

Leu Asn Gly'Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Gly Glu Pro Leu Leu Ala
        435                 440                 445

Gln Gln His Ile Glu Gly His Tyr Gln Val Asp Pro Ser Leu Phe Lys
    450                 455                 460

Pro Asn Ala Asp Phe Leu Leu Arg Val Ser Gly Met Ser Met Lys Asp
465                 470                 475                 480

Ile Gly Ile Met Asp Gly Asp Leu Leu Ala Val His Lys Thr Gln Asp
                485                 490                 495

Val Arg Asn Gly Gln Val Val Val Ala Arg Ile Asp Asp Glu Val Thr
            500                 505                 510

Val Lys Arg Leu Lys Lys Gln Gly Asn Lys Val Glu Leu Leu Pro Glu
        515                 520                 525

Asn Ser Glu Phe Lys Pro Ile Val Val Asp Leu Arg Gln Gln Ser Phe
    530                 535                 540

Thr Ile Glu Gly Leu Ala Val Gly Val Ile Arg Asn Gly Asp Trp Leu
545                 550                 555                 560

<210>  306

<211>  535

<212>  PRT

<213>  HeLa

<400>  306

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ala Gln Gln
            260                 265                 270

Thr Leu Met Trp His Val Lys Asn Lys Arg Ala Leu Leu Asp Ala Leu

        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ile Leu Trp His Glu Met
        435                 440                 445

Trp His Glu Gly Leu Glu Glu Ala Ser Arg Leu Tyr Phe Gly Glu Arg
    450                 455                 460

Asn Val Lys Gly Met Phe Glu Val Leu Glu Pro Leu His Ala Met Met
465                 470                 475                 480

Glu Arg Gly Pro Gln Thr Leu Lys Glu Thr Ser Phe Asn Gln Ala Tyr
                485                 490                 495

Gly Arg Asp Leu Met Glu Ala Gln Glu Trp Cys Arg Lys Tyr Met Lys
            500                 505                 510

Ser Gly Asn Val Lys Asp Leu Leu Gln Ala Trp Asp Leu Tyr Tyr His
        515                 520                 525

Val Phe Arg Arg Ile Ser Lys
    530                 535

<210>  307

<211>  552

<212>  PRT

<213>  HeLa

<400>  307

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ala Gln Gln
            260                 265                 270

Thr Leu Met Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Gly Val Gln Val Glu Thr
        435                 440                 445

Ile Ser Pro Gly Asp Gly Arg Thr Phe Pro Lys Arg Gly Gln Thr Cys
    450                 455                 460

Val Val His Tyr Thr Gly Met Leu Glu Asp Gly Lys Lys Phe Asp Ser
465                 470                 475                 480

Ser Arg Asp Arg Asn Lys Pro Phe Lys Phe Met Leu Gly Lys Gln Glu
                485                 490                 495

Val Ile Arg Gly Trp Glu Glu Gly Val Ala Gln Met Ser Val Gly Gln
            500                 505                 510

Arg Ala Lys Leu Thr Ile Ser Pro Asp Tyr Ala Tyr Gly Ala Thr Gly
        515                 520                 525

His Pro Gly Ile Ile Pro Pro His Ala Thr Leu Val Phe Asp Val Glu
    530                 535                 540

Leu Leu Lys Leu Glu Thr Ser Tyr
545                 550

<210>  308

<211>  535

<212>  PRT

<213>  HeLa

<400>  308

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ser Gln Pro Thr Leu Tyr Arg Asn Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly

85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ser Gln Pro
            260                 265                 270

Thr Leu Tyr Arg Asn Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ile Leu Trp His Glu Met
        435                 440                 445

Trp His Glu Gly Leu Glu Glu Ala Ser Arg Leu Tyr Phe Gly Glu Arg
    450                 455                 460

Asn Val Lys Gly Met Phe Glu Val Leu Glu Pro Leu His Ala Met Met
465                 470                 475                 480

Glu Arg Gly Pro Gln Thr Leu Lys Glu Thr Ser Phe Asn Gln Ala Tyr
                485                 490                 495

Gly Arg Asp Leu Met Glu Ala Gln Glu Trp Cys Arg Lys Tyr Met Lys
            500                 505                 510

Ser Gly Asn Val Lys Asp Leu Leu Gln Ala Trp Asp Leu Tyr Tyr His
        515                 520                 525

Val Phe Arg Arg Ile Ser Lys
    530                 535

<210>  309

<211>  552

<212>  PRT

<213>  HeLa

<400>  309

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ser Gln Pro Thr Leu Tyr Arg Asn Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ser Gln Pro
            260                 265                 270

Thr Leu Tyr Arg Asn Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Gly Val Gln Val Glu Thr
        435                 440                 445

Ile Ser Pro Gly Asp Gly Arg Thr Phe Pro Lys Arg Gly Gln Thr Cys
    450                 455                 460

Val Val His Tyr Thr Gly Met Leu Glu Asp Gly Lys Lys Phe Asp Ser
465                 470                 475                 480

Ser Arg Asp Arg Asn Lys Pro Phe Lys Phe Met Leu Gly Lys Gln Glu
                485                 490                 495

Val Ile Arg Gly Trp Glu Glu Gly Val Ala Gln Met Ser Val Gly Gln

            500                 505                 510

Arg Ala Lys Leu Thr Ile Ser Pro Asp Tyr Ala Tyr Gly Ala Thr Gly
        515                 520                 525

His Pro Gly Ile Ile Pro Pro His Ala Thr Leu Val Phe Asp Val Glu
    530                 535                 540

Leu Leu Lys Leu Glu Thr Ser Tyr
545                 550

<210> 310

<211> 477

<212> PRT

<213> HeLa

<400> 310

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ala Gln Gln
            260                 265                 270

Thr Leu Met Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Leu Gln Arg Val Lys Gln
        435                 440                 445

Leu Glu Asp Lys Val Glu Glu Leu Leu Ser Lys Asn Tyr His Leu Glu
    450                 455                 460

Asn Glu Val Ala Arg Leu Lys Lys Leu Val Gly Glu Arg
465                 470                 475

<210>  311

<211>  477

<212>  PRT

<213>  HeLa

<400>  311

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ser Gln Pro Thr Leu Tyr Arg Asn Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ser Gln Pro
            260                 265                 270

Thr Leu Tyr Arg Asn Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His

370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Leu Gln Arg Val Lys Gln
        435                 440                 445

Leu Glu Asp Lys Val Glu Glu Leu Leu Ser Lys Asn Tyr His Leu Glu
    450                 455                 460

Asn Glu Val Ala Arg Leu Lys Lys Leu Val Gly Glu Arg
465                 470                 475

<210>  312

<211>  208

<212>  PRT

<213>  Escherichia coli

<400>  312

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  313

<211>  208

<212>  PRT

<213>  Escherichia coli

<400>  313

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210> 314

<211> 208

<212> PRT

<213> Escherichia coli

<400> 314

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  315

<211>  208

<212>  PRT

<213>  Escherichia coli

<400>  315

Met Ser Arg Leu Asp Lys Ser Lys Val Ile Asn Ser Ala Leu Ala Leu
1               5                   10                  15

Gly Asn Glu Val Gly Ile Glu Gly Val Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly

85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  316

<211>  208

<212>  PRT

<213>  Escherichia coli

<400>  316

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Pro Asp Gly
                85                  90                  95

Ala Lys Glu His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

<210>  317

<211>  571

<212>  PRT

<213>  HeLa

<400>  317

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ser Ala Tyr Ser Arg Ala
        435                 440                 445

Arg Thr Lys Asn Asn Tyr Gly Ser Thr Ile Glu Gly Leu Leu Asp Leu
    450                 455                 460

Pro Asp Asp Asp Ala Pro Glu Glu Ala Gly Leu Ala Ala Pro Arg Leu
465                 470                 475                 480

Ser Phe Leu Pro Ala Gly His Thr Arg Arg Leu Ser Thr Ala Pro Pro
                485                 490                 495

Thr Asp Val Ser Leu Gly Asp Glu Leu His Leu Asp Gly Glu Asp Val
            500                 505                 510

Ala Met Ala His Ala Asp Ala Leu Asp Asp Phe Asp Leu Asp Met Leu
        515                 520                 525

Gly Asp Gly Asp Ser Pro Gly Pro Gly Phe Thr Pro His Asp Ser Ala
    530                 535                 540

Pro Tyr Gly Ala Leu Asp Met Ala Asp Phe Glu Phe Glu Gln Met Phe
545                 550                 555                 560

Thr Asp Ala Leu Gly Ile Asp Glu Tyr Gly Gly
                565                 570

<210>  318

<211>  571

<212>  PRT

<213>  HeLa

<400>  318

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Ser Ala Tyr Ser Arg Ala
        435                 440                 445

Arg Thr Lys Asn Asn Tyr Gly Ser Thr Ile Glu Gly Leu Leu Asp Leu
    450                 455                 460

Pro Asp Asp Asp Ala Pro Glu Glu Ala Gly Leu Ala Ala Pro Arg Leu
465                 470                 475                 480

Ser Phe Leu Pro Ala Gly His Thr Arg Arg Leu Ser Thr Ala Pro Pro
                485                 490                 495

Thr Asp Val Ser Leu Gly Asp Glu Leu His Leu Asp Gly Glu Asp Val
            500                 505                 510

Ala Met Ala His Ala Asp Ala Leu Asp Asp Phe Asp Leu Asp Met Leu
        515                 520                 525

Gly Asp Gly Asp Ser Pro Gly Pro Gly Phe Thr Pro His Asp Ser Ala
    530                 535                 540

Pro Tyr Gly Ala Leu Asp Met Ala Asp Phe Glu Phe Glu Gln Met Phe
545                 550                 555                 560

Thr Asp Ala Leu Gly Ile Asp Glu Tyr Gly Gly
                565                 570

<210>  319

<211>  256

<212>  PRT

<213>  HeLa

<400>  319

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Ala Met Leu Ala Glu Asn His
    50                  55                  60

Thr His Ser Val Pro Arg Ala Asp Asp Asp Trp Arg Ser Phe Leu Ile
65                  70                  75                  80

Gly Asn Ala Arg Ser Phe Arg Gln Ala Leu Leu Ala Tyr Arg Asp Gly
                85                  90                  95

Ala Arg Ile His Ala Gly Thr Arg Pro Gly Ala Pro Gln Met Glu Thr
            100                 105                 110

Ala Asp Ala Gln Leu Arg Phe Leu Cys Glu Ala Gly Phe Ser Ala Gly
        115                 120                 125

Asp Ala Val Asn Ala Leu Met Thr Ile Ser Tyr Phe Thr Val Gly Ala
    130                 135                 140

Val Leu Glu Glu Gln Ala Gly Asp Ser Asp Ala Gly Glu Arg Gly Gly
145                 150                 155                 160

Thr Val Glu Gln Ala Pro Leu Ser Pro Leu Leu Arg Ala Ala Ile Asp
                165                 170                 175

Ala Phe Asp Glu Ala Gly Pro Asp Ala Ala Phe Glu Gln Gly Leu Ala
            180                 185                 190

Val Ile Val Asp Gly Leu Ala Lys Arg Arg Leu Val Val Arg Asn Val
        195                 200                 205

Glu Gly Pro Arg Lys Gly Gly Pro Ala Asp Ala Leu Asp Asp Phe Asp
    210                 215                 220

Leu Asp Met Leu Pro Ala Asp Ala Leu Asp Asp Phe Asp Leu Asp Met
225                 230                 235                 240

Leu Pro Ala Asp Ala Leu Asp Asp Phe Asp Leu Asp Met Leu Pro Gly
                245                 250                 255

<210>  320

<211>  248

<212>  PRT

<213>  HeLa

<400>  320

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys

35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  321

<211>  260

<212>  PRT

<213>  HeLa

<400>  321

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Ala Met Leu Thr Ile Asn His
    50                  55                  60

Thr His Ser Thr Pro Arg Asp Asp Asp Asp Trp Arg Ser Phe Leu Lys
65                  70                  75                  80

Gly Asn Ala Cys Ser Phe Arg Arg Ala Leu Leu Ala Tyr Arg Asp Gly
                85                  90                  95

Ala Arg Ile His Ala Gly Thr Arg Pro Ala Ala Pro Gln Met Glu Lys
            100                 105                 110

Ala Asp Ala Gln Leu Arg Phe Leu Cys Asp Ala Gly Phe Ser Ala Gly
        115                 120                 125

Asp Ala Thr Tyr Ala Leu Met Ala Ile Ser Tyr Phe Thr Val Gly Ala
    130                 135                 140

Val Leu Glu Gln Gln Ala Ser Glu Ala Asp Ala Glu Glu Arg Gly Glu
145                 150                 155                 160

Asp Gln Leu Thr Thr Ser Ala Ser Thr Met Pro Ala Arg Leu Gln Ser
                165                 170                 175

Ala Met Lys Ile Val Tyr Glu Gly Gly Pro Asp Ala Ala Phe Glu Arg
            180                 185                 190

Gly Leu Ala Leu Ile Ile Gly Gly Leu Glu Lys Met Arg Leu Thr Thr
        195                 200                 205

Asn Asp Ile Glu Val Leu Lys Asn Val Gly Gly Pro Ala Asp Ala Leu
    210                 215                 220

Asp Asp Phe Asp Leu Asp Met Leu Pro Ala Asp Ala Leu Asp Asp Phe
225                 230                 235                 240

Asp Leu Asp Met Leu Pro Ala Asp Ala Leu Asp Asp Phe Asp Leu Asp
                245                 250                 255

Met Leu Pro Gly
            260

<210>  322

<211>  249

<212>  PRT

<213>  HeLa

<400>  322

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  323

<211>  254

<212>  PRT

<213>  HeLa

<400>  323

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Met Asn Met Leu Ser Glu Ala Ile Leu Ala Lys His His
    50                  55                  60

Thr Arg Ser Ala Pro Leu Pro Thr Glu Ser Trp Gln Gln Phe Leu Gln
65                  70                  75                  80

Glu Asn Ala Leu Ser Phe Arg Lys Ala Leu Leu Val His Arg Asp Gly
                85                  90                  95

Ala Arg Leu His Ile Gly Thr Ser Pro Thr Pro Pro Gln Phe Glu Gln
            100                 105                 110

Ala Glu Ala Gln Leu Arg Cys Leu Cys Asp Ala Gly Phe Ser Val Glu

115                 120                 125

Glu Ala Leu Phe Ile Leu Gln Ser Ile Ser His Phe Thr Leu Gly Ala
    130                 135                 140

Val Leu Glu Glu Gln Ala Thr Asn Gln Ile Glu Asn Asn His Val Ile
145                 150                 155                 160

Asp Ala Ala Pro Pro Leu Leu Gln Glu Ala Phe Asn Ile Gln Ala Arg
                165                 170                 175

Thr Ser Ala Glu Met Ala Phe His Phe Gly Leu Lys Ser Leu Ile Phe
            180                 185                 190

Gly Phe Ser Ala Gln Leu Asp Glu Lys Lys His Thr Pro Ile Glu Asp
        195                 200                 205

Gly Asn Lys Gly Gly Pro Ala Asp Ala Leu Asp Asp Phe Asp Leu Asp
    210                 215                 220

Met Leu Pro Ala Asp Ala Leu Asp Asp Phe Asp Leu Asp Met Leu Pro
225                 230                 235                 240

Ala Asp Ala Leu Asp Asp Phe Asp Leu Asp Met Leu Pro Gly
                245                 250

<210>  324

<211>  251

<212>  PRT

<213>  HeLa

<400>  324

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Ala Met Leu Ala Glu Arg His
    50                  55                  60

Thr Arg Ser Leu Pro Glu Glu Asn Glu Asp Trp Arg Val Phe Leu Lys
65                  70                  75                  80

Glu Asn Ala Leu Ser Phe Arg Thr Ala Leu Leu Ser Tyr Arg Asp Gly
                85                  90                  95

Ala Arg Ile His Ala Gly Thr Arg Pro Thr Glu Pro Asn Phe Gly Thr
            100                 105                 110

Ala Glu Thr Gln Ile Arg Phe Leu Cys Ala Glu Gly Phe Cys Pro Lys
        115                 120                 125

Arg Ala Val Trp Ala Leu Arg Ala Val Ser His Tyr Val Val Gly Ser
    130                 135                 140

Val Leu Glu Gln Gln Ala Ser Asp Ala Asp Glu Arg Val Pro Asp Arg
145                 150                 155                 160

Pro Asp Val Ser Glu Gln Ala Pro Ser Ser Phe Leu His Asp Leu Phe
                165                 170                 175

His Glu Leu Glu Thr Asp Gly Met Asp Ala Ala Phe Asn Phe Gly Leu
            180                 185                 190

Asp Ser Leu Ile Ala Gly Phe Glu Arg Leu Arg Ser Ser Thr Thr Asp
        195                 200                 205

Gly Gly Pro Ala Asp Ala Leu Asp Asp Phe Asp Leu Asp Met Leu Pro
    210                 215                 220

Ala Asp Ala Leu Asp Asp Phe Asp Leu Asp Met Leu Pro Ala Asp Ala
225                 230                 235                 240

Leu Asp Asp Phe Asp Leu Asp Met Leu Pro Gly
                245                 250

<210>  325

<211>  250

<212>  PRT

<213>  HeLa

<400>  325

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Pro Asn Glu Thr Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Glu Ser Gln Phe Glu Thr
            100                 105                 110

Ser Glu Gln Gln Leu Gln Phe Leu Cys Asp Ala Gly Phe Ser Leu Ser
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Glu Thr Asp Thr Val Ala Tyr Pro Pro Leu Leu Thr Gln Ala Val
                165                 170                 175

Ala Ile Met Asp Ser Asp Asn Gly Asp Ala Ala Phe Leu Phe Val Leu
            180                 185                 190

Asp Val Met Ile Ser Gly Leu Glu Thr Val Leu Lys Ser Ala Lys Gly
        195                 200                 205

Gly Pro Ala Asp Ala Leu Asp Asp Phe Asp Leu Asp Met Leu Pro Ala
    210                 215                 220

Asp Ala Leu Asp Asp Phe Asp Leu Asp Met Leu Pro Ala Asp Ala Leu
225                 230                 235                 240

Asp Asp Phe Asp Leu Asp Met Leu Pro Gly
                245                 250

<210>  326

<211>  250

<212>  PRT

<213>  HeLa

<400>  326

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Ala Asn Glu Ser Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Ala Asn Gln Phe Glu Thr
            100                 105                 110

Ser Glu Gln Gln Leu Gln Phe Leu Cys Asp Ala Gly Phe Thr Leu Thr
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Pro Lys Thr Glu Ile Asn Tyr Pro Pro Leu Leu Thr Gln Ala Ile
                165                 170                 175

Asp Ile Met Asp Ser Asp Asn Gly Glu Ala Ala Phe Leu Phe Val Leu
            180                 185                 190

Asp Val Met Ile Ser Gly Leu Glu Thr Val Leu Asn Asn His His Gly
        195                 200                 205

Gly Pro Ala Asp Ala Leu Asp Asp Phe Asp Leu Asp Met Leu Pro Ala

210                 215                 220

Asp Ala Leu Asp Asp Phe Asp Leu Asp Met Leu Pro Ala Asp Ala Leu
225                 230                 235                 240

Asp Asp Phe Asp Leu Asp Met Leu Pro Gly
                245                 250

<210>  327

<211>  238

<212>  PRT

<213>  HeLa

<400>  327

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Ala Ala Leu Ala Ser Asp Val Leu Asp Arg Glu His
    50                  55                  60

His Ala Ser Phe Pro Glu Ser Asp Glu Lys Trp Asp Ala Phe Leu Leu
65                  70                  75                  80

Arg Asn Ala Arg Ser Phe Arg His Ala Leu His Ala Val Arg Asp Gly
                85                  90                  95

Ala Arg Leu His Ala Glu His His Arg Arg Pro Ser Gly Asp Asp Thr
            100                 105                 110

Asp Glu Gly Ala Glu Ala Ser Gly Gln Gln Val Gly Leu Leu Val Ser
        115                 120                 125

Gln Gly Phe Asp Glu Gln Thr Ala Ile Ser Met Leu Ile Ala Val Ser
    130                 135                 140

Arg Tyr Thr Val Gly Val Val Leu Glu Glu Gln Thr Ala Glu Val Gly
145                 150                 155                 160

Asn Ala Ser Val Pro Glu Arg Asp Arg Glu Phe Asp Phe Gly Leu Thr
                165                 170                 175

Ala Leu Ile Asp Gly Phe Ser His Ser Arg Thr Ala Ser Leu Thr Ser
            180                 185                 190

Ser Glu Ser Gly Gly Pro Ala Asp Ala Leu Asp Asp Phe Asp Leu Asp
        195                 200                 205

Met Leu Pro Ala Asp Ala Leu Asp Asp Phe Asp Leu Asp Met Leu Pro
    210                 215                 220

Ala Asp Ala Leu Asp Asp Phe Asp Leu Asp Met Leu Pro Gly
225                 230                 235

<210>  328

<211>  250

<212>  PRT

<213>  HeLa

<400>  328

Met Ser Arg Leu Asp Lys Glu Gln Val Ile Asp Asn Ala Leu Ile Leu
1               5                   10                  15

Leu Asn Glu Val Gly Ile Glu Gly Leu Thr Thr Arg Lys Leu Ala Gln
            20                  25                  30

Lys Ile Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Pro Asn Glu Thr Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Glu Ser Gln Phe Glu Thr
            100                 105                 110

Ser Glu Gln Gln Leu Gln Phe Leu Cys Asp Ala Gly Phe Ser Leu Ser
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Glu Thr Asp Thr Val Ala Tyr Pro Pro Leu Leu Thr Gln Ala Val
                165                 170                 175

Ala Ile Met Asp Ser Asp Asn Gly Asp Ala Ala Phe Leu Phe Val Leu
            180                 185                 190

Asp Val Met Ile Ser Gly Leu Glu Thr Val Leu Lys Ser Ala Lys Gly
        195                 200                 205

Gly Pro Ala Asp Ala Leu Asp Asp Phe Asp Leu Asp Met Leu Pro Ala
    210                 215                 220

Asp Ala Leu Asp Asp Phe Asp Leu Asp Met Leu Pro Ala Asp Ala Leu
225                 230                 235                 240

Asp Asp Phe Asp Leu Asp Met Leu Pro Gly
                245                 250

<210>  329

<211>  250

<212>  PRT

<213>  HeLa

<400>  329

Met Ser Arg Leu Asp Lys Glu Gln Val Ile Asp Asn Ala Leu Ile Leu
1               5                   10                  15

Leu Asn Glu Val Gly Met Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Glu Thr Ile Leu Gln Lys His His
    50                  55                  60

His His Val Leu Pro Leu Ala Asn Glu Ser Trp Gln Asp Phe Leu Arg
65                  70                  75                  80

Asn Asn Ala Lys Ser Phe Arg Gln Ala Leu Leu Met Tyr Arg Asp Gly
                85                  90                  95

Gly Lys Ile His Ala Gly Thr Arg Pro Ser Ala Asn Gln Phe Glu Thr
            100                 105                 110

Ser Glu Gln Gln Leu Gln Phe Leu Cys Asp Ala Gly Phe Thr Leu Thr
        115                 120                 125

Gln Ala Val Tyr Ala Leu Ser Ser Ile Ala His Phe Thr Leu Gly Ser
    130                 135                 140

Val Leu Glu Thr Gln Glu His Gln Glu Ser Gln Lys Glu Arg Glu Lys
145                 150                 155                 160

Val Pro Lys Thr Glu Ile Asn Tyr Pro Pro Leu Leu Thr Gln Ala Ile
                165                 170                 175

Asp Ile Met Asp Ser Asp Asn Gly Glu Ala Ala Phe Leu Phe Val Leu
            180                 185                 190

Asp Val Met Ile Ser Gly Leu Glu Thr Val Leu Asn Asn His His Gly
        195                 200                 205

Gly Pro Ala Asp Ala Leu Asp Asp Phe Asp Leu Asp Met Leu Pro Ala
    210                 215                 220

Asp Ala Leu Asp Asp Phe Asp Leu Asp Met Leu Pro Ala Asp Ala Leu
225                 230                 235                 240

Asp Asp Phe Asp Leu Asp Met Leu Pro Gly
                245                 250

<210> 330

<211> 249

<212> PRT

<213> HeLa

<400> 330

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln

            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Gln Ser Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210> 331

<211> 249

<212>  PRT

<213>  HeLa

<400>  331

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Val Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Glu Tyr Asp Thr
            100                 105                 110

Val Glu Thr Glu Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  332

<211>  249

<212>  PRT

<213>  HeLa

<400>  332

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Lys
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  333

<211>  250

<212>  PRT

<213>  HeLa

<400>  333

Met Ser Arg Ser Arg Leu Asp Lys Ser Lys Val Ile Asn Ser Ala Leu
1               5                   10                  15

Glu Leu Leu Asn Glu Val Gly Ile Glu Gly Leu Thr Thr Arg Lys Leu
            20                  25                  30

Ala Gln Lys Leu Gly Val Ala Gln Gln Thr Leu Met Trp His Val Lys
        35                  40                  45

Asn Lys Arg Ala Leu Leu Asp Ala Leu Ala Ile Glu Met Leu Asp Arg
    50                  55                  60

His His Thr His Phe Cys Pro Leu Glu Gly Glu Ser Trp Gln Asp Phe
65                  70                  75                  80

Leu Arg Asn Asn Ala Lys Ser Phe Arg Cys Ala Leu Leu Ser His Arg
                85                  90                  95

Asp Gly Ala Lys Val His Leu Gly Thr Arg Pro Thr Glu Lys Gln Tyr
            100                 105                 110

Glu Thr Leu Glu Asn Gln Leu Ala Phe Leu Cys Gln Gln Gly Phe Ser

115                 120                 125

Leu Glu Asn Ala Leu Tyr Ala Leu Ser Ala Val Gly His Phe Thr Leu
    130                 135                 140

Gly Cys Val Leu Glu Asp Gln Glu His Gln Val Ala Lys Glu Glu Arg
145                 150                 155                 160

Glu Thr Pro Thr Thr Asp Ser Met Pro Pro Leu Leu Arg Gln Ala Ile
                165                 170                 175

Glu Leu Phe Asp His Gln Gly Ala Glu Pro Ala Phe Leu Phe Gly Leu
            180                 185                 190

Glu Leu Ile Ile Cys Gly Leu Glu Lys Gln Leu Lys Cys Glu Ser Gly
        195                 200                 205

Gly Pro Ala Asp Ala Leu Asp Asp Phe Asp Leu Asp Met Leu Pro Ala
    210                 215                 220

Asp Ala Leu Asp Asp Phe Asp Leu Asp Met Leu Pro Ala Asp Ala Leu
225                 230                 235                 240

Asp Asp Phe Asp Leu Asp Met Leu Pro Gly
                245                 250

<210>  334

<211>  248

<212>  PRT

<213>  HeLa

<400>  334

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  335

<211>  249

<212>  PRT

<213>  HeLa

<400>  335

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  336

<211>  249

<212>  PRT

<213>  HeLa

<400>  336

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp

210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  337

<211>  249

<212>  PRT

<213>  HeLa

<400>  337

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ile His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  338

<211>  481

<212>  PRT

<213>  HeLa

<400>  338

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210>  339

<211>  481

<212>  PRT

<213>  HeLa

<400>  339

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg

65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210> 340

<211> 481

<212> PRT

<213> HeLa

<400> 340

Met Ser Arg Leu Asp Lys Ser Lys Val Ile Asn Ser Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Gly Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210>  341

<211>  339

<212>  PRT

<213>  HeLa

<400>  341

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Ser Pro Lys Lys Lys Arg Lys Val Asp Gly Gly Gly Ala Leu
    210                 215                 220

Ser Pro Gln His Ser Ala Val Thr Gln Gly Ser Ile Ile Lys Asn Lys
225                 230                 235                 240

Glu Gly Met Asp Ala Lys Ser Leu Thr Ala Trp Ser Arg Thr Leu Val
                245                 250                 255

Thr Phe Lys Asp Val Phe Val Asp Phe Thr Arg Glu Glu Trp Lys Leu
            260                 265                 270

Leu Asp Thr Ala Gln Gln Ile Val Tyr Arg Asn Val Met Leu Glu Asn
        275                 280                 285

Tyr Lys Asn Leu Val Ser Leu Gly Tyr Gln Leu Thr Lys Pro Asp Val
    290                 295                 300

Ile Leu Arg Leu Glu Lys Gly Glu Glu Pro Trp Leu Val Glu Arg Glu
305                 310                 315                 320

Ile His Gln Glu Thr His Pro Asp Ser Glu Thr Ala Phe Glu Ile Lys
                325                 330                 335

Ser Ser Val

<210> 342

<211> 481

<212> PRT

<213> HeLa

<400> 342

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Glu Gln Pro
            260                 265                 270

Thr Leu Tyr Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210>  343

<211>  481

<212>  PRT

<213>  HeLa

<400>  343

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Pro Ile Glu Met Leu Asp Arg His His

50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Ala Gln Gln
            260                 265                 270

Thr Leu Met Trp His Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210>  344

<211>  481

<212>  PRT

<213>  HeLa

<400>  344

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Ser Gln Pro Thr Leu Tyr Arg Asn Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Gly Ala Leu Glu Leu Leu Asn Gly Val Gly Ile Glu
                245                 250                 255

Gly Leu Thr Thr Arg Lys Leu Ala Gln Lys Leu Gly Val Ser Gln Pro
            260                 265                 270

Thr Leu Tyr Arg Asn Val Lys Asn Lys Arg Ala Leu Leu Asp Ala Leu
        275                 280                 285

Pro Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Glu Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp Arg Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210>  345

<211>  481

<212>  PRT

<213>  HeLa

<400>  345

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ala Gln Gln
            260                 265                 270

Thr Leu Met Trp His Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe
        435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210> 346

<211> 481

<212> PRT

<213> HeLa

<400> 346

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ser Gln Pro Thr Leu Tyr Arg Asn Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Asp Ser
        195                 200                 205

Gly Gly Gly Gly Ser Gly Gly Gly Gly Ser Gly Gly Gly Gly Ser Gly
    210                 215                 220

Gly Gly Gly Ser Gly Gly Gly Gly Ser Arg Ser Arg Leu Asp Lys Ser
225                 230                 235                 240

Lys Val Ile Asn Ser Ala Leu Glu Leu Leu Asn Glu Val Gly Ile Glu
                245                 250                 255

Gly Leu Thr Thr Arg Lys Leu Ala Gln Lys Leu Gly Val Ser Gln Pro
            260                 265                 270

Thr Leu Tyr Arg Asn Val Lys Asn Lys Arg Ala Leu Leu Asp Ala Leu
        275                 280                 285

Ala Ile Glu Met Leu Asp Arg His His Thr His Phe Cys Pro Leu Glu
    290                 295                 300

Gly Glu Ser Trp Gln Asp Phe Leu Arg Asn Asn Ala Lys Ser Phe Arg
305                 310                 315                 320

Cys Ala Leu Leu Ser His Arg Asp Gly Ala Lys Val His Leu Gly Thr
                325                 330                 335

Arg Pro Thr Glu Lys Gln Tyr Glu Thr Leu Glu Asn Gln Leu Ala Phe
            340                 345                 350

Leu Cys Gln Gln Gly Phe Ser Leu Glu Asn Ala Leu Tyr Ala Leu Ser
        355                 360                 365

Ala Val Gly His Phe Thr Leu Gly Cys Val Leu Glu Asp Gln Glu His
    370                 375                 380

Gln Val Ala Lys Glu Glu Arg Glu Thr Pro Thr Thr Asp Ser Met Pro
385                 390                 395                 400

Pro Leu Leu Arg Gln Ala Ile Glu Leu Phe Asp His Gln Gly Ala Glu
                405                 410                 415

Pro Ala Phe Leu Phe Gly Leu Glu Leu Ile Ile Cys Gly Leu Glu Lys
            420                 425                 430

Gln Leu Lys Cys Glu Ser Gly Gly Pro Ala Asp Ala Leu Asp Asp Phe

435                 440                 445

Asp Leu Asp Met Leu Pro Ala Asp Ala Leu Asp Asp Phe Asp Leu Asp
    450                 455                 460

Met Leu Pro Ala Asp Ala Leu Asp Asp Phe Asp Leu Asp Met Leu Pro
465                 470                 475                 480

Gly

<210>  347

<211>  248

<212>  PRT

<213>  HeLa

<400>  347

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ser Gln Pro Thr Leu Tyr Arg Asn Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  348

<211>  249

<212>  PRT

<213>  HeLa

<400>  348

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Ala Gln Gln Thr Leu Met Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210> 349

<211> 248

<212> PRT

<213> HeLa

<400> 349

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  350

<211>  248

<212>  PRT

<213>  HeLa

<400>  350

Met Ser Arg Leu Asp Lys Ser Lys Val Ile Asn Ser Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  351

<211>  248

<212>  PRT

<213>  HeLa

<400>  351

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210> 352

<211> 249

<212> PRT

<213> HeLa

<400> 352

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  353

<211>  249

<212>  PRT

<213>  HeLa

<400>  353

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly

                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210> 354

<211> 249

<212> PRT

<213> HeLa

<400> 354

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  355

<211>  249

<212>  PRT

<213>  HeLa

<400> 355

Met Ser Arg Leu Asp Lys Ser Lys Val Ile Asn Ser Ala Leu Ala Leu
1               5                   10                  15

Gly Asn Glu Val Gly Ile Glu Gly Val Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210> 356

<211> 249

<212> PRT

<213> HeLa

<400> 356

Met Ser Arg Leu Asp Lys Ser Lys Val Ile Asn Ser Ala Leu Ala Leu
1               5                   10                  15

Gly Asn Glu Val Gly Ile Glu Gly Val Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His His
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Ser Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser

180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  357

<211>  249

<212>  PRT

<213>  HeLa

<400>  357

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Val Glu Ile Leu Ala Arg His Lys
    50                  55                  60

Asp Tyr Ser Leu Pro Ala Ala Gly Glu Ser Trp Gln Ser Phe Leu Arg
65                  70                  75                  80

Asn Asn Ala Met Ser Phe Arg Arg Ala Leu Leu Arg Tyr Arg Asp Gly
                85                  90                  95

Ala Lys Val His Leu Gly Thr Arg Pro Asp Glu Lys Gln Tyr Asp Thr
            100                 105                 110

Val Glu Thr Gln Leu Arg Phe Met Thr Glu Asn Gly Phe Ser Leu Arg
        115                 120                 125

Asp Gly Leu Tyr Ala Ile Leu Ala Val Ser His Phe Thr Leu Gly Ala
    130                 135                 140

Val Leu Glu Gln Gln Glu His Thr Ala Ala Leu Thr Asp Arg Pro Ala
145                 150                 155                 160

Ala Pro Asp Glu Asn Leu Pro Pro Leu Leu Arg Glu Ala Leu Gln Ile
                165                 170                 175

Met Asp Ser Asp Asp Gly Glu Gln Ala Phe Leu His Gly Leu Glu Ser
            180                 185                 190

Leu Ile Arg Gly Phe Glu Val Gln Leu Thr Ala Leu Leu Gln Ile Val
        195                 200                 205

Pro Ala Asp Ala Leu Asp Asp Phe Asp Leu Asp Met Leu Pro Ala Asp
    210                 215                 220

Ala Leu Asp Asp Phe Asp Leu Asp Met Leu Pro Ala Asp Ala Leu Asp
225                 230                 235                 240

Asp Phe Asp Leu Asp Met Leu Pro Gly
                245

<210>  358

<211>  12

<212>  PRT

<213>  Escherichia coli

<400>  358

Trp Thr Trp Asn Ala Tyr Ala Phe Ala Ala Pro Ser
1               5                   10

<210>  359

<211>  16

<212>  PRT

<213>  Escherichia coli

<400>  359

Trp Thr Trp Asn Ala Tyr Ala Phe Ala Ala Pro Ser Gly Gly Gly Ser
1               5                   10                  15

<210>  360

<211>  20

<212>  PRT

<213>  Escherichia coli

<400>  360

Ser Gly Gly Ala Trp Thr Trp Asn Ala Tyr Ala Phe Ala Ala Pro Ser
1               5                   10                  15

Gly Gly Gly Ser
            20

<210>  361

<211>  20

<212>  PRT

<213>  Escherichia coli

<400>  361

Ser Gly Gly Ala Trp Thr Trp Asn Ala Tyr Ala Phe Ala Ala Ala Ser
1               5                   10                  15

Gly Gly Gly Ser
            20

<210>  362

<211>  20

<212>  PRT

<213>  Escherichia coli

<400>  362

Ser Gly Gly Ala Trp Thr Trp Asn Ala Tyr Ala Phe Ala Ala Pro Ala
1               5                   10                  15

Gly Gly Gly Ser
            20

<210>  363

<211>  20

<212>  PRT

<213>  Escherichia coli

<400>  363

Ser Gly Gly Ala Trp Thr Trp Asn Ala Tyr Ala Phe Ala Ala Pro Ser
1               5                   10                  15

Gly Arg Gly Ser
            20

<210>  364

<211>  20

<212>  PRT

<213>  Escherichia coli

<400>  364

Ser Gly Gly Ala Trp Thr Trp Asn Ala Tyr Ala Phe Ala Ala Pro Ser
1               5                   10                  15

Asp Gly Gly Leu
            20

<210>  365

<211>  20

<212>  PRT

<213>  Escherichia coli

<400>  365

Ser Gly Gly Ala Trp Thr Trp Asn Ala Tyr Ala Phe Ala Ala Pro Ser
1               5                   10                  15

Gly Gly Ala Leu
            20

<210>  366

<211>  20

<212>  PRT

<213>  Escherichia coli

<400>  366

Ser Gly Gly Ala Trp Thr Trp Asn Ala Tyr Ala Phe Ala Ala Pro Ser
1               5                   10                  15

Gly Gly Arg Ser
            20

<210>  367

<211>  20

<212>  PRT

<213>  Escherichia coli

<400>  367

Ser Gly Gly Ala Trp Thr Trp Asn Ala Tyr Ala Phe Ala Ala Pro Ser
1               5                   10                  15

Arg Arg Gly Ser
            20

<210>  368

<211>  20

<212>  PRT

<213>  Escherichia coli

<400>  368

Ser Gly Gly Ala Trp Thr Trp Asn Ala Tyr Ala Phe Ala Ala Pro Ser
1               5                   10                  15

Gly Gly Asp Ser
            20

<210>  369

<211>  20

<212>  PRT

<213>  Escherichia coli

<400>  369

Ser Gly Gly Ala Trp Thr Trp Asn Ala Tyr Ala Phe Ala Ala Pro Ser
1               5                   10                  15

Gly Glu Gly Ser
            20

<210>  370

<211>  20

<212>  PRT

<213>  Escherichia coli

<400>  370

Ser Gly Gly Ala Trp Thr Trp Asn Ala Tyr Ala Phe Ala Ala Pro Ser
1               5                   10                  15

Gly Gly Gly Trp
            20

<210>  371

<211>  20

<212>  PRT

<213>  Escherichia coli

<400>  371

Ser Gly Gly Ala Trp Thr Trp Asn Ala Tyr Ala Phe Ala Ala Pro Ser
1               5                   10                  15

Gly Gly Cys Ser
            20

<210>  372

<211>  42

<212>  DNA

<213>  Escherichia coli

<400>  372

aaatataatg accctcttga taacccaaga gggcattttt ta                    42

<210>  373

<211>  103

<212>  DNA

<213>  Bacteriophage lambda

<400>  373
gactcctgtt gatagatcca gtaatgacct cagaactcca tctggatttg ttcagaacgc      60

tcggttgccg ccgggcgttt tttattggtg agaatccaag cta                       103

<210>  374

<211>  66

<212>  DNA

<213>  Escherichia coli

<400>  374
aggcatcaaa taaaacgaaa ggctcagtcg aaagactggg cctttcgttt tatctgttgt      60

ttgtcg                                                                 66

<210>  375

<211>  45

<212>  DNA

<213>  Escherichia coli

<400>  375
ttaagcagaa ggccatcctg acggatggcc tttttgcgtt tctac                      45

<210>  376

<211>  55

<212>  DNA

<213>  Bacteriophage T7

<400>  376
ctagcataac cccttggggc ctctaaacgg gtcttgaggg gttttttgct gaaag           55

<210> 377

<211> 23

<212> DNA

<213> Bacteriophage T7

<400> 377
cctccctata gtgagtcgta tta 23

<210> 378

<211> 22

<212> DNA

<213> Bacteriophage T3

<400> 378
ggaattaacc ctcactaaag gg 22

<210> 379

<211> 20

<212> DNA

<213> Bacteriophage SP6

<400> 379
atttaggtga cactatagaa 20

<210> 380

<211> 19

<212> DNA

<213> Transposon Tn5

<400> 380
ctgtctctta tacacatct 19

<210> 381

<211> 19

<212> DNA

<213> Transposon Tn5

<400> 381
agatgtgtat aagagacag 19

<210> 382

<211> 23

<212> DNA

<213> Transposon Tn10

<400> 382
ctgatgaatc ccctaatgat ttt 23

<210> 383

<211> 23

<212> DNA

<213> Transposon Tn10

<400> 383
aaaatcatta ggggattcat cag 23

<210> 384

<211> 199

<212> DNA

<213> Transposon Tn7

<400> 384
tgtgggcgga caataaagtc ttaaactgaa caaaatagat ctaaactatg acaataaagt 60

cttaaactag acagaatagt tgtaaactga aatcagtcca gttatgctgt gaaaaagcat 120

actggacttt tgttatggct aaagcaaact cttcattttc tgaagtgcaa attgcccgtc 180

gtattaaaga ggggcgtgg 199

<210> 385

<211> 166

<212> DNA

<213> Transposon Tn7

<400> 385
aaccagataa gtgaaatcta gttccaaact attttgtcat ttttaatttt cgtattagct 60

tacgacgcta cacccagttc ccatctattt tgtcactctt ccctaaataa tccttaaaaa 120

ctccatttcc acccctccca gttcccaact attttgtccg cccaca 166

<210> 386

<211> 21

<212> DNA

<213> Bacteriophage Mu

<400> 386
tgaagcggcg cacgaaaaac g 21

<210> 387

<211> 21

<212> DNA

<213> Bacteriophage Mu

<400> 387
cgtttttcgt gcgccgcttc a 21

<210> 388

<211> 29

<212> DNA

<213> Drosophila mauritiana

<400> 388
taacaggttg gctgataagt ccccggtct 29

<210> 389

<211> 29

<212> DNA

<213> Drosophila mauritiana

<400> 389

agaccgggga cttatcagcc aacctgtta                                          29

<210>  390

<211>  18

<212>  DNA

<213>  Apis cerana

<400>  390
atacgtatta tatatttt                                                    18

<210>  391

<211>  18

<212>  DNA

<213>  Apis cerana

<400>  391
aaaatatata tatactat                                                    18

<210>  392

<211>  627

<212>  DNA

<213>  Poliovirus

<400>  392
ttaaaacagc tctggggttg tacccacccc agaggcccac gtggcggcta gtactccggt      60

attgcggtac ccttgtacgc ctgttttata ctcccttccc gtaacttaga cgcacaaaac     120

caagttcaat agaagggggt acaaaccagt accaccacga acaagcactt ctgtttcccc     180

ggtgatgtcg tatagactgc ttgcgtggtt gaaagcgacg gatccgttat ccgcttatgt     240

acttcgagaa gcccagtacc acctcggaat cttcgatgcg ttgcgctcag cactcaaccc     300

cagagtgtag cttaggctga tgagtctgga catccctcac cggtgacggt ggtccaggct     360

gcgttggcgg cctacctatg gctaacgcca tgggacgcta gttgtgaaca aggtgtgaag     420

agcctattga gctacataag aatcctccgg cccctgaatg cggctaatcc caacctcgga     480

gcaggtggtc acaaaccagt gattggcctg tcgtaacgcg caagtccgtg gcggaaccga     540

ctactttggg tgtccgtgtt tccttttatt ttattgtggc tgcttatggt gacaatcaca     600

gattgttatc ataaagcgaa ttggatt                                         627

<210>  393

<211>  586

<212>  DNA

<213>  Encephalomyocarditis-Virus

<400>  393
cgcccctctc cctccccccc ccctaacgtt actggccgaa gccgcttgga ataaggccgg      60

tgtgcgtttg tctatatgtg attttccacc atattgccgt cttttggcaa tgtgagggcc     120

cggaaacctg gccctgtctt cttgacgagc attcctaggg gtctttcccc tctcgccaaa     180

ggaatgcaag gtctgttgaa tgtcgtgaag gaagcagttc ctctggaagc ttcttgaaga     240

caaacaacgt ctgtagcgac cctttgcagg cagcggaacc ccccacctgg cgacaggtgc     300

ctctgcggcc aaaagccacg tgtataagat acacctgcaa aggcggcaca accccagtgc     360

cacgttgtga gttggatagt tgtggaaaga gtcaaatggc tctcctcaag cgtattcaac     420

aaggggctga aggatgccca gaaggtaccc cattgtatgg gatctgatct ggggcctcgg     480

tgcacatgct ttacatgtgt ttagtcgagg ttaaaaaaac gtctaggccc cccgaaccac     540

ggggacgtgg ttttcctttg aaaaacacga tgataagctt gccaca                    586

## Description

The present invention relates to an insertion element, wherein said insertion element is a recombinant nucleic acid molecule which comprises, in 5' → 3' arrangement: (i) a first recombinase binding site; (ii) a resistance gene, operatively linked to a promoter and, optionally, to transcription control sequences; (iii) a tetracycline-controlled promoter; and (iv) a second recombinase binding site, wherein said tetracycline-controlled promoter is oriented to allow transcription in direction of the second recombinase binding site. The present invention also relates to a method for the generation of a conditional knock-out mutant of a cell, comprising the steps of: (a) introducing a nucleic acid molecule comprising the insertion element of any one of claims 1 to 16 into a cell, wherein the insertion element is transfected into the cell concomitant with purified recombinase; (b) integrating the insertion element into the cellular genome; and (c) selecting cells for resistance against a substrate for which the resistance gene of the insertion element confers resistance; wherein prior to or concomitant with step (a) an expression construct for a tetracycline-sensitive (poly)peptide is introduced into the cell, said (poly)peptide being capable of binding to the tetracycline-controlled promoter of the insertion element and of modulating its expression. Finally, the present invention relates to a cell containing the insertion element of the present invention, to a method for identifying genetic determinants of pathogen survival and to a method for the generation of a transgenic animal and plant.

Several documents are cited throughout the text of this specification. The disclosure content of the documents cited herein (including any manufacturer's specifications, instructions, etc.) is herewith incorporated by reference.

Despite tremendous advances in our comprehension of the molecular basis of infectious diseases or diseases such as cancer, substantial gaps remain both in our understanding of disease pathogenesis and in the development of effective strategies for early diagnosis and for treatment. Proteomic approaches to investigate diseases will overcome some of the limitations of other approaches. The opportunities as well as the challenges facing disease proteomics are formidable. Particularly promising areas of research include: delineation of altered protein expression, not only at the whole-cell or tissue levels, but also in subcellular structures, in protein complexes and in biological fluids; the development of novel biomarkers for diagnosis and early detection of disease; and the identification of new targets for therapeutics and the potential for accelerating drug development through more effective strategies to evaluate therapeutic effect and toxicity.

Despite earlier predictions to the contrary, infectious diseases remain a leading cause of worldwide death. A complicating factor in therapy for infectious diseases is the development of resistance to commonly used drugs (for example, as has occurred in tuberculosis), which heightens the need for developing effective new therapies. Interest in the application of proteomics to microbiology goes back at least two decades, with the pioneering work of Fred Neidhardt to characterize protein expression patterns in *Escherichia coli* under different growth conditions (VanBogelen, R. A., Schiller, E. E., Thomas, R. D. & Neidhardt, F. C. Diagnosis of cellular states of microbial organisms using proteomics. Electrophoresis 20, 2149-2159 (1999)).

At present, the characterization of genes involved in bacterial pathogenesis or of genes involved in cancer onset is primarily based on biochemical analysis of individual proteins or protein sets [see Hanash, 2003 and Jain, 2000 for reviews and references cited therein]. For proteome analysis, pathogenic microorganisms are often grown *in vitro* (in a chemostat, for example) under conditions known or assumed to mimic conditions in a host organism *[Streptococcus mutans -* (Len et al., 2003); *Salmonella typhimurium -* (Deiwick et al., 2002); *Pseudomonas aeruginosa -* (Guina et al., 2003); *Leishmania infantum -* (EI Fakhry et al., 2002); *Mycobacterium tuberculosis -* (Rosenkrands et al., 2002)]. The quest for identifying new tissue- or disease-specific proteins is hampered by the fact that methods like tissue micro-dissection, Protein Chip Array technologies, MALDI-TOF mass spectrometry or mass spectrometric analysis of *in situ* tissue sections are time-consuming, expensive and technically demanding (Hanash, 2003; von Eggeling et al., 2000).

In contrast to protein based analysis systems, analytic systems based on randomized genetic approaches are generally more efficient and versatile. It would therefore be desirable to have such a genetic system at hands, allowing to randomly modify the genes of a cell to control their gene expression by external control elements. This would help to understand the impact of these genes and their gene products on pathogenicity.

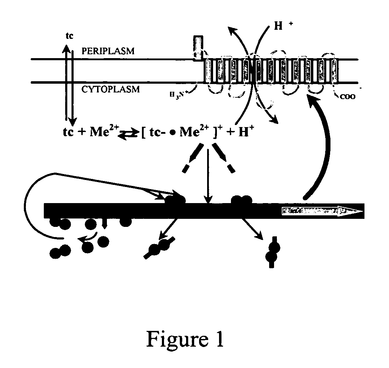

A number of control elements have previously been described Fussenegger, Biotechnol. Prog. 2001, 17, 1-51. The tetracycline-dependent gene regulation system is based upon a tetracycline resistance determinant discovered in Gram-negative and Gram-positive bacteria. Thereby, a repressor regulates transcription of the resistance gene *tetA* and its own gene by binding to two operators. The tetracycline repressor (TetR) reacts very efficiently to tetracycline (or its derivatives like anhydrotetracycline, atc), by binding two molecules of the drug and detaching from its operator sequence mediated by a conformational change. Thereby transcription of *tetR* and *tetA* is relieved (Figure 1). Meanwhile, TetR mutants are known which display the contrary phenotype (2). These, so called reverse Tet repressors allow turning off genes upon addition of tetracycline or its analogues, thereby opening new possibilities for application. The properties of this system are applied to gain stringent and highly sensitive transcriptional control over both pro-and eukaryotic genes of choice (3).

Numerous transposon based mutagenesis systems, which can be utilised in different organisms, have been described in the art. Most of these systems contain a vector transferring the donor-DNA together with a transposase gene into the target cell, where transposition of the donor-DNA into the chromosome takes place (4). These systems are disadvantageous in mostly being apt just for a specific target organism and in not ensuring genetic stability, as the transposase encoding gene is present in the cell. By contrast, in *in vitro* systems transposase is added to the donor- and target-DNA, and the modified target-DNA is brought back into the chromosome of the target cell (5). Goryshin *et al*. have developed an *in vitro* transposition system based upon the Tn5 transposon, where a stable complex of transposase and insertion element is electroporated into a target cell. This system can be applied in a broad range of different organisms (6). In 2000, Judson *et al.* published the first *in vitro* mutagenesis system vested with a regulable promoter to gain regulatory control over downstream genes (7). This system is based upon the arabinose-inducible promoter pBAD. The inducer arabinose, however, is not suitable for investigations in mammalian infection models, as it is metabolised there. In addition, not all bacteria posses an arabinose uptake system, therefore the choice of bacteria to be examined is limited. This is also the case in the transposon Tn*10* based *in vivo* mutagenesis system T-POP, which has been described elsewhere (8). The Tn*10* transposon has the disadvantage not to insert randomly but having insertion hot spots in the chromosome. This interferes with the successful creation of a totally randomized mutant pool. The T-POP system consists of a deletion variant of the whole Tn*10* transposon so all *tet* promoters (P_{tetR1}, P_{tetR2} and P_{tetA}) are active. Moreover the gene for tetracycline resistance *tetA* is present. So transcription from the *tetA* promoter has to exceed a long DNA fragment, before it reaches its destination i.e. the chromosomal DNA downstream of the insertion element. More important is the fact, that the *tetA* gene, if expressed unnecessarily, decreases the bacterial fitness by imbalancing the proton gradient of the cell wall. This is another crucial point for a versatile genetic tool not to influence the host cell's fitness by expressing unnecessary genes.

The complete sequencing of a number of microbial genomes has provided a framework for characterizing the role of various proteins and their relevance for pathogenic persistence or for disease progression. A crucial aspect of the continuing fight against pathogenic organisms is the question of whether or not it will be possible to identify those proteins within the pathogenic proteome, the expression of which is essential for the survival of the pathogen. To this end, it will be important to have a versatile system allowing to screen a large number of genetic components within a manageable time frame. In particular, it will be important to be able to selectively switch on (or off) genes, since this would allow to study their impact on disease onset or disease progression.

Thus, the technical problem underlying the present invention was to provide means and methods for generating conditional knock-out mutants of cells. The solution to this technical problem is achieved by providing the embodiments characterized in the claims.

Accordingly, the present invention relates to an insertion element, wherein said insertion element is a recombinant nucleic acid molecule which comprises, in 5' → 3' arrangement: (i) a first recombinase binding site; (ii) a resistance gene, operatively linked to a promoter and, optionally, to transcription control sequences; (iii) a tetracycline-controlled promoter; and (iv) a second recombinase binding site, wherein said tetracycline-controlled promoter is oriented to allow transcription in direction of the second recombinase binding site.

The term "insertion element" as used herein refers to a nucleic acid molecule which is incorporated into a larger nucleic acid molecule, particularly into the chromosome of a eukaryotic or bacterial cell.

As used herein, the term "nucleic acid molecule" refers to DNA and RNA which may be single stranded or double stranded, circular or linear. The term "recombinant" refers to the fact that the subunits of the insertion element have been combined by recombinant means.

The term "5' → 3' arrangement" refers to the polarity of DNA and RNA molecules as described for example in standard textbooks of genetics such e.g. Genes, B. Lewin (ed.), 1983, Wiley and Sons, Inc. New York.

The term "transcription control sequences" comprises elements such as e.g. promoter, enhancer, terminator, operator or silencer.

The insertion element of the present invention is flanked by a 5' and a 3' recognition sequence for a recombinase. As used herein, the term "recombinase" refers to transposases as well as recombinases. These recognition sequences are also called "first" and "second" recombinase binding site", wherein the 5' recognition sequence is the "first" and the 3' recognition sequence is the "second" recombinase binding site. Recombinase is the enzyme which catalyses a recombination reaction. In the context of the present invention, a recombinase activity is required to insert the insertion element of the present invention into the host cell genome which is usually made of DNA or RNA. A recombinase which is in accordance with the present invention must have the minimal functions of (1) inserting a break into a genomic nucleic acid molecule of the host cell and (2) of joining the resulting free ends with the free ends of the insertion element of the present invention. This activity is carried out, inter alia, by transposases or recombinases. However, any other protein, riboprotein or RNA-enzyme with this activity may be used in accordance with the teaching of the present invention.

The term "resistance gene" refers to a gene encoding a protein conferring resistance to a compound, wherein said compound is usually an antibiotic. Therefore, the term "resistance gene", as used herein, can also be understood as a gene encoding a protein conferring antibiotic resistance. "Antibiotic resistance" is the ability of a microorganism or of a cell to withstand the effects of an antibiotic.

The term "tetracycline-controlled promoter" refers to a promoter which is modulated by the presence and activity of one or more control elements which are located in the vicinity of said promoter. This control element, which is also called "tet operator" (*tetO*), represents the binding site for a tetracycline-sensitive (poly)peptide. Preferably a single *tetO* is selected from any one of SEQ ID NOs: 1 to 6. the following sequence motifs.

The term "promoter" as used throughout the specification means binding site of the RNA polymerase enabling transcription.

The term "(poly)peptide" refers alternatively to peptide or to polypeptide. Peptides conventionally are covalently linked amino acids of up to 30 residues, whereas polypeptides (also referred to as "proteins") comprise 31 and more amino acid residues.

In one embodiment of the present invention, the tetracycline-sensitive (poly)peptide is the tetracycline repressor (TetR) which is well known in the art (Berens 2003). It is envisaged by the teaching of the present invention that any tetracycline sensitive (poly)peptide may be used, provided that it is capable of (1) binding to a nucleic acid based binding site and (2) modulating the level of transcription of the nearby promoter, wherein modulation of transcription refers to an upregulation or downregulation of transcription, including shut-off of transcription. Useful tetracycline sensitive (poly)peptides and binding sites for such peptides are disclosed herein.

The insertion element of the present invention contains in its simplest embodiment one, two or three *tetO* sequences. However, in order to improve control by tetracycline sensitive (poly)peptides it is possible to add further *tetO* sequences. The *tetO* sequences need not be the same, as long as they provide a functional binding site for tetracycline sensitive (poly)peptides.

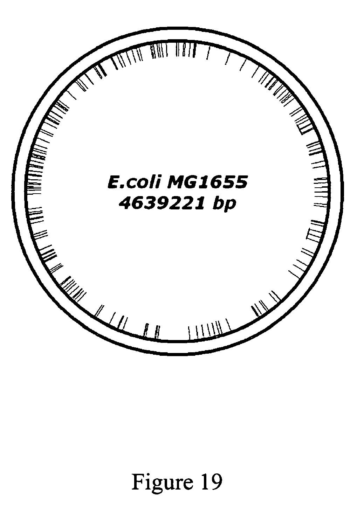

The liquid media screening systems described in the examples of the present invention yields a plethora of phenotypes of different E. *coli* transponants. This is due to the differences in growth of the candidates with or without atc, which can hardly be differentiated on solid media plates. Besides auxotrophs, candidates are found that display a reverse regulable phenotype, i.e. growth only in the absence of atc. During all screenings, 202 different genes were detected where the element had inserted (Fig. 19). These include mutants that could not be verified on solid medium.

The utilisation of two regulators with different phenotypes, wtTetR and revTetR, resulted in a different spectrum of mutants: While in the wtTetR screening reverse regulable mutants were found, the revTetR screen yielded essential genes. This demonstrates that different kinds of regulators can be applied to reach the goal of the screening. The analysis of the 15 regulable candidates described above revealed that some of them are already published, such as *mraZ,* the first gene in the *dcw* cluster, responsible for cell division and cell wall synthesis. The transcription of this cluster has previously been put under the control of a regulable promoter and analysed (13). By contrast, the yrbK gene was identified, whose function is unknown so far. This mutant could now be subjected to further analysis to elucidate this gene's function and to possibly obtain a novel drug target. Table 1 (see below) summarises all found regulable auxotroph mutants as well as the assigned function, product or biosynthesis pathway of the respective gene. The numbers presented in the Examples of the present invention clarify that the present invention's technique can be applied to generate numerous mutants with different phenotypes. Recently, element one has been improved by adding additional features. In these additional experiments, the kanamycine resistance cassette is now flanked by two FRT sites to optionally excise the cassette by using Flp recombinase. This opens the possibility to insert an additional element into the chromosome of a distinct mutant utilizing the "recycled" selection marker. A R6Kγ-ori was also added to the element, which can initiate replication only when the pi-protein, which was deleted from this ori, is present elsewhere in a suitable strain. This ori simplifies the recovery of the insertion site by the so called "rescue cloning" technique. The last feature added to the element was a T7 promoter. This is widely used for in vitro transcription to obtain transcripts from the chromosome for further analysis e.g. in microarrays. This modified element improves versatility as tool for the search and examination of new drug targets.

The term "oriented to allow transcription in direction of the second recombinase binding site" means the tetracycline dependent promoter enables transcription directly out of the insertion element.

In a preferred embodiment of the present invention, at least one stop codon is located between the first recombinase binding site and the resistance gene. This stop codon has the function to suppress translation of open reading frames (ORF) located 5' of the resistance gene. In the case of bacterial cells, stop codons may be selected from UAA, UAG, UGA, in the case of eukaryotic cells, stop codons may be selected from UAA, UAG, UGA. In order to block translation of all open reading frames, it is preferred to have three neighboring stop codons which are positioned within all possible reading frames. However, sometimes it may be sufficient that the insertion element only comprises two or even one stop codon. Stop codons may overlap or be separated by additional nucleotides.

In another preferred embodiment of the present invention, at least one terminator of transcription is located 5' of the resistance gene. In most cases, it is sufficient to use one or two terminators of transcription. However, it is conceivable to position two, three, four or even five terminators next to each other. The terminator sequences do not need to be the same and any known terminator sequences may be used at the indicated position within the insertion element. In fact, it is also possible to use artificial terminator sequences. Generally any sequence is suitable in this regard, as along as it results in a reduction of the level of transcription. The skilled person can determine transcription termination activity of a given nucleotide sequence by conventional assays such as Northern Blot analysis. Typical examples of terminator sequences which are in accordance with the present invention are the Transposon Tn10 transcriptional terminator sequence = SeqlD No. 372 (aaatataatgaccctcttgataacccaagagggcatttttta) (NAR, Vol.13, No.12 (1985), Schollmeier K. et.al.); Bacteriophage Lambda T0 transcriptional terminator = SeqID No. 373.
(gactcctgttgatagatccagtaatgacctcagaactccatctggatttgttcagaacgctcggttgccgccgggcgttt tttattggtgagaatccaagcta) (Ref: NCBI Nucleotide entry: AY737006); Escherichia coli rrnB T1 transcriptional terminator =SeqID No.374 (aggcatcaaataaaacgaaaggctcagtcgaaagactgggcctttcgttttatctgttgtttgtcg) (JMB, 269 (1997), Lyakhof D.L. et al. ); Escherichia coli rrnB T2 transcriptional terminator =SeqID No.375 (ttaagcagaaggccatcctgacggatggcctttttgcgtttctac) (JMB, 269 (1997), Lyakhof D.L. et al. ); Bacteriophage T7 transcription terminator =SeqID No. 376 (ctagcataaccccttggggcctctaaacgggtcttgaggggttttttgctgaaag) (JMB, 269 (1997), Lyakhof D.L. et al. ).

In another preferred embodiment of the present invention, the resistance gene is selected from the group consisting of cat, aph, bla, ermB, rpsL, aada5, hph, rpoB. The following table shows the proteins encoded by the aforementioned genes and the corresponding antibiotic resistance.

| antibiotic resistance | gene | product |
|---|---|---|
| Chloramphenicol | *cat* | chloramphenicol acetyl transferase |
| Kanamycin | *aph* | aminoglycosyl transferases |
| Ampicillin, Penicillin G | *bla* | beta-lactamases |
| Erythromycin | *ermB* | erythromycinmethylase |
| Streptomycin | *rpsL, aada5* | modified ribosomal protein S12, aminoglycoside-3'-adenylyltransferase |
| Hygromycin B | hph | hygromycin B phosphotransferase |
| Rifampicin | rpoB | modified RNA polymerase subunit beta |

In another preferred embodiment of the present invention, the resistance gene is flanked by additional recombinase binding sites which are different from the first and second recombinase binding site. The presence of such additional recombinase binding sites allows to selectively remove the resistance gene at later stages of the present invention's method. For example, after selecting cells with the desired properties, recombinase activity specific for the third and fourth recombinase binding site may be used to excise the resistance gene. Particularly in cases when undesired reactions of the host environment against the presence of the resistance gene are to be expected, removal of the resistance gene can prevent such reactions.

In a more preferred embodiment of the present invention, said additional recombinase binding sites are selected from FLP recombinase dependent FRT sites and Cre recombinase dependent loxP sites.

In yet another preferred embodiment, the insertion element of the present invention comprises a prokaryotic origin of replication. Generally, any ori of replication may be used in accordance with the teaching of the present invention. R6Kγ is a preferred ori.

In another preferred embodiment of the present invention, at least one viral promoter is located upstream of the tetracycline controlled promoter. Such a promoter may be obtainable from a virus of a eukaryotic or prokaryotic cell. Preferably, the viral promoter is a promoter obtainable from a eukaryotic virus or from a prokaryotic virus . Preferred promoters are promoters obtainable from the bacteriophages T7, T3 or SP6. Examples of such promoters are the T7-promoter (SeqID No.377: CCTCCCTATAGTGAGTCGTATTA); the T3-promoter (SeqID No.378: GGAATTAACCCTCACTAAAGGG) and the SP6-promoter: (SeqID No.379: ATTTAGGTGACACTATAGAA).

In another preferred embodiment, the insertion element of the present invention may comprise an additional coding sequence for a tetracycline repressor, wherein the coding sequence is (a) operatively linked to a promoter and, optionally, to transcription control sequences; or (b) operatively linked to an internal ribosomal entry site and arranged 3' to a promoter sequence of the insertion element, allowing expression of the repressor from a multicistronic RNA. As pointed out above, the present invention's teaching relates to insertion elements comprising a tetracycline-controlled promoter. It is envisaged by the present invention that the tetracycline sensitive (poly)peptide, controlling the promoter by binding to one or more *tetO* sites, may be encoded by an open reading frame located either on the same nucleic acid molecule as the *tetO* (located in *cis)* or located on a different nucleic acid molecule (located in *trans).* A suitable promoter may be any of the promoters described herein above. A suitable transcription control sequence may be any of the transcription control sequences described herein above. The term "ribosomal entry site" (IRES) refers to a nucleic acid sequence on them RNA which mediates internal entry and start of ribosomal translation. Such sites are particularly useful for enabling translation of (poly)peptides encoded by open reading frames located 3' of the 5' proximal open reading frame, i.e. for expressing the open reading frames of a multicistronic RNA. A multicistronic RNA is an RNA which contains more than one open reading frame. IRES elements may, however, also be used for expressing the open reading frame of a monocistronic RNA. Preferably, IRES elements are selected from the group consisting of poliovirus-IRES and EMCV-IRES (see SEQ ID NO.s 392 and 393).

In another preferred embodiment of the present invention, the promoter is a prokaryotic promoter. Preferably, said prokaryotic promoter is selected from the group consisting of T7, T3, xylA, tetA or SP6. Examples of such promoters are the T7-promoter (SeqID No.377: CCTCCCTATAGTGAGTCGTATTA); the T3-promoter (SeqID No.378: GGAATTAACCCTCACTAAAGGG) and the SP6-promoter: (SeqID No.379: ATTTAGGTGACACTATAGAA).

In a more preferred embodiment of the present invention, the promoter is controlled by at least one tet-operator binding site sequence selected from (a) SEQ ID NO: 1 to 6; or from (b) SEQ ID NO: 7 to 13; or permutations thereof. The sequences of the tet-operators are as follows (see also SEQID NOs: 1 to 6):

tetO1 ACTCTATCATTGATAGAGT
TGAGATAGTAACTATCTCA

tetO2 TCCCTATCAGTGATAGAGA
AGGGATAGTCACTATCTCT

tetO1-4C ACTCTGTCATTGACAGAGT
TGAGACAGTAACTGTCTCA

tetO1-6C ACTGTATCATTGATACAGT
TGACATAGTAACTATGTCA

tetO2-4C TCCCTGTCAGTGACAGAGA

AGGGACAGTCACTGTCTCT
tetO2-6C TCCGTATCAGTGATACAGA
AGGCATAGTCACTATGTCT

The other sequences SEQ ID NOs: 7 to 13 represent complete promoters containing tet-operators.

In another preferred embodiment of the present invention, the promoter is a eukaryotic promoter.

In a more preferred embodiment of the present invention, the eukaryotic promoter is selected from the group consisting of CMV, RSV, SV40, Cadherin and hAPC and β-Act, AFP, CD43, CD45, CD68, c-erbB2, EF1, E2F-1, EGR1, FerL, GAPDH, HSP70, IFNB, Mb, PGK-1, TRP1.

In a more preferred embodiment of the present invention, the eukaryotic promoter is selected from any one of SEQ ID NOs: 14 to 21.

In another more preferred embodiment of the present invention, the recombinase binding site is a binding site obtainable from the transposases Tn5, Tn10, Tn7, or from phage Mu.

Tn5 binding sites: =SeqID No. 380/381 [CTGTCTCTTATACACATCT (binding site 1), agatgtgtataagagacag (binding site 2)]; Tn10 binding sites: =SeqID No.382/383 [CTGATGAATCCCCTAATGATTTT (BINDING SITE 1), AAAATCATTAGGGGATTCATCAG (BINDING SITE 2)]; Tn7 binding sites: =SeqID No.384/385 [TGTGGGCGGACAATAAAGTCTTAAACTGAACAAAATAGATCTAAACTATGACAA TAAAGTCTTAAACTAGACAGAATAGTTGTAAACTGAAATCAGTCCAGTTATGCT GTGAAAAAGCATACTGGACTTTTGTTATGGCTAAAGCAAACTCTTCATTTTCTGA AGTGCAAATTGCCCGT CGTATTAAAGAGGGGCGTGG (BINDING SITE 1), AACCAGATAAGTGAAATCTAGTTCCAAACTATTTTGTCATTTTTAATTTTCGTATT AGCTTACGACGCTACACCCAGTTCCCATCTATTTTGTCACTCTTCCCTAAATAAT CCTTAAAAACTCCATTTCCACCCCTCCCAGTTCCCAACTATTTTGTCCGCCCAC A (BINDING SITE 2)]; Phage Mu binding sites: SeqID No.386/387 [TGAAGCGGCGCACGAAAAACG (BINDING SITE 1), CGTTTTTCGTGCGCCGCTTCA (BINDING SITE 2)].

In yet another more preferred embodiment of the present invention, the recombinase binding site is selected from the group consisting of tc1/mariner and minos. Himar1 binding sites: =SeqID No.388/389 comprise the nucleotide sequences TAACAGGTTGGCTGATAAGTCCCCGGTCT (binding site 1) and AGACCGGGGACTTATCAGCCAACCTGTTA (binding site 2), NCBI Nucleotide entry: AY680862. Acmar1 binding sites =SeqID No.390/391 comprise the nucleotide sequences ATACGTATTATATATTTT (binding site 1) and AAAATATATATATACTAT (binding site 2), NCBI Nucleotide entry: AB081476. These binding sites are more or less perfect inverted terminal repeats of the transposable elements.

The present invention also relates to a vector, comprising an insertion element of the present invention. The term "vector", as used here, refers to prokaryotic and eukaryotic vectors. Such vectors may be specific for prokaryotic or eukaryotic expression. However, the insertion element of the present invention may also be part of a vector functional in prokaryotes and eukaryotes.

A typical prokaryotic expression vector usually contains a promoter element, which mediates the initiation of transcription of mRNA, the protein coding sequence, and signals required for the termination of transcription of the transcript. Transcription of DNA is dependent upon the presence of a promoter which is a DNA sequence that directs the binding of RNA polymerase and thereby promotes mRNA synthesis. The DNA sequences of eukaryotic promoters differ from those of prokaryotic promoters. Furthermore, eukaryotic promoters and accompanying genetic signals may not be recognized in or may not function in a prokaryotic system, and, further prokaryotic promoters are not recognized and do not function in eukaryotic cells. Similarly, translation of mRNA in prokaryotes depends upon the presence of the proper prokaryotic signals which differ from those of eukaryotes. Promoters vary in their "strength" (i.e. their ability to promote transcription). In some cases it may be desirable to use strong promoters in order to obtain a high level of transcription and, hence, expression of a protein. Depending upon the host cell system utilized, any one of a number of suitable promoters may be used. For instance, when using *E*. *coli,* its bacteriophages, or plasmids, promoters such as the T7 phage promoter, *lac* promoter, *trp* promoter, *recA* promoter, ribosomal RNA promoter, the *P*_{R}. and *P*_{L} promoters of coliphage lambda and others, including but not limited, to *lac*UV5, *ompF, bla, Ipp*, and the like, may be used to direct high levels of transcription of adjacent DNA segments. Additionally, a hybrid *trp-lac*UV5 *(tac)* promoter or other *E. coli* promoters produced by recombinant DNA or other synthetic DNA techniques may be used to provide for transcription of the inserted gene. Specific initiation signals are also required for efficient gene transcription and translation in prokaryotic cells. These transcription and translation initiation signals may vary in "strength" as measured by the quantity of gene specific messenger RNA and protein synthesized, respectively. The DNA expression vector or nucleic acid molecule encoding a (poly)peptide, which contains a promotor, may also contain any combination of various "strong" transcription and/or translation initiation signals. For instance, efficient translation in *E. coli* requires an SD sequence about 7-9 bases 5' to the initiation codon ("ATG") to provide a ribosome binding site. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, Methods in Enzymology, 68:473 (1979), which is hereby incorporated by reference. Thus, an SD-ATG combination that can be utilized by host cell ribosomes may be employed. Such combinations include but are not limited to the SD-ATG combination from the cro gene or the N gene of coliphage lambda, or from the *E. coli* tryptophan E, D, C, B or A genes. Additionally, any SD-ATG combination produced by recombinant DNA or other techniques involving incorporation of synthetic nucleotides may be used. Suitable expression vectors for use in practicing the present invention include, for example, vectors such as pET expression vectors (Novagen), pQE expression vectors (Qiagen), pASK expression vectors (IBA), pBAD expression vectors (Invitrogen), pWH1950 (Ettner et al., 1996), pWH1520 (MoBiTec), pWH353 with a Xyl/Tet promoter (Geissendörfer and Hillen, 1990), pLZ vectors with SPAC, Xyl and Xyl/Tet promoter systems (Zhang et al., 2000) or pNZ8008 with a *nisA* promoter (Eichenbaum et al., 1998).

A typical eukaryotic expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin, EF-1α and ubiquitin C promoters). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Host cells that could be used include human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the encoded (poly)peptides can be expressed in stable cell lines that contain a nucleic acid molecule of interest integrated into a chromosome. The co-transfection with a selectable marker such as dhfr, gpt, neomycin, hygromycin allows the identification and isolation of the transfected cells. The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy et al., *Biochem J. 227*:277-279 (1991); Bebbington et al., *Bio*/*Technology* 10:169-175 (1992)). Using these markers, the cells are grown in selective medium and the cells with the highest resistance are selected. These cell lines contain the amplified gene(s) integrated into a chromosome. Chinese hamster ovary (CHO) and NSO cells are often used for the production of proteins. The expression vectors pC1 and pC4 contain the strong promoter (LTR) of the Rous Sarcoma Virus (Cullen et *al., Molecular and Cellular Biology,* 438-447 (March, 1985)) plus a fragment of the CMV-enhancer (*Boshart et al., Cell 41*:521-530 (1985)). Multiple cloning sites, e.g., with the restriction enzyme cleavage sites *Bam*Hl, *Xbal* and *Asp*718, facilitate the cloning of the gene of interest. The vectors contain in addition the 3' intron, the polyadenylation and termination signal of the rat preproinsulin gene. As indicated above, the vectors of the present invention will preferably include at least one selectable marker. Such markers include dihydrofolate reductase, Herpes simplex virus thymidine kinase, G418 or puromycin or zeocin resistance for eukaryotic cell culture and tetracycline, hygromycin, apramycin, zeocin, kanamycin or ampicillin resistance genes for culturing in E. *coli* and other bacteria. Appropriate culture mediums and conditions for the above-described host cells are known in the art.

The present invention also relates to a method for the generation of a conditional knock-out mutant of a cell, comprising the steps of: (a) introducing a nucleic acid molecule comprising the insertion element of the present invention into a cell; wherein (i) the insertion element is transfected into the cell concomitant with purified recombinase ; (b) integrating the insertion element into the cellular genome; and (c) selecting cells for resistance against a substrate for which the resistance gene of the insertion element confers resistance, wherein prior to or concomitant with step (a) an expression construct for a tetracycline-sensitive (poly)peptide is introduced into the cell, said (poly)peptide being capable of binding to the tetracycline-controlled promoter of the insertion element and of modulating its expression.

The term "conditional knock-out mutant" refers to cells which can only express the affected gene under controlled conditions.

The term "introducing a nucleic acid molecule" means bringing DNA or RNA molecules into the cell. This can be achieved by various methods which comprise transfection, transformation, infection, transduction or electrotransformation.

The term "concomitant with purified recombinase", as used herein, means that at the same time the nucleic acid is introduced into the cell, recombinase proteins are introduced. To this end, the nucleic acid and recombinase molecules may be mixed and pre-incubated prior to the transfection step.

The term "selecting cells for resistance against a substrate" means identifying cells with a particular phenotype, i.e. those cells which can grow in the presence of a substrate which is a cytotoxic substrate or a cytostatic substrate for the cell or which reduces growth and/ or cell division.

The term "binding" ... "and" "modulating its expression" means, that in dependence of the presence of inducer the expression of downstream genes can be modulated by binding or by a lack of binding of TetR to its operator sequence

This method may also be used within a method for generating a transgenic animal. In this case, the cell used for the generation of the conditional knock-out mutant of a cell is an embryonic stem cell, an adult stem cell or a cell of an established cell line and from such a cell a viable organism is grown. A typical method for the generation of a transgenic mammal may comprise the steps of (a) injecting e.g. a selected embryonic stem cell into blastocysts, (b) transfer of blastocysts into the uterus of a mammal and (c) delivering the transgenic mammal.

In a preferred embodiment of the present invention, the tetracycline-sensitive (poly)peptide is a repressor of transcription. The term "tetracycline-sensitive repressor of transcription", as used herein, is a (poly)peptide which, in the absence of tetracycline, is capable of binding to a Tet operator and as a consequence thereof represses transcription from a nearby promoter sequence. In contrast, whenever sufficient amounts of tetracycline are present, the repressor binds to tetracycline and its affinity to the tet operator sequence is reduced so that transcription from the nearby promoter can proceed.

In a more preferred embodiment of the present invention, the tetracycline-sensitive (poly)peptide comprises the amino acid sequence of any one of (a) SEQ ID NOs: 22 to 41; (b) SEQ ID NOs: 42 to 63; (c) SEQ ID NOs: 64 to 256; (d) SEQ ID NOs: 257 to 266; (e) SEQ ID NOs: 267 to 311or (f) SEQ ID NOs: 312 to 316 or a tetracycline-sensitive mutant of this sequence, wherein said mutant has a sequence identity of at least 92%, is capable of binding to the operator sequence and is inducible by a tetracycline.

In a further preferred embodiment of the present invention, the tetracycline-sensitive (poly)peptide is an activator of transcription. The term "tetracycline-sensitive activator of transcription", as used herein, is a (poly)peptide which, in the absence of tetracycline, is capable of binding to a Tet operator and as a consequence thereof activates transcription from a nearby promoter sequence. Whenever sufficient amounts of tetracycline are present, however, the activator binds to tetracycline and its affinity to the tet operator sequence is reduced so that transcription from the nearby promoter is no longer activated and vice versa in the case of the reverse TetR alleles (activating transcription in the presence of tetracycline.

In a more preferred embodiment of the present invention, the tetracycline-sensitive (poly)peptide comprises the amino acid sequence of any one of SEQ ID NOs: 317 to 357.

In a preferred embodiment of the present invention, the cells are grown in the presence of tetracycline or of a functional homolog thereof, wherein said homolog is selected from the group consisting of Tetracycline, Doxycycline, Minocycline, Sancycline, Anhydrotetracycline, Chlorotetracycline, 4-DDMA-Anhydrotetracycline, Oxytetracycline, 4-DDMA-tetracycline, 2-Nitrilo-tetracycline, 4-Dedimethyl-aminosancycline, 4-DDMA-chlor-tetracycline, 4-DDMA-11,12-pyrazolo-tetracycline, 4-DDMA-4-hydroxy-tetracycline, 4-DDMA-12a-dehydroxy-tetracycline, 4-DDMA-doxycycline.

Moreover, any derivative may be used provided that it can induce the tetracycline sensitive (poly)peptide described in the embodiments of the present invention. The functional homologue may also be a (poly)peptide capable of binding to the tet repressor

In another preferred embodiment of the present invention, the cells are grown in the presence of a peptide-based functional homolog of tetracycline or in the presence of a (poly)peptide comprising said peptide, wherein said peptide-based homolog or said (poly)peptide comprises any one of SEQ ID NOs: 358 to 371. The "peptide-based functional homologue" may be a short peptide or be part of a polypeptide comprising the amino acid sequence of such a peptide.

The present invention also relates to a cell containing the insertion element or the vector of the present invention or which is obtainable by the method of the present invention.

In a preferred embodiment of the present invention, said cell is a eukaryotic or prokaryotic cell.

In a more preferred embodiment of the present invention, said cell is selected from the group consisting of vertebrate and invertebrate cell. Preferably, said cell is a cell obtainable from *Fugu, C.elegans, Leishmania, Plasmodium, Toxoplasma,* an insect cell obtainable from *Drosophila, Anopheles,* a fungal cell obtainable from *Candida, Aspergillus, Dictyostelium* or a bacterial cell.

In an even more preferred embodiment of the present invention, the vertebrate cell is a cell of xenopus, zebrafish or a mammalian cell.

In a most preferred embodiment of the present invention, the mammalian cell is an embryonic stem cell, an adult stem cell or a somatic cell.

In another more preferred embodiment of the present invention, the eukaryotic cell is a plant cell. Preferably, said plant cell is from Arabidopsis, Zea mais, Oryza.

In another more preferred embodiment of the present invention, the bacterial cell is selected from the group consisting of *Acinetobacter, Actinobacillus, Actinomyces, Aeromonas, Bacillus, Bacteroides, Bdellovibrio, Bordetella, Borrelia, Branhamella, Brucella, Burkholderia, Campylobacter, Capnocytophaga, Clamydia, Citrobacter, Clostridiae, Corynebacterium, Coxiella, Eikenella, Erysipelothrix, Escherichia, Francisella, Fusobacterium, Haemophilus, Helicobacter, Klebsiella, Legionella, Leptospira, Listeria, Meningococcus, Moraxella, Mycobacteria, Mycoplasma, Neisseriaceae, Nocardia, Pasteurella, Peptostreptococcus, Pneumococcus, Proteus, Providencia, Pseudomonas, Rickettsia, Salmonella, Selenomonas, Serratia, Shigella, Spirillum, Staphylococcus, Streptococcus, Treponema, Veillonella, Vibrio, Wolinella, Yersinia.*

The present invention also relates to a method for identifying genetic determinants of pathogen survival, comprising the steps of: (a) infecting an organism with a pathogen, wherein the pathogen is the cell of the present invention or a multi-cellular organism comprising said cell; (b) growing the cell in the presence and absence of tetracycline or in the presence or absence of a functional homolog thereof, wherein said homolog is (i) a peptide-based functional homolog of tetracycline or a (poly)peptide comprising said peptide, wherein said peptide-based homolog or said (poly)peptide comprises any one of SEQ ID NOs: 358 to 371; or (ii) a homolog which is selected from the group consisting of Tetracycline, Doxycycline, Minocycline, Sancycline, Anhydrotetracycline, Chlorotetracycline, 4-DDMA-Anhydrotetracycline, Oxytetracycline, 4-DDMA-tetracycline, 2-Nitrilo-tetracycline, 4-Dedimethyl-aminosancycline, 4-DDMA-chlor-tetracycline, 4-DDMA-11,12-pyrazolo-tetracycline, 4-DDMA-4-hydroxy-tetracycline, 4-DDMA-12a-dehydroxy-tetracycline, 4-DDMA-doxycycline; (c) selecting cells, the growth of which is affected by the presence of tetracycline or the functional homolog thereof; and (d) determining which gene or gene product of the cell of (c) is effected by the insertion of the insertion element, wherein the gene or gene product identified in step (d) is a genetic determinant for the survival of the pathogen in the infected organism.

The term "identifying genetic determinants of pathogen survival" means identifying those nucleic acid sequences within the genome of the pathogenic cell which have an impact on its survival within the host. In most cases, the genetic determinant will be a gene or the gene product such as a regulatory RNA or a (poly)peptide.

The term "pathogen" refers to organisms or particles that can lead to a more or less severe disease of an organism.

The term "selecting cells ..." means identifying and isolating individual cells, the growth of which is affected by the presence of tetracycline or its homolog.

The term "determining which gene or gene product ..." means establishing by sequencing the insertion region in the chromosome.

The presence of the tetracycline-controlled promoter sequence located within the insertion element of the present invention provides an external means of control of expression of said genetic determinant. For example, if the insertion element of the present invention is integrated between the native promoter sequence of said gene and its coding sequence, transcription from the native promoter will generally be impaired and, be halted if a transcription terminator sequence is located within the insertion element 5' to the tetracycline-controlled promoter. On the other hand, transcription of said coding sequence can be induced e.g. by adding tetracycline to the host environment.

In a preferred embodiment of the present invention, the infected organism is a eukaryotic or prokaryotic cell, for example a pathogenic bacterial cell infecting a eukaryotic cell.

In another preferred embodiment of the present invention, the infected organism is a vertebrate or invertebrate animal. Preferably, vertebrates are selected from the group consisting of Xenopus, zebrafish, human cells, mouse. Preferably, the invertebrate is C. elegans.

In a more preferred embodiment of the present invention, the vertebrate is selected from mammalian, xenopus and zebrafish. Preferred mammalians are human, sus scrofa, mouse, equus.

The present invention also relates to a method for the generation of transgenic non-human animal, comprising the step of propagating the cell of the present invention and developing from said cell a viable organism, wherein said cell is non-human.

As described above, the cell of the present invention may be used to develop a viable organism. Such a cell may be for example an embryonic stem cell or an adult stem cell. A typical method for the generation of a transgenic mammal may comprise, for example, the steps of (a) injecting e.g. a selected embryonic stem cell of the present invention into blastocysts, (b) transfer of blastocysts into the uterus of a mammal and (c) delivering the transgenic mammal.

In a preferred embodiment of the present invention, said animal is a mammalian animal.

The present invention also relates to a transgenic non-human animal produced by the method of the present invention. Such an animal may be a vertebrate or an invertebrate. It may be for example a mammalian, a xenopus or zebrafish. Preferred mammalians are selected from the group consisting of sus scrofa, human, mouse, equus. Such animals may be useful in varies areas: The transgenic non-human animal of the present invention may be used, for example, to investigate how the animal reacts (tetracycline-dependently) on different pathogen invasion. Another example is to create conditionally lethal embryonic mutations to investigate important functions of development. The present invention also relates to a method for the production of a vaccine composition, comprising the steps of (a) growing the bacterial cell of the present invention in the presence and absence of tetracycline and selecting cells which display an attenuated phenotype in the absence of tetracycline; and optionally (b) formulating the bacteria with a pharmaceutically acceptable carrier. The steps of this method may be combined with other steps or modified. For example, it is conceivable that not the initially identified cell is used in the vaccine composition but rather a mutant thereof. To generate such a mutant, the specific genetic determinant(s) of pathogenicity might be identified. If the genetic determinant is a gene, for example, various specific mutants of this gene may be generated. Such a mutant may be e.g. a more efficient knock-out mutant. Alternatively, it is conceivable to generate mutants with various degrees of gene expression of a pathogenic gene. In this way the degree of attenuation might be modulated.

By "vaccine" is meant an agent used to stimulate the immune system of an individual so that protection is provided against one or more antigen(s) of the bacterial cell, not recognized as a self-antigen by the immune system. The term "immune system" refers to the organ responsible for the process of inducing a continuing high level of antibody and/or cellular immune response in which T-lymphocytes can either kill the invading microbe and/or activate other cells (e.g., phagocytes) to do so in an individual, which is directed against a microbe to which the organism has been previously exposed. The phrase "immune system" is intended to refer to the anatomical features and mechanisms by which an individual produces antibodies against an antigenic material which invades the cells of the individual or the extra-cellular fluid of the individual and is also intended to include cellular immune responses. In the case of antibody production, the antibody so produced can belong to any of the immunological classes, such as immunoglobulins, A, D, E, G or M. Vaccines of the nature described herein are likely to produce a broad range of immune responses, for example cellular and humoral immunity. Immune responses to microbes and antigens are well studied and widely reported. A survey of immunology is provided in Elgert, Klaus D., Immunology, Wiley Liss, Inc., (1996); Stites et al., Basic & Clinical Immunology; 7th Ed., Appleton & Lange, (1991) the entirety of which are incorporated herein by reference.

An individual treated with a vaccine of the present invention is intended to mean one of the organisms described in the embodiments of the present invention. The vaccine can be prepared by growing the vaccine strain in suitable growth media and then used as is or formed into a vaccine composition by combining the growing culture, or the cells therefrom, with a suitable diluent. Suitable diluents are preferably liquids and are more preferably a liquid that does not adversely effect the stability and vitality of the vaccine culture. The diluent is preferably free of chlorine, antibiotics, antimicrobials, or any other agent that may be harmful to the live vaccine organisms. Vaccine should be dispersible in the diluent so that no solid lumps or chunks of vaccine remain and the diluent should be at a temperature that is not harmful to the live vaccine microbes. Examples of suitable diluents include water, distilled water, de-ionized water, skim milk, water containing Marek's vaccine stabilizer, buffered saline with gelatin, and similar compositions that are well-known to persons of skill in the art. The vaccine is preferably introduced into the diluent while the diluent is at a temperature of approximately room temperature or cooler more preferably from about 34° C. to about 15° C.

The present invention also relates to a vaccine composition prepared by the method of the present invention.

The present invention also relates to the use of the bacterial cell of the present invention for the preparation of a vaccine composition for ameliorating or preventing diseases caused by the bacterial cell. The methods described herein are used to generate attenuation mutants of bacterial cells. Typically, such mutants will contain an insertion element of the present invention, located in the vicinity of a genetic determinant of pathogenicity. Such a mutant may be e.g. a mutant of Actinobacillus pleuropneumoniae (APP) or a derivative thereof which may be used for the preparation of a vaccine composition for ameliorating or preventing a disease caused by the naturally occurring Actinobacillus pleuropneumoniae (APP). In fact, any of the bacteria described herein may be used for the preparation of a vaccine composition comprising an attenuation mutant as described above, for ameliorating or preventing a disease caused by the naturally occurring bacteria.

The present invention also relates to a method for generating a transgenic plant, comprising the steps of: (a) cultivating the cell of the present invention or a protoplast derived therefrom in suitable media; and (b) regeneration of plants.

Moreover, the present invention also relates to a plant or a product of the plant produced by the method of the present invention.

Finally, the present invention relates to a method of cultivating a plant, comprising the step of growing the plant of the present invention.

The figures show:
- Figure 1:: Diagram of the tetracycline determinants in Gram-negative bacteria.
The upper part displays activities at the cytoplasmic membrane, while the lower part depicts the genetic organisation and induction of the *tet* genes. Tetracycline (tc) diffuses in its neutral form across the cytoplasmic membrane into the cell (left upper part). There it is complexed by divalent cations (Me²⁺), and can then act as the substrate for the proton-tc-antiporter TetA, depicted as a 12-α-helical membrane bound protein (right upper part). The same tc-complex is the molecular inducer (black boxes) of Tet repressor (TetR, blue filled circles), encoded by *tetR* (blue arrow). In the absence of tc, TetR binds as a dimer to the tandem operators O₁ und O₂, located between the two genes *tetR* and *tetA*. These divergently oriented genes are transcribed by overlapping promoters (not shown). The [tc-Me]⁺⁻complex binds to TetR and thereby causes a conformational change in the protein so that it can no longer bind to *tet*-operator but quickly dissociates from the DNA (1).
- Figure 2:: Schematic depiction of the insertion element one. Between the specific binding sites for transposase (ME), stop codons (red upper case "S"es), a transcriptional terminator (stem loop), the kanamycine resistance gene (Km^{R}) and the tetracycline dependent promoter (right directed grey arrow) containing one tet operator (grey box) are located. The tetracycline dependent promoter drives transcription outward of the element.

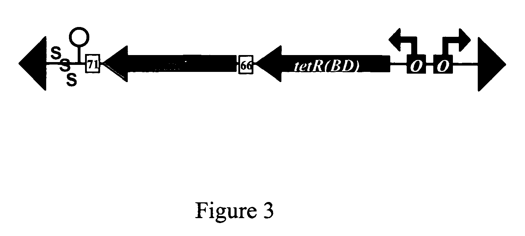

- Figure 3:: Schematic depiction of the insertion element two. Between the specific binding sites for transposase (ME), stop codons (red upper case "S"es), a transcriptional terminator (stem loop), the kanamycine resistance gene (Km^{R}), flanked by two asymmetric loxP sites (yellow boxes) are located. As a major difference to element one, the tetracycline repressor gene is located on the element, transcribed by an autoregulated promoter (left directed grey arrow). The tetracycline dependent promoter driving transcription (right directed grey arrow) contains one tet operator in this example (or two, see text).
- Figure. 4 :: β-galactosidase activity measurement to assay the regulatory function of the insertion element. The ordinate shows the relative β-galactosidase activity w/o TetR displays the 100%-value. TetR(BD)wt, revTetR_1 and revTetR_2 are three measured TetR variants with (yellow bars) or without anhydrotetracycline (blue bars) as effector.
- Figure 5:: β-galactosidase activity measurement to assay the regulatory function of insertion element two. The ordinate shows the absolute β-galactosidase activity. depicted are measurements of two differet elements, containing one or two tet operators within the outward promoter (as indicated). Measurements were conducted with (yellow bars) or without anhydrotetracycline (blue bars) as effector.
- Figure 6 :: Plot of molar excess of transposase against the number of kanamycine resistant colonies.
- Figure 7:: Insertion loci (blue lines) of the insertion element into the target plasmid.
- Figure 8 :: Cyclic representation of the *E.coli*-genome with insertion loci (black lines).
- Figure 9:: Depiction of the insertion loci of the auxotroph candidates in the genome of *E.coli*-Genom. The gene products are given and the regulable candidates are marked.
- Figure 10:: Minimal media plates to confirm the regulable auxotroph candidates.
The two auxotroph candidates with disruptions of *argG* and *serA* served as control, as they do not grow on either plate. The two regulable candidates with insertion near *cysP* and *thiC* do not grow without, but with atc. The incubation time was 36 h at 37°C
- Figure 11.: Depiction of the locus and the direction of insertion of the element (black arrow) in the regulable auxotroph mutant *cysP*.
- Figure 12:: Depiction of the locus and the direction of insertion of the element (black arrow) in the regulable auxotroph mutant *thiC*
- Figure 13:: Minimal media plates to confirm the regulable auxotroph *Bacillus* candidates. A prototroph bacillus strained, grwing on either plate served as control. The three regulable candidates with insertions near kinA and yhaO only grow in the absence of atc. Te insertion locus of the third auxotroph was not determined. The incubation time was 48 h at 37°C.
- Figure 14:: Depiction of the locus and the direction of insertion of element two in the first regulable auxotroph *Bacillus* strain. The putatively regulated target gene may be the gene *yhaM* that displays similarity to similar to CMP-binding factors.
- Figure 15:: Depiction of the locus and the direction of insertion of element two in the second regulable auxotroph *Bacillus* strain. The putatively regulated target gene may be the gene *patA* that encodes an aminotransferase or *kinA* whose gene product is part of a two-component system.
- Figure 16:: Depiction of the different phenotypes in liquid medium. "WT" corresponds to *E.coli* MG1655 pWH1411BD+. Highlighted in yellow are regulable candidates without auxotroph phenotype, candidates in orange display a regulable (white characters) or a constitutive growth defect (black characters). The candidates highlighted red comprise regulable auxotrophy (white characters) or constitutive auxotrophy (black characters). "F" denotes the ratio in OD₅₉₅ between growth with and without atc, as a means for regulation.
- Figure 17:: Cyclic depiction of the *E.coli*-genome and the loci of insertion (black lines).
- Figure 18:: Distribution of the insertion loci concerning the insertion sequence. Given are the base pairs, the insertion element has inserted into, as well as the frequency.
- Figure 19:: Cyclic depiction of the *E.coli*-genoms and the 202 different insetion loci (black lines). The randomised insertion was clearly observed.
- Figure 20:: Depiction of the plasmid containing the enhanced element one. In between the two Mosaic elements (ME) there are the R6Kγ-ori (light blue), the kanamycine resistance cassette (dark blue) flanked by two FRT sites (orange), the T7 promoter (pT7, pink), two transcriptional terminators (T, green) and the tetracycline dependent promoter (p*tetA*, blue). Additionally the plasmid contains a ampicillin resistance cassette (bla, red).

The Examples illustrate the invention:

### Example I: Construction of insertion elements

Two different insertion elements were constructed. While element one is intended to be used in Gram negative bacteria, element two is supposed to be applied in Gram positive organisms.

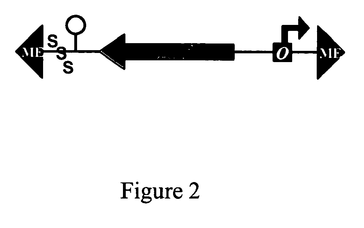

Construction of insertion element one: The construction of the first insertion element was accomplished by oligonucleotide hybridisation and subsequent cloning into a suitable vector. After successful cloning of the plasmid, a selectable resistance cassette was cloned into the insertion element, now consisting of 1350 bp. It contains the following components (see Fig. 2): Between two specific transposase binding sites (ME), there are three staggered stop-codons at the 5' end followed by a transcriptional terminator to oppress possible upstream translation and transcription effects. Downstream there is a kanamycine resistance cassette and directly at the 3' end in front of the Mosaic Element there is the tetracycline dependent promoter transcribing outward of the insertion element. The entire element can be cut out of the carrier-vector by *Pvu*II, allowing the application as *in vitro* system.

Construction of insertion element two: The construction of the second element was accomplished likewise. In contrast to element one, the resistance cassette is flanked by asymmetric *loxP* sites which are the targets for cre recombinase (9). These will allow the excision of the selection marker. Furthermore, the *tetR* gene is positioned on the element, which is transcribed by an autoregulative promoter (Fig. 3).

There are two versions of the tetracycline dependent promoter transcribing outward of the element: One contains a single *tet* operator (see Fig. 3) and corresponds to p_{*xyl*/*tet*} of plasmid pWH353 (10), while the other has two of these TetR binding sites (not shown) and resembles the promoter of pWH354 (10). Like element one, element two can be cut out of its carrier vector with *Pvu*II.

### Example II: Tests of the regulable promoters

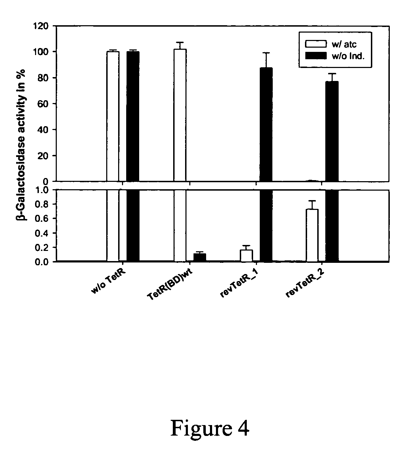

Test of the outward promoter of element one: to test the regulable promoter of the element one, the DNA fragment was cloned upstream of an episomal promoterless *lacZ* gene. The resulting reporter plasmid was cotransformed into the strain *E. coli* DH5α together with an expression plasmid for TetR. Thereby, different *tetR* alleles with different phenotypes were used. By means of β-galactosidase assays, the anhydrotetracycline dependent regulation was demonstrated and quantified (Fig.4). As shown in the figure, the regulatory window with a factor of 888 for wild type TetR and 524, or 105, respectively for the two analysed reverse TetR variants is very broad.

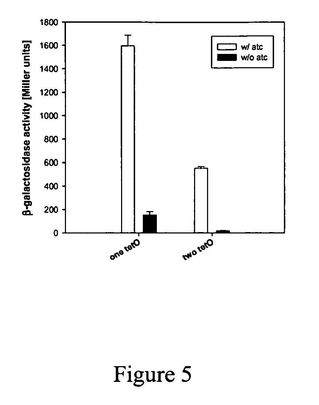

Test of the outward promoter of element two: To test the regulation capacities of element two, the respective DNA fragments were cloned upstream of a promoterless *lacZ* gene flanked by up- and downstream *amyE* sequences of *Bacillus subtilis*. The resulting reporter plasmid was transferred into *B. subtilis* 168 (wild type) and was partially integrated into the *amyE* locus by double homologous recombination. Via β-galactosidase assays, the anhydrotetracycline dependent regulation was demonstrated and quantified (Fig.5).

### Example III: Functionality test of the system by in vitro insertion experiments

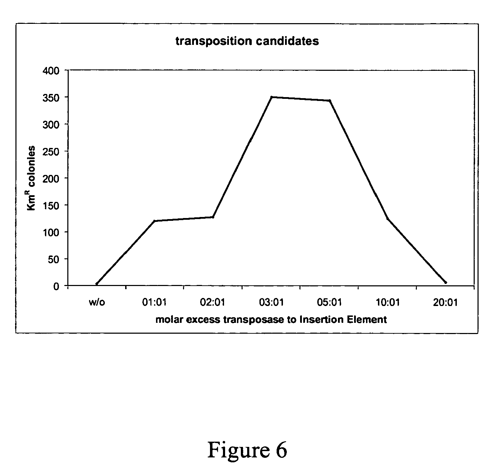

Functionality of element one: To test for the functionality of the systems, *in vitro* insertion experiments were conducted. As target DNA the plasmid pUC19 was used. The cut and purified insertion element and the transposase were applied in different ratios, to gain optimal yield of insertion mutants. Subsequently, E. *coli* DH5α was transformed with the reaction mixtures and plated on LB media plates containing kanamycine. The different obtained colony numbers were plotted against the molar excess of transposase over insertion element (Fig.6).

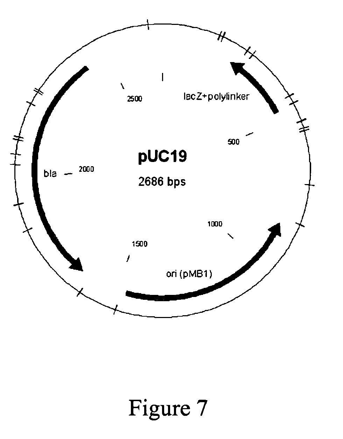

The highest yield of kanamycine resistant colonies was achieved at a molar ratio of insertion element to transposase of 1:5. Of cells of some colonies, plasmids were extracted to determine the site of insertion. Of 30 examined candidates, 24 showed a different insertion locus (Fig. 7). Therefore, the activity of the transposase could be confirmed and indication was gained for random integration instead of certain preferred loci of the plasmid.

Functionality of element two: As element two also contains Mosaic Elements and in general shares a quite common architecture with element one, tests for its functionality were not so extensive. Applying the optimised reaction set-ups (see in the figure), it was demonstrated that the element can integrate into a vector *in vitro.* Of three prepared new plasmids of the respective *E. coli* transformants, restriction patterns confirmed different insertion sites (data not shown).

### Example IV: Test of the insertion activity into bacterial genomes

Assembly of the insertion complex: For the assembly of the protein-DNA complex, purified transposase is added to the respective insertion element and the reaction mixture is incubated 30 min at room temperature. No magnesium ions must be present in the mixture to avoid undesired insertion reactions.

Electroporation of the complex into *E. coli* cells: Electroporation of element one into *E. coli* was performed according to the instruction of Sambrook (11). 1µl of the protein-DNA-complex and an aliquot of the electrocompetent cell suspension was used. The recipient strain was E. *coli* MG1655, bearing pWH1411BD+. MG1655 is the reference strain of the nucleotide database whose complete sequence is known. The plasmid pWH1411BD+ constitutively expresses TetR(BD) (2). Afterwards the cells were streaked out on plates containing kanamycine and chloramphenicol as selection agents for the insertion element and the plasmid. Plates were incubated over night at 37°C and colonies were counted the next day. Of the deployed cells, approximately 0.3% survived the electroporation procedure. Among these, around 0.6% carried the insertion element.

Electroporation of the complex into *B. subtilis* cells: Electroporation of element two into *B. subtilis* was performed according to an instruction described in the PhD thesis of Fuchsbauer (12). The reaction set-up again somewhat reflects the method applied for *E. coli.* The entire reaction mixture of the protein-DNA-complex was added to 300µl of electrocompetent cells of *B. subtilis* 168, whose complete sequence is known. Possible transponants were streaked out on plates containing 15µg/l kanamycine for selection.

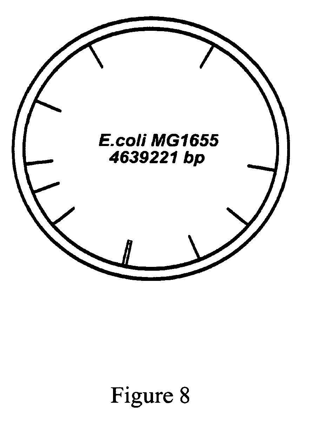

Characterisation of insertion *E. coli* candidates: Of cultures of some of the kanamycine resistant *E. coli* candidates putatively containing the insertion element one, chromosomal DNA was prepared without pre-selection. After precipitation with polyethylene glycol, the DNA was restricted with *Eco*RI and sequenced via the cycle-sequencing reaction. The sequences again displayed random integration into the genome. A total of eleven candidates' DNA was assayed, whereas no insertion locus appeared twice (Fig. 8).

### Example V: Screening system for E. coli on solid medium

Construction of mutant pool of E. coli by insertion of the DNA fragments: For detailed examination of the insertion reaction, a mutant pool of E. *coli* strains apt for a selection system should be generated. Therefore, electroporation was performed and the total number of kanamycine resistant colonies was determined to be around 7,200.

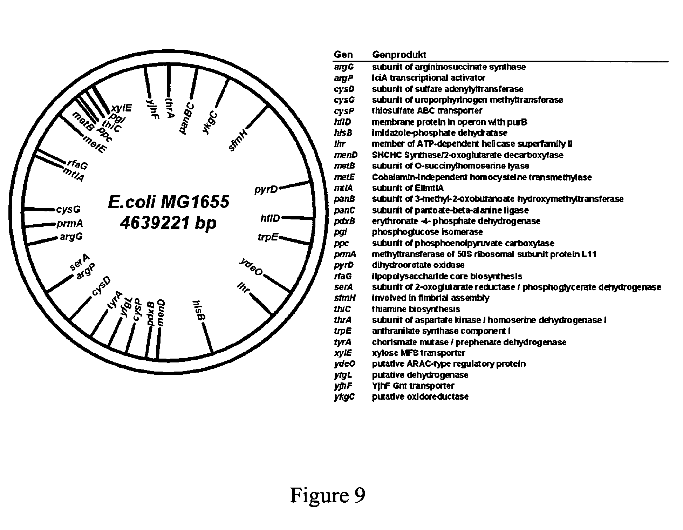

The screening system: first a screen on minimal agar plates with glucose as sole carbon source with or without anhydrotetracycline was conducted. A total of 2,400 colonies were streaked out. After over night incubation of the plates at 37°C they were analysed for growth of the candidates. 35 auxotroph candidates were found, two of which with a regulable auxotrophy (red in Fig. 9). All auxotroph candidates were streaked out on LB-agar plates, inoculated in liquid media and their chromosomal DNA was extracted using the Qiagen DNA-Mini kit. After restriction with *Eco*RI a sequencing reaction was performed and the resulting sequences were subjected to BLASTN analyses to determine the insertion locus in the genome. Like before, the random manner of insertion was confirmed.

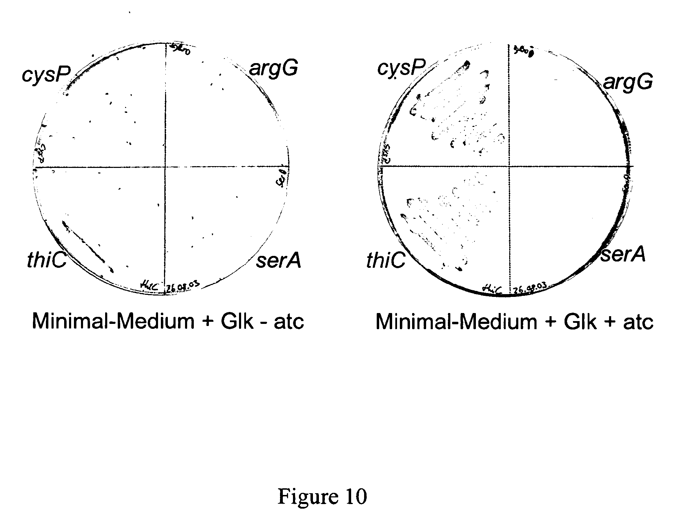

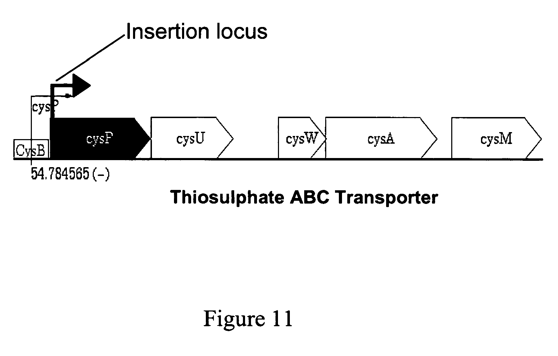

The two regulable candidates were re-streaked on minimal media plates to verify their phenotype (Fig. 10). Clearly, the two regulable candidates can not grow in the absence (left part of Fig. 10) but in the presence of atc (Fig. 10 right part). The two other not regulable auxotroph candidates served as controls. The insertion locus of the two regulable candidates was determined by sequencing their chromosomal DNA. One carries the insertion element downstream of the start codon of the gene *cysP* (Fig. 11), whereby the natural start codon is replaced by the GTG sequence of the Mosaic Element. Thus the tetracycline dependent promoter has gained transcriptional control over the cys-operon *cysPUWAM*, encoding the thiosulphate-ABC-transporter.

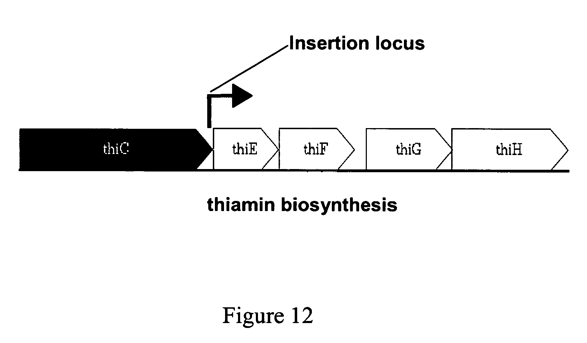

The other mutant carries the insertion element at the 3'-end of the gene *thiC* (Fig.12). Therefore, the rest of the thi-operon is controlled by the tetracycline dependent promoter. The *thi*-operon encodes proteins for biosynthesis of thiamine.

A massive constraint of this kind of screening system is the need to quantify the colony size by eye.

### Example VI: Screening system for B. subtilis on solid medium

Construction of mutant pool of B. subtilis by insertion of the DNA fragments: a similar approach was taken for the creation of a mutant bank of *B. subtilis.* Upon electroporation a total of approximately 390 kanamycine resistant candidates, putatively harbouring element two were obtained.

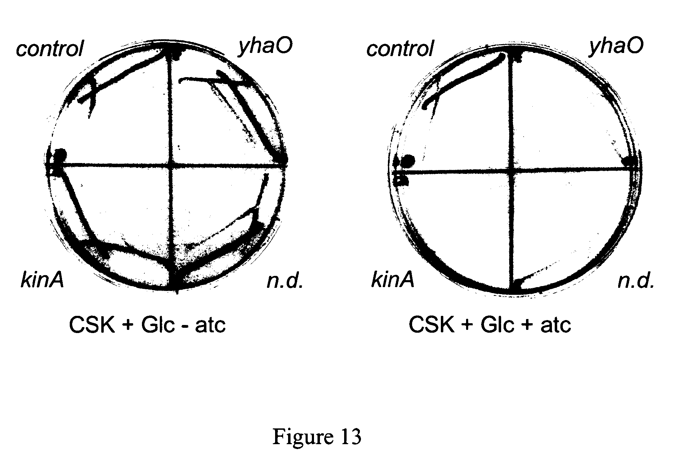

Like for E. *coli,* a screen on minimal agar plates with glucose as sole carbon source with and without 0.4µM atc was conducted to screen for auxotroph bacilli. However, CSK instead of M9 minimal medium was used. All 387 obtained candidates were streaked out. After several days of incubation at 37°C they were analysed for growth of the candidates. 11 auxotroph candidates were found, three of which with a regulable auxotrophy (red in Fig. 13).

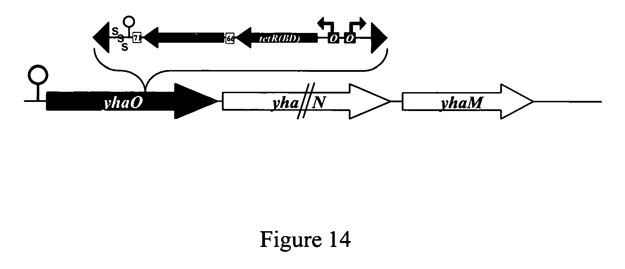

The procedure for detecting the insertion loci was essentially like for *E. coli.* Of the three candidates' DNA only two sequences could be obtained. The two insertion loci are around 400 kb apart (data not shown). In the first candidate the element had inserted into the *yhaO* gene (see Fig. 14). The putatively regulated target gene may be the gene *yhaM that* displays similarity to CMP-binding factors.

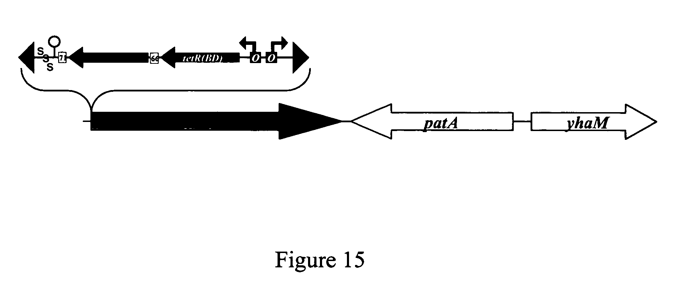

The second candidate has the element integrated in the ATG start codon of *kinA* (Fig. 15). As is the case in one of the regulable E. *coli* candidates, the GTG sequence of the Mosaic Element may compensate for the disrupted start codon.

### Example VII: Screening system for E. coli in liquid medium

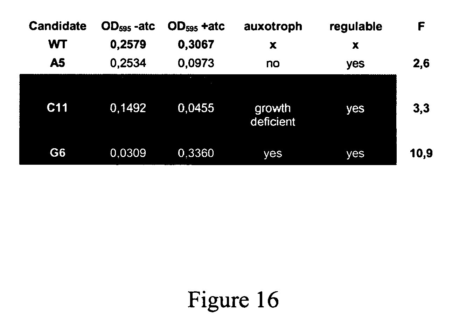

We have developed a 96-well plate liquid culture broth screening system for auxotroph *E. coli* strains. Thus the growth of the candidates can be monitored by measuring the optical density (OD₆₀₀). Minimal medium with glucose as sole carbon source, and kanamycine (Km) and chloramphenicol (Cm) as selection markers was used. For preparation, half of the well plates were filled with 200µl of minimal medium per well and stored at 4°C. Furthermore, a stock culture of the mutant pool was streaked onto LB/Km/Cm-plates such that single colonies appeared after incubation. At the next day, each well was inoculated with one colony. Each well-plate also contained minimal medium without culture and the parent strain as references. After inoculation wells were mixed using a multi-channel pipette and half of the inoculated medium was transferred into well containing 25µl of a 2µM atc solution as inducer. Finally 25µl of fresh minimal medium was transferred into the wells without atc to equalise the volumes. Plates were then incubated over night at 37°C in an incubator. The next day, after mixing of the cultures, the well plates could be evaluated in a TECAN Multiplate-Reader by measuring the optical density OD₅₉₅. The following phenotypes were encountered (Fig. 16):

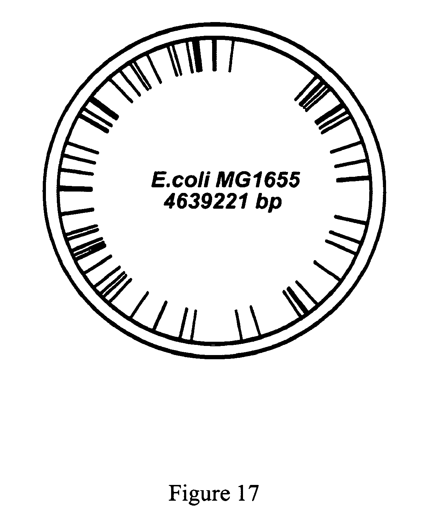

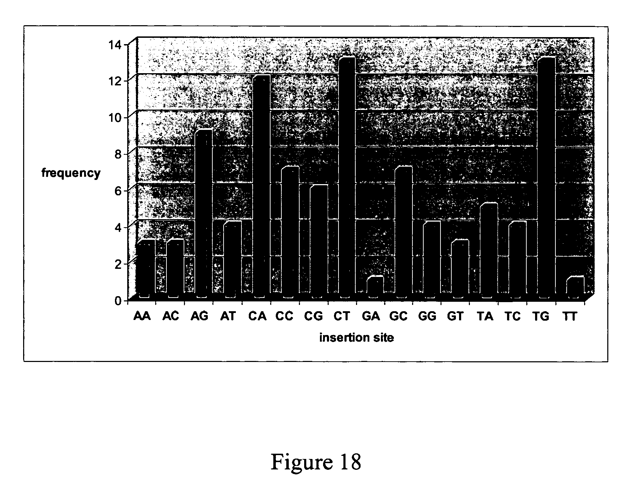

The candidates highlighted yellow display regulation without auxotroph phenotype. The candidates given in orange display growth defect. Among them, regulables are given in white characters and non-regulables are in black characters. Finally the red highlighted candidates display an auxotroph phenotype, separated into non-regulable (black characters) and regulable (white characters). Of the latter phenotype, four candidates were identified. "F" denotes the ratio of OD₅₉₅ with and without atc, as a means for regulation. The most interesting candidates, i.e. all auxotrophs and the best of the other regulables were inoculated and their chromosomal DNA was prepared. After restriction with *Eco*RI the insertion locus was determined by sequencing. The sequence analysis again confirmed the random fashion of insertion into the genome (Fig. 17). Likewise, the sequence of the insertion loci was determined in all candidates. No preference of the insertion for a particular base pair was detected (Fig. 18).

### Example VIII: Accomplishment of further screens in liquid medium and verification of the results

Meanwhile, further mutant pools of E. *coli* MG1655 were created and screened for regulable auxotroph candidates, as described above. For this purpose, also tetracycline repressors with a reverse phenotype (revTetR) were utilised to possibly yield further phenotypes, e.g. regulability of essential genes. The total number of mutants in the "WT-pool" has now expanded to 25,000, while the total number of the "revTetR-pools" is 105,000. 7,520 candidates were subjected to screening in liquid medium. It turned out that not all phenotypes were consistent in this screen. This was assayed on solid minimal medium, where the interesting candidates were streaked out. It turned out that only around 20% of the candidates could be confirmed according to their phenotype. Eventually, of 396 candidates streaked out, 75 could be confirmed (including the mutants of the solid medium screen described elsewhere herein). Of these, 15 candidates displayed a regulable phenotype that can be divided into two subgroups: The auxotroph regulable phenotype was found 11 times and the auxotroph regulable phenotype four times. The growth defect phenotypes could not be confirmed on solid media plates.

### Example IX: Summary and future perspectives

The liquid media screening systems yields a plethora of phenotypes of different E. *coli* transponants. This is due to the differences in growth of the candidates with or without atc, which can hardly be differentiated on solid media plates. Besides auxotrophs, candidates are found that display a reverse regulable phenotype, i.e. growth only in the absence of atc. During all screenings, 202 different genes were detected where the element had inserted (Fig. 19). These include mutants that could not be verified on solid medium.

The utilisation of two regulators with different phenotypes, wtTetR and revTetR, resulted in a different spectrum of mutants: While in the wtTetR screening reverse regulable mutants were found, the revTetR screen yielded essential genes. This demonstrates that different kinds of regulators can be applied to reach the goal of the screening. The analysis of the 15 regulable candidates described above revealed that some of them are already published, such as *mraZ*, the first gene in the *dcw* cluster, responsible for cell division and cell wall synthesis. The transcription of this cluster has previously been put under the control of a regulable promoter and analysed (13). By contrast, the *yrbK* gene was identified, whose function is unknown so far. This mutant could now be subjected to further analysis to elucidate this gene's function and to possibly obtain a novel drug target. Table 1 summarises all found regulable auxotroph mutants as well as the assigned function, product or biosynthesis pathway of the respective gene.

**Table 1: List of all reguable mutants obtained from the liquid- and solid media screenings. Given are genes directly affected by insertion. "Regaux" denotes auxotroph regulable mutants, while "revreg" reverse regulable mutants.**

| gene | function, gene product or biosynthesis pathway | kind of regulation |
|---|---|---|
| *aroB* | 3-Dehydroquinatesynthase | regaux |
| *cysP* | thiosulphate ABC transporter | regaux |
| *cysQ* | sulphite synthesis | regaux |
| *ilvY* | transcriptional dual regulator | regaux |
| *mraZ* | involved in cell wall synthesis and cell division | regaux |
| *nirC* | nitrite FNT transporter | regaux |
| *pyrF* | orotidine-5'-phosphate-decarboxylase | regaux |
| *rplM* | 50S ribosomal subunit protein L13 | regaux |
| *thic* | thiamine biosynthesis | regaux |
| *trpE*/*trpL* | subunit of anthranilate synthetase | regaux |
| *yrbK* | conserved hypothetical protein | regaux |
| *b3557* | IS5 protein | revreg |
| *b3557* | IS5 protein | revreg |
| *b3557* | IS5 protein | revreg |
| *cspA* | transcriptional activator CspA | revreg |

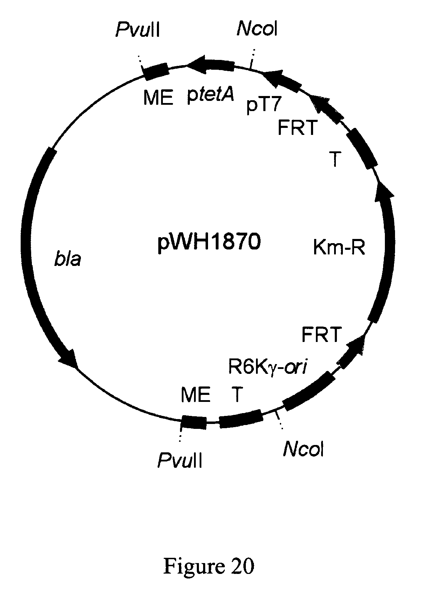

These numbers clarify that this technique can be applied to generate numerous mutants with different phenotypes. In the time coming up, we are trying to establish even more efficient methods. Furthermore, this insertion analysis is planned to be performed in pathogen bacteria like *Salmonella typhimurium*. Recently, element one has been improved by adding additional features (Fig. 20). The kanamycine resistance cassette is now flanked by two FRT sites to optionally excise the cassette by using Flp recombinase. This opens the possibility to insert an additional element into the chromosome of a distinct mutant utilizing the "recycled" selection marker. A R6Kγ-ori was also added to the element, which can initiate replication only when the pi-protein, which was deleted from this ori, is present elsewhere in a suitable strain. This ori simplifies the recovery of the insertion site by the so called "rescue cloning" technique. The last feature added to the element was a T7 promoter. This is widely used for *in vitro* transcription to obtain transcripts from the chromosome for further analysis e.g. in microarrays. These features are currently being tested. So this element may have gained a lot more versatility as tool for the search and examination of new drug targets.

For *Bacillus,* the efficiency of electroporation has to be enhanced. In addition, the application of *in vitro* transposition followed by transformation of recombinant DNA may be a promising approach. Despite the relatively low amount of transponant *Bacilli,* three auxotroph strains with a regulable phenotype were discovered. It is planned to exchange components of the second element to gain a broader range of regulation as well as to exploit revTetR regulation, as in E. *coli.* The functionality of the asymmetric IoxP sites for cre-recombinase mediated excision of the selection marker has already been confirmed in E. coli but will have to be established for *B*. *subtilis.* Furthermore application of element two in pathogen Gram positive bacteria like *Listeria, Staphylococcus,* or *Streptococcus* are envisioned.

### Literature

1. Hillen, W. & Berens, C. (1994) *Annu Rev Microbiol* **48,** 345-369.
2. Scholz, O., Henssler, E. M., Bail, J., Schubert, P., Bogdanska-Urbaniak, J., Sopp, S., Reich, M., Wisshak, S., Köstner, M., Bertram, R. & Hillen, W. (2004) *Mol Microbiol* **53,** 777-789.
3. Berens, C. & Hillen, W. (2003) *Eur J Biochem* **270,** 3109-3121.
4. Hare, R. S., Walker, S. S., Dorman, T. E., Greene, J. R., Guzman, L. M., Kenney, T. J., Sulavik, M. C., Baradaran, K., Houseweart, C., Yu, H., Foldes, Z., Motzer, A., Walbridge, M., Shimer, G. H., Jr. & Shaw, K. J. (2001) J *Bacteriol* **183,** 1694-1706.
5. Akerley, B. J., Rubin, E. J., Novick, V. L., Amaya, K., Judson, N. & Mekalanos, J. J. (2002) *Proc Natl Acad Sci U S A* 99, 966-971.
6. Goryshin, I. Y., Jendrisak, J., Hoffman, L. M., Meis, R. & Reznikoff, W. S. (2000) *Nat Biotechnol* **18**, 97-100.
7. Judson, N. & Mekalanos, J. J. (2000) *Nat Biotechnol* **18**, 740-5.
8. Rappleye, C. A. & Roth, J. R. (1997) *J Bacteriol* **179,** 5827-34.
9. Yoon, Y. G., Posfai, G., Szybalski, W. & Kim, S. C. (1998) *Gene* 223, 67-76.
10. Geissendörfer, M. & Hillen, W. (1990) *Appl Microbiol Biotechnol* **33,** 657-663.
11. Sambrook, J. & Russel, D. (2001) *Molecular cloning: A Laboratory Manual*. (Cold Spring Harbor Laboratory Press, New York).
12. Fuchsbauer, N. (2003) *PhD thesis.*
13. Mengin-Lecreulx, D., Ayala, J., Bouhss, A., van Heijenoort, J., Parquet, C. & Hara, H. (1998) *J Bacteriol* **180,** 4406-4412.

## Claims

1. Insertion element, wherein said insertion element is a recombinant nucleic acid molecule which comprises, in 5' → 3' arrangement:
(i) a first recombinase binding site;
(ii) a resistance gene, operatively linked to a promoter and, optionally, to transcription control sequences;
(iii) a tetracycline-controlled promoter; and
(iv) a second recombinase binding site;
wherein said tetracycline-controlled promoter is oriented to allow transcription in direction of the second recombinase binding site.

2. The insertion element of claim 1, wherein at least one stop codon is located between the first recombinase binding site and the resistance gene.

3. The insertion element of claim 1 or 2, wherein at least one terminator of transcription is located 5' of the resistance gene.

4. The insertion element of any one of claims 1 to 3, wherein the resistance gene is selected from the group consisting of cat, aph, bla, ermB, rpsL, aada5, hph, rpoB.

5. The insertion element of any one of claims 1 to 4, wherein the resistance gene is flanked by additional recombinase binding sites which are different from the first and second recombinase binding site.

6. The insertion element of claim 5, wherein said additional recombinase binding sites are selected from FLP recombinase dependent FRT sites and Cre recombinase dependent loxP sites.

7. The insertion element of any one of claims 1 to 6, comprising a prokaryotic origin of replication.

8. The insertion element of any one of claims 1 to 7, wherein at least one viral promoter is located upstream of the tetracycline controlled promoter.

9. The insertion element of any one of claims 1 to 8, comprising an additional coding sequence for a tetracycline repressor, wherein the coding sequence is
(a) operatively linked to a promoter and, optionally, to transcription control sequences; or
(b) operatively linked to an internal ribosomal entry site and arranged 3' to a promoter sequence of the insertion element, allowing expression of the repressor from a multicistronic RNA.

10. The insertion element of any one of claims 1 to 9, wherein the promoter is a prokaryotic promoter.

11. The insertion element of claim 10, wherein the promoter is controlled by at least one tet-operator binding site sequence selected from
(a) SEQ ID NO: 1 to 6; or from
(b) SEQ ID NO: 7 to 13;
or permutations thereof.

12. The insertion element of any one of claims 1 to 6, wherein the promoter is a eukaryotic promoter.

13. The insertion element of claim 12, wherein the eukaryotic promoter is selected from the group consisting of CMV, RSV, SV40, Cadherin and hAPC and β-Act , AFP, CD43, CD45, CD68, c-erbB2, EF1, E2F-1, EGR1, FerL, GAPDH, HSP70, IFNB, Mb, PGK-1, TRP1.

14. The insertion element of claim 13, wherein the eukaryotic promoter is selected from any one of SEQ ID NOs: 14 to 21.

15. The insertion element of claim 10 or 11, wherein the recombinase binding site is a binding site obtainable from the transposases Tn5, Tn10, Tn7, or from phage Mu.

16. The insertion element of claim 13 or 14, wherein the recombinase binding site is selected from the group consisting of tc1/mariner and minos.

17. A vector, comprising an insertion element of any one of claims 1 to 16.

18. A method for the generation of a conditional knock-out mutant of a cell, comprising the steps of:
(a) introducing a nucleic acid molecule comprising the insertion element of any one of claims 1 to 16 into a cell;
wherein the insertion element is transfected into the cell concomitant with purified recombinase
(b) integrating the insertion element into the cellular genome; and
(c) selecting cells for resistance against a substrate for which the resistance gene of the insertion element confers resistance;
wherein prior to or concomitant with step (a) an expression construct for a tetracycline-sensitive (poly)peptide is introduced into the cell, said (poly)peptide being capable of binding to the tetracycline-controlled promoter of the insertion element and of modulating its expression.

19. The method of claim 18, wherein the tetracycline-sensitive (poly)peptide is a repressor of transcription.

20. The method of claim 19, wherein the tetracycline-sensitive (poly)peptide comprises the amino acid sequence of any one of
(a) SEQ ID NOs: 22 to 41;
(b) SEQ ID NOs: 42 to 63;
(c) SEQ ID NOs: 64 to 256;
(d) SEQ ID NOs: 257 to 266;
(e) SEQ ID NOs: 267 to 311 or
(f) SEQ ID NOs: 312 to 316
or a tetracycline-sensitive mutant of this sequence, wherein said mutant has a sequence identity of at least 92%, is capable of binding the operator sequence and is inducible by a tetracycline.

21. The method of claim 18, wherein the tetracycline-sensitive (poly)peptide is an activator of transcription.

22. The method of claim 21, wherein the tetracycline-sensitive (poly)peptide comprises the amino acid sequence of any one of SEQ ID NOs: 317 to 357.

23. The method of any one of claims 18 to 22, wherein the cells are grown in the presence of tetracycline or of a functional homolog thereof, wherein said homolog is selected from the group consisting of Tetracycline, Doxycycline, Minocycline, Sancycline, Anhydrotetracycline, Chlorotetracycline, 4-DDMA-Anhydrotetracycline, Oxytetracycline, 4-DDMA-tetracycline, 2-Nitrilo-tetracycline, 4-Dedimethyl-aminosancycline, 4-DDMA-chlor-tetracycline, 4-DDMA-11,12-pyrazolo-tetracycline, 4-DDMA-4-hydroxy-tetracycline, 4-DDMA-12a-dehydroxy-tetracycline, 4-DDMA-doxycycline.

24. The method of any one of claims 18 to 22, wherein the cells are grown in the presence of a peptide-based functional homolog of tetracycline or in the presence of a (poly)peptide comprising said peptide, wherein said peptide-based homolog or said (poly)peptide comprises any one of SEQ ID NOs: 358 to 371.

25. A cell containing the insertion element of any one of claims 1 to 16, the vector of claim 17 or which is obtainable by the method of any one of claims 18 to 24.

26. The cell of claim 25, wherein said cell is eukaryotic or prokaryotic cell.

27. The cell of claim 26, wherein said cell is selected from the group consisting of vertebrate and invertebrate cell, insect cell, fungal cell or bacterial cell.

28. The cell of claim 27, wherein said vertebrate cell is a cell of xenopus, zebrafish or a mammalian cell.

29. The cell of claim 28, wherein said mammalian cell is an embryonic stem cell, an adult stem cell or a somatic cell.

30. The cell of claim 26, wherein said eukaryotic cell is a plant cell.

31. The cell of claim 27, wherein said bacterial cell is selected from the group consisting of *Acinetobacter, Actinobacillus, Actinomyces, Aeromonas, Bacillus, Bacteroides, Bdellovibrio, Bordetella, Borrelia, Branhamella, Brucella, Burkholderia, Campylobacter, Capnocytophaga, Clamydia, Citrobacter, Clostridiae, Corynebacterium, Coxiella, Eikenella, Erysipelothrix, Escherichia, Francisella, Fusobacterium, Haemophilus, Helicobacter, Klebsiella, Legionella, Leptospira, Listeria, Meningococcus, Moraxella, Mycobacteria, Mycoplasma, Neisseriaceae, Nocardia, Pasteurella, Peptostreptococcus, Pneumococcus, Proteus, Providencia, Pseudomonas, Rickettsia, Salmonella, Selenomonas, Serratia, Shigella, Spirillum, Staphylococcus, Streptococcus, Treponema, Veillonella, Vibrio, Wolinella, Yersinia.*

32. A method for identifying genetic determinants of pathogen survival, comprising the steps of:
(a) infecting an organism with a pathogen, wherein the pathogen is the cell of any one of claims 25 to 31 or a multi-cellular organism comprising said cell;
(b) growing the cell in the presence and absence of tetracycline or in the presence or absence of a functional homolog thereof, wherein said homolog is
(i) a peptide-based functional homolog of tetracycline or a (poly)peptide comprising said peptide, wherein said peptide-based homolog or said (poly)peptide comprises any one of SEQ ID NOs: 358 to 371; or
(ii) a homolog which is selected from the group consisting of Tetracycline, Doxycycline, Minocycline, Sancycline, Anhydrotetracycline, Chlorotetracycline, 4-DDMA-Anhydrotetracycline, Oxytetracycline, 4-DDMA-tetracycline, 2-Nitrilo-tetracycline, 4-Dedimethyl-aminosancycline, 4-DDMA-chlor-tetracycline, 4-DDMA-11,12-pyrazolo-tetracycline, 4-DDMA-4-hydroxy-tetracycline, 4-DDMA-12a-dehydroxy-tetracycline, 4-DDMA-doxycycline;
(c) selecting cells, the growth of which is affected by the presence of tetracycline or the functional homolog thereof; and
(d) determining which gene or gene product of the cell of (c) is effected by the insertion of the insertion element;
wherein the gene or gene product identified in step (d) is a genetic determinant for the survival of the pathogen in the infected organism.

33. The method of claim 32, wherein the infected organism is a eukaryotic or prokaryotic cell.

34. The method of claim 32, wherein the infected organism is a vertebrate or invertebrate animal.

35. The method of claim 34, wherein the vertebrate is selected from mammalian, zebrafish and xenopus.

36. A method for the generation of transgenic non-human animal, comprising the step of propagating the cell of claim 28 or 29 and developing from said cell a viable organism, wherein said cell is non-human.

37. The method of claim 36, wherein said animal is a mammalian animal.

38. A transgenic non-human animal produced by the method of claim 36 or 37.

39. A method for the production of a vaccine composition, comprising the steps of:
(a) growing the bacterial cell of claim 27 or 31 in the presence and absence of tetracycline or a homolog thereof and selecting cells which display an attenuated phenotype in the absence of tetracycline; and optionally
(b) formulating the bacteria with a pharmaceutically acceptable carrier.

40. A vaccine composition prepared by the method of claim 39.

41. Use of the bacterial cell of claim 31 for the preparation of a vaccine composition for ameliorating or preventing diseases caused by the bacterial cell.

42. A method for generating a transgenic plant, comprising the steps of:
(a) cultivating the cell of claim 30 or a protoplast derived therefrom in suitable media; and
(b) regeneration of plants.

43. A plant or a product of the plant produced by the method of claim 42.

44. A method of cultivating a plant, comprising the step of growing the plant of claim 43.
